# EUROPEAN PATENT SPECIFICATION

(11) **EP 3 008 055 B1**
(45) Date of publication and mention of the grant of the patent: **19.07.2017**
(21) Application number: 14730804.3
(22) Date of filing: 04.06.2014
(51) Int. Cl.: C07D 403/12, C07D 413/14, C07D 401/04, C07D 401/06, C07D 401/12, C07D 403/04, C07D 403/06, C07D 403/14, C07D 413/06, C07D 257/04, C07D 491/107, A61P 25/00, A61K 31/41, A61K 31/4439, A61K 31/454

(54) **TETRAZOLONE DERIVATIVES**
TETRAZOLON-DERIVATE
DÉRIVÉS DE TÉTRAZOLONE

(30) Priority: 11.06.2013 EP 13171479
(43) Date of publication of application: 20.04.2016
(73) Proprietor: F. Hoffmann-La Roche AG, 4070 Basel (CH)
(72) Inventor: GRETHER, Uwe, 79588 Efringen-Kirchen (DE); NETTEKOVEN, Matthias, 79639 Grenzach-Wyhlen (DE); PUELLMANN, Bernd, CH-4147 Aesch (CH); ROEVER, Stephan, 79594 Inzlingen (DE); ROGERS-EVANS, Mark, CH-4103Bottmingen (CH); SCHULZ-GASCH, Tanja, CH-4417 Ziefen (CH)
(74) Representative: Pomeranc, Didier
(86) International application number: PCT/EP2014/061527
(87) International publication number: WO 2014/198592

(56) References cited:
- WO-A1-2009/117444
- WO-A1-2011/109324
- KANG ET AL: "Tetrazole-biarylpyrazole derivatives as cannabinoid CB1 receptor antagonists", BIOORGANIC & MEDICINAL CHEMISTRY LETTERS, PERGAMON, AMSTERDAM, NL, vol. 18, no. 7, 29 February 2008 (2008-02-29), pages 2385-2389, XP022574967, ISSN: 0960-894X, DOI: 10.1016/J.BMCL.2008.02.061

## Description

The present invention relates to organic compounds useful for therapy and/or prophylaxis in a mammal, and in particular to compounds that are preferential agonists of the Cannabinoid Receptor 2.

The invention relates in particular to a compound of formula (I) 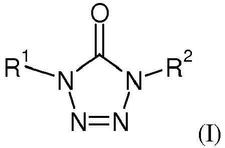 wherein R¹
is n-propyl, iso-butyl, cyclopropyl, 3-fluorophenyl, 6-chloropyridin-3-yl, 3-chlorophenyl, 4-chlorophenyl, 3-trifluoromethoxyphenyl, 2-(trifluoromethyl)phenyl, 3-(trifluoromethyl)phenyl, 4-(trifluoromethyl)phenyl, 2,6-difluorophenyl, 2,5-difluorophenyl, 3,4-difluorophenyl, 3,5-difluorophenyl, 3-methylphenyl, 4-methylphenyl, 3-methoxyphenyl, 4-methoxyphenyl, 2,3-dichlorophenyl, 1-methylindolyl or 3-bromophenyl;
R² is -C(O)NR³R⁴ or R⁵;
one of R³ and R⁴ is hydrogen, alkyl, cycloalkyl or alkoxyalkyl and the other one is -(CH₂)ₙ-R⁶;
or R³ and R⁴ together with the nitrogen atom to which they are attached form heterocyclyl or substituted heterocyclyl, wherein heterocyclyl is piperidinyl, morpholinyl, pyrrolidinyl, 3,4-dihydro-1H-isoquinolinyl, azetidinyl, piperazinyl, 1,1-dioxothiomorpholinyl or 2-oxa-8-aza-spiro[4.5]decyl, and wherein substituted heterocyclyl is heterocyclyl substituted with one substituent selected from halogen, alkyl, cyano, alkoxyalkyl, aminocarbonyl, dialkylaminocarbonyl, dialkylaminocarbonylalkyl, alkylaminocarbonylalkyl, phenyl, halophenyl, phenylalkyl, halophenylalkyl, methylpyrazolyl, methylisoxazolyl, alkoxyalkyl, alkylcarbonylamino, alkylcarbonyl, alkoxycarbonyl, alkyl-[1,2,4]oxadiazolyl, pyrrolidinylcarbonylalkyl, pyrazinyl, (alkyl)(alkylcarbonyl)amino, alkylisoxazolyl and morpholinylcarbonylalkyl, or with two substituents independently selected from alkyl, halogen, alkoxycarbonyl and alkoxycarbonylamino;
R⁵ is halophenylalkyl, alkoxycarbonylpiperidinyl, alkoxycarbonylalkyl, (alkylsulfonyl)(alkyl)[1,2,4]triazolylalkyl or morpholinylcarbonylalkyl;
R⁶ is alkoxy, dialkoxyphenyl, cyano, phenyl, pyridinyl or alkoxycarbonylalkyl; and
n is 0, 1, 2 or 3;
or a pharmaceutically acceptable salt therof;
provided that
1-Cyclopropyl-4-(pyrrolidine-1-carbonyl)-1,4-dihydro-tetrazol-5-one;
1-Cyclopropyl-4-(2-methyl-pyrrolidine-1-carbonyl)-1,4-dihydro-tetrazol-5-one;
1-Cyclopropyl-4-(2,5-dimethyl-pyrrolidine-1-carbonyl)-1,4-dihydro-tetrazol-5-one;
1-Cyclopropyl-4-(2,6-dimethyl-piperidine-1-carbonyl)-1,4-dihydro-tetrazol-5-one;
4-Cyclopropyl-5-oxo-4,5-dihydro-tetrazole-1-carboxylic acid benzyl-isopropylamide;
[4-(4-Chloro-phenyl)-5-oxo-4,5-dihydro-tetrazol-1-yl]-acetic acid ethyl ester;
2-[4-(4-Chloro-phenyl)-5-oxo-4,5-dihydro-tetrazol-1-yl]-propionic acid ethyl ester;
3-[4-(3-Chloro-phenyl)-5-oxo-4,5-dihydro-tetrazol-1-yl]-propionic acid methyl ester;
1-(2,3-Dichloro-phenyl)-4-(2-methyl-piperidine-1-carbonyl)-1,4-dihydro-tetrazol-5-one;
1-(2,6-difluorophenyl)-4-(3,4-dihydro-1H-isoquinoline-2-carbonyl)tetrazol-5-one;
4-(2,6-difluorophenyl)-5-oxo-N-phenyl-N-propyl-tetrazole-1-carboxamide;
4-(2,6-difluorophenyl)-N-ethyl-5-oxo-N-phenyl-tetrazole-1-carboxamide;
4-(2,6-difluorophenyl)-N-methyl-5-oxo-N-phenyl-tetrazole-1-carboxamide;
4-(2,6-difluorophenyl)-N-isopropyl-5-oxo-N-phenyl-tetrazole-1-carboxamide;
N-cyclohexyl-4-(2,6-difluorophenyl)-5-oxo-N-phenyl-tetrazole-1-carboxamide;
4-(2,6-difluorophenyl)-N-methyl-5-oxo-N-(2-pyridyl)tetrazole-1-carboxamide;
N,4-dicyclopropyl-5-oxo-N-phenyl-tetrazole-1-carboxamide;
N-butyl-4-cyclopropyl-5-oxo-N-phenyl-tetrazole-1-carboxamide;
4-cyclopropyl-N-isobutyl-5-oxo-N-phenyl-tetrazole-1-carboxamide;
4-cyclopropyl-N-isopropyl-5-oxo-N-phenyl-tetrazole-1-carboxamide;
4-cyclopropyl-5-oxo-N-phenyl-N-propyl-tetrazole-1-carboxamide;
4-cyclopropyl-N-methyl-5-oxo-N-phenyl-tetrazole-1-carboxamide;
4-cyclopropyl-N-ethyl-5-oxo-N-phenyl-tetrazole-1-carboxamide;
4-cyclopropyl-5-oxo-N-phenyl-N-sec-butyl-tetrazole-1-carboxamide ;
N-(1-benzyl-2-methyl-propyl)-4-cyclopropyl-5-oxo-N-phenyl-tetrazole-1-carboxamide;
1-(2-methylpiperidine-1-carbonyl)-4-(p-tolyl)tetrazol-5-one;
1-(2-methylpiperidine-1-carbonyl)-4-(m-tolyl)tetrazol-5-one;
N-isopropyl-5-oxo-N-phenyl-4-propyl-tetrazole-1-carboxamide;
1-(4-chlorophenyl)-4-[(2-fluorophenyl)methyl]tetrazol-5-one;
1-(4-chlorophenyl)-4-[(3-fluorophenyl)methyl]tetrazol-5-one;
1-(4-chlorophenyl)-4-[(3-chlorophenyl)methyl]tetrazol-5-one;
1-[(4-bromophenyl)methyl]-4-(4-chlorophenyl)tetrazol-5-one; and
methyl 3-[5-oxo-4-(p-tolyl)tetrazol-1-yl]propanoate;
are excluded.

The compound of formula (I) is particularly useful in the treatment or prophylaxis of e.g. pain, atherosclerosis, age-related macular degeneration, diabetic retinopathy, glaucoma, retinal vein occlusion, retinopathy of prematurity, ocular ischemic syndrome, geographic atrophy, diabetes mellitus, inflammation, inflammatory bowel disease, ischemia-reperfusion injury, acute liver failure, liver fibrosis, lung fibrosis, kidney fibrosis, systemic fibrosis, acute allograft rejection, chronic allograft nephropathy, diabetic nephropathy, glomerulonephropathy, cardiomyopathy, heart failure, myocardial ischemia, myocardial infarction, systemic sclerosis, thermal injury, burning, hypertrophic scars, keloids, gingivitis pyrexia, liver cirrhosis or tumors, regulation of bone mass, amyotrophic lateral sclerosis, multiple sclerosis, Alzheimer's disease, Parkinson's disease, stroke, transient ischemic attack or uveitis.

The compound of formula (I) is in particular useful in the treatment or prophylaxis of diabetic retinopathy, retinal vein occlusion or uveitis.

The cannabinoid receptors are a class of cell membrane receptors belonging to the G protein-coupled receptor superfamily. There are currently two known subtypes, termed Cannabinoid Receptor 1 (CB 1) and Cannabinoid Receptor 2 (CB2). The CB1 receptor is mainly expressed in the central nervous (i.e. amygdala cerebellum, hippocampus) system and to a lesser amount in the periphery. CB2, which is encoded by the CNR2 gene, is mostly expressed peripherally, on cells of the immune system, such as macrophages and T-cells (Ashton, J. C. et al. Curr Neuropharmacol 2007, 5(2), 73-80; Miller, A. M. et al. Br J Pharmacol 2008, 153(2), 299-308; Centonze, D., et al. Curr Pharm Des 2008, 14(23), 2370-42), and in the gastrointestinal system (Wright, K. L. et al. Br J Pharmacol 2008, 153(2), 263-70). The CB2 receptor is also widely distributed in the brain where it is found primarily on microglia and not neurons (Cabral, G. A. et al. Br J Pharmacol 2008, 153(2): 240-51).

The interest in CB2 receptor agonists has been steadily on the rise during the last decade (currently 30-40 patent applications/year) due to the fact that several of the early compounds have been shown to have beneficial effects in pre-clinical models for a number of human diseases including chronic pain (Beltramo, M. Mini Rev Med Chem 2009, 9(1), 11-25), atherosclerosis (Mach, F. et al. J Neuroendocrinol 2008, 20 Suppl 1, 53-7), regulation of bone mass (Bab, I. et al. Br J Pharmacol 2008, 153(2), 182-8), neuroinflammation (Cabral, G. A. et al. J Leukoc Biol 2005, 78(6), 1192-7), ischemia/reperfusion injury (Pacher, P. et al. Br J Pharmacol 2008, 153(2), 252-62), systemic fibrosis (Akhmetshina, A. et al. Arthritis Rheum 2009, 60(4), 1129-36; Garcia-Gonzalez, E. et al. Rheumatology (Oxford) 2009, 48(9), 1050-6), liver fibrosis (Julien, B. et al. Gastroenterology 2005, 128(3), 742-55; Munoz-Luque, J. et al. J Pharmacol Exp Ther 2008, 324(2), 475-83).

Ischemia/reperfusion (I/R) injury is the principal cause of tissue damage occurring in conditions such as stroke, myocardial infarction, cardiopulmonary bypass and other vascular surgeries, and organ transplantation, as well as a major mechanism of end-organ damage complicating the course of circulatory shock of various etiologies. All these conditions are characterized by a disruption of normal blood supply resulting in an insufficient tissue oxygenation. Re-oxygenation e.g., reperfusion is the ultimate treatment to restore normal tissue oxygenation. However the absence of oxygen and nutrients from blood creates a condition in which the restoration of circulation results in further tissue damage. The damage of reperfusion injury is due in part to the inflammatory response of damaged tissues. White blood cells, carried to the area by the newly returning blood, release a host of inflammatory factors such as interleukins as well as free radicals in response to tissue damage. The restored blood flow reintroduces oxygen within cells that damages cellular proteins, DNA, and the plasma membrane.

Remote ischemic preconditioning (RIPC) represents a strategy for harnessing the body's endogenous protective capabilities against the injury incurred by ischemia and reperfusion. It describes the intriguing phenomenon in which transient non-lethal ischemia and reperfusion of one organ or tissue confers resistance to a subsequent episode of "lethal" ischemia reperfusion injury in a remote organ or tissue. The actual mechanism through which transient ischemia and reperfusion of an organ or tissue confers protection is currently unknown although several hypotheses have been proposed.

The humoral hypothesis proposes that the endogenous substance (such as adenosine, bradykinin, opioids, CGRP, endocannabinoids, Angiotensin I or some other as yet unidentified humoral factor) generated in the remote organ or tissue enters the blood stream and activates its respective receptor in the target tissue and thereby recruiting the various intracellular pathways of cardioprotection implicated in ischemic preconditioning.

Recent data indicates that endocannabinnoids and their receptors, in particular CB2 might be involved in pre-conditioning and contribute to prevent reperfusion injury by downregulation of the inflammatory response (Pacher, P. et al. Br J Pharmacol 2008, 153(2), 252-62). Specifically, recent studies using CB2 tool agonists demonstrated the efficacy of this concept for reducing the I/R injury in the heart (Defer, N. et al. Faseb J 2009, 23(7), 2120-30), the brain (Zhang, M. et al. J Cereb Blood Flow Metab 2007, 27(7), 1387-96), the liver (Batkai, S. et al. Faseb J 2007, 21(8), 1788-800) and the kidney (Feizi, A. et al. Exp Toxicol Pathol 2008, 60(4-5), 405-10).

Moreover, over the last few years, a growing body of literature indicates that CB2 can also be of interest in sub-chronic and chronic setting. Specific upregulation of CB1 and CB2 has been shown to be associated in animal models of chronic diseases associated with fibrosis (Garcia-Gonzalez, E. et al. Rheumatology (Oxford) 2009, 48(9), 1050-6; Yang, Y. Y. et al. Liver Int 2009, 29(5), 678-85) with a relevant expression of CB2 in myofibroblasts, the cells responsible for fibrosis progression.

Activation of CB2 receptor by selective CB2 agonist has in fact been shown to exert anti-fibrotic effect in diffuse systemic sclerosis (Garcia-Gonzalez, E. et al. Rheumatology (Oxford) 2009, 48(9), 1050-6) and CB2 receptor has emerged as a critical target in experimental dermal fibrosis (Akhmetshina, A. et al. Arthritis Rheum 2009, 60(4), 1129-36) and in in liver pathophysiology, including fibrogenesis associated with chronic liver diseases (Lotersztajn, S. et al. Gastroenterol Clin Biol 2007, 31(3), 255-8; Mallat, A. et al. Expert Opin Ther Targets 2007, 11(3), 403-9; Lotersztajn, S. et al. Br J Pharmacol 2008, 153(2), 286-9).

Further background information can be found in WO 2011/109324; WO 2009/117444; and Kang et al., Bioorganic & Medicinal Chemistry Letters 2008, 18, 2385-2389.

The compounds of the invention bind to and modulate the CB2 receptor and have lower CB1 receptor activity.

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C₁-C₈ alkyl groups are methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl and pentyl. Particular examples of alkyl are methyl, ethyl, propyl, isopropyl, butyl and pentyl.

The term "cycloalkyl", alone or in combination, signifies a cycloalkyl ring with 3 to 8 carbon atoms and particularly a cycloalkyl ring with 3 to 6 carbon atoms. Examples of cycloalkyl are cyclopropyl, cyclobutyl, cyclopentyl and cyclohexyl, cycloheptyl and cyclooctyl. A particular example of "cycloalkyl" is cyclopropyl.

The term "alkoxy", alone or in combination, signifies a group of the formula alkyl-O- in which the term "alkyl" has the previously given significance, such as methoxy, ethoxy, n-propoxy, isopropoxy, n-butoxy, isobutoxy, sec-butoxy and tert.-butoxy. Particular "alkoxy" are methoxy, ethoxy and tert.-butoxy.

The terms "halogen" or "halo", alone or in combination, signifies fluorine, chlorine, bromine or iodine and particularly fluorine, chlorine or bromine, more particularly fluorine and chlorine. The term "halo", in combination with another group, denotes the substitution of said group with at least one halogen, particularly substituted with one to five halogens, particularly one to four halogens, i.e. one, two, three or four halogens. Particular "halogen" are fluorine, chlorine and bromine.

The term "haloalkyl", alone or in combination, denotes an alkyl group substituted with at least one halogen, particularly substituted with one to five halogens, particularly one to three halogens. A particular "haloalkyl" is trifluoromethyl.

The terms "hydroxyl" and "hydroxy", alone or in combination, signify the -OH group.

The term "carbonyl", alone or in combination, signifies the -C(O)- group.

The term "oxy", alone or in combination, signifies the -O- group.

The term "amino", alone or in combination, signifies the primary amino group (-NH₂), the secondary amino group (-NH-), or the tertiary amino group (-N-). A particular amino is -NH-.

The term "sulfonyl", alone or in combination, signifies the -S(O)₂- group.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared form addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula (I) can also be present in the form of zwitterions. Particularly preferred pharmaceutically acceptable salts of compounds of formula (I) are the salts of hydrochloric acid, hydrobromic acid, sulfuric acid, phosphoric acid and methanesulfonic acid.

"Pharmaceutically acceptable esters" means that the compound of general formula (I) may be derivatised at functional groups to provide derivatives which are capable of conversion back to the parent compounds in vivo. Examples of such compounds include physiologically acceptable and metabolically labile ester derivatives, such as methoxymethyl esters, methylthiomethyl esters and pivaloyloxymethyl esters.

If one of the starting materials or compounds of formula (I) contain one or more functional groups which are not stable or are reactive under the reaction conditions of one or more reaction steps, appropriate protecting groups (as described *e.g.* in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compound of formula (I) can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

The term "asymmetric carbon atom" means a carbon atom with four different substituents. According to the Cahn-Ingold-Prelog Convention an asymmetric carbon atom can be of the "R" or "S" configuration.

The invention relates in particular to:
A compound of formula (I) wherein when R¹ is 2,6-difluorophenyl, then R² is -C(O)NR³R⁴ and R³ and R⁴, together with the nitrogen atom to which they are attached, form morpholinyl;
A compound of formula (I) wherein when R¹ is n-propyl or cyclopropyl, then R² is -C(O)NR³R⁴, R³ is methyl and R⁴ is pyridinyl;
A compound of formula (I) wherein R¹ is 3-fluorophenyl, 3-chlorophenyl or 4-chlorophenyl;
A compound of formula (I) wherein one of R³ and R⁴ is alkyl and the other one is -(CH₂)ₙ-R⁶;
A compound of formula (I) wherein one of R³ and R⁴ is methyl or ethyl and the other one is -(CH₂)ₙ-R⁶;
A compound of formula (I) wherein R³ and R⁴ together with the nitrogen atom to which they are attached form heterocyclyl or substituted heterocyclyl, wherein heterocyclyl is piperidinyl, pyrrolidinyl or morpholinyl, and wherein substituted heterocyclyl is heterocyclyl substituted with one substitutent selected from alkyl and alkylcarbonylamino, or with two substituents independently selected from alkyl;
A compound of formula (I) wherein R³ and R⁴ together with the nitrogen atom to which they are attached form morpholinyl, dimethylpiperidinyl or methylcarbonylaminopyrrolidinyl;
A compound of formula (I) wherein R⁵ is tert.-butyloxycarbonylpiperidinyl, fluorophenylmethyl, ethoxycarbonylmethyl, ethoxycarbonyl(dimethyl)methyl, (methylsulfonyl)(methyl)[1,2,4]triazolyl or morpholinylcarbonylmethyl;
A compound of formula (I) wherein R⁶ is alkoxy, dialkoxyphenyl, phenyl or pyridinyl;
A compound of formula (I) wherein R⁶ is methoxy, dimethoxyphenyl, phenyl or pyridinyl;
A compound of formula (I) wherein n is 1, 2 or 3;
The invention further relates to a compound of formula (I) selected from:
   4-(3-Fluoro-phenyl)-5-oxo-4,5-dihydro-tetrazole-1-carboxylic acid [2-(3,4-dimethoxy-phenyl)-ethyl] -methyl-amide;
   4-(3-Fluoro-phenyl)-5-oxo-4,5-dihydro-tetrazole-1-carboxylic acid (2-cyano-ethyl)-methyl-amide;
   1-[4-(3-Fluoro-phenyl)-5-oxo-4,5-dihydro-tetrazole-1-carbonyl]-piperidine-3-carboxylic acid amide;
   1-(3-Fluoro-phenyl)-4-(morpholine-4-carbonyl)-1,4-dihydro-tetrazol-5-one;
   4-(3-Fluoro-phenyl)-5-oxo-4,5-dihydro-tetrazole-1-carboxylic acid methylphenethyl-amide;
   4-(3-Fluoro-phenyl)-5-oxo-4,5-dihydro-tetrazole-1-carboxylic acid ethyl-pyridin-4-ylmethyl-amide;
   4-(3-Fluoro-phenyl)-5-oxo-4,5-dihydro-tetrazole-1-carboxylic acid ethyl-(2-methoxy-ethyl)-amide;
   4-(3-Fluoro-phenyl)-5-oxo-4,5-dihydro-tetrazole-1-carboxylic acid methyl-(3-phenyl-propyl)-amide;
   1-(2-Benzyl-pyrrolidine-1-carbonyl)-4-(3-fluoro-phenyl)-1,4-dihydro-tetrazol-5-one;
   1-(3-Fluoro-phenyl)-4-[2-(4-fluoro-phenyl)-pyrrolidine-1-carbonyl]-1,4-dihydrotetrazol-5-one;
   1-(6-Fluoro-3,4-dihydro-1H-isoquinoline-2-carbonyl)-4-(3-fluoro-phenyl)-1,4-dihydro-tetrazol-5-one;
   4-(3-Fluoro-phenyl)-5-oxo-4,5-dihydro-tetrazole-1-carboxylic acid benzyl-ethyl-amide;
   (2S,3S)-2-{[4-(3-Fluoro-phenyl)-5-oxo-4,5-dihydro-tetrazole-1-carbonyl]-methyl-amino}-3-methyl-pentanoic acid methyl ester;
   1-(4-Fluoro-benzyl)-4-(3-fluoro-phenyl)-1,4-dihydro-tetrazol-5-one;
   4-[4-(3-Fluoro-phenyl)-5-oxo-4,5-dihydro-tetrazol-1-yl]-piperidine-1-carboxylic acid tert-butyl ester;
   2-[4-(3-Fluoro-phenyl)-5-oxo-4,5-dihydro-tetrazol-1-yl]-2-methyl-propionic acid ethyl ester;
   1-(3-Fluoro-phenyl)-4-(5-methanesulfonyl-4-methyl-4H-[1,2,4]triazol-3-ylmethyl)-1,4-dihydro-tetrazol-5-one;
   1-(3-Fluoro-phenyl)-4-[2-(2-methyl-2H-pyrazol-3-yl)-pyrrolidine-l-carbonyl]-1,4-dihydro-tetrazol-5-one;
   1-(3-Fluoro-phenyl)-4-[2-(1-methyl-1H-pyrazol-3-yl)-pyrrolidine-1-carbonyl]-1,4-dihydro-tetrazol-5-one;
   1-(3-Fluoro-phenyl)-4-[2-(3-methyl-isoxazol-5-yl)-pyrrolidine-1-carbonyl]-1,4-dihydro-tetrazol-5-one;
   1-(3-Fluoro-phenyl)-4-((S)-2-methoxymethyl-pyrrolidine-1-carbonyl)-1,4-dihydro-tetrazol-5-one;
   (S)-1-[4-(3-Fluoro-phenyl)-5-oxo-4,5-dihydro-tetrazole-1-carbonyl]-pyrrolidine-2-carboxylic acid amide;
   N-{1-[4-(3-Fluoro-phenyl)-5-oxo-4,5-dihydro-tetrazole-1-carbonyl]-pyrrolidin-3-yl}-acetamide;
   1-[4-(3-Fluoro-phenyl)-5-oxo-4,5-dihydro-tetrazole-1-carbonyl]-azetidine-3-carboxylic acid methyl ester;
   1-(3-Fluoro-phenyl)-4-[2-(3-methyl-[1,2,4]oxadiazol-5-yl)-pyrrolidine-1-carbonyl]-1,4-dihydro-tetrazol-5-one;
   N-{1-[4-(3-Fluoro-phenyl)-5-oxo-4,5-dihydro-tetrazole-1-carbonyl]-piperidin-4-yl}-acetamide;
   1-(3-Fluoro-phenyl)-4-[4-(2-oxo-2-pyrrolidin-1-yl-ethyl)-piperazine-1-carbonyl]-1,4-dihydro-tetrazol-5-one;
   1-(4,4-Dimethyl-piperidine-1-carbonyl)-4-(3-fluoro-phenyl)-1,4-dihydro-tetrazol-5-one;
   1-(1,1-Dioxo-1λ6-thiomorpholine-4-carbonyl)-4-(3-fluoro-phenyl)-1,4-dihydro-tetrazol-5-one;
   1-(3-Fluoro-phenyl)-4-(2,3,5,6-tetrahydro-[1,2']bipyrazinyl-4-carbonyl)-1,4-dihydro-tetrazol-5-one;
   4-(3-Fluoro-phenyl)-5-oxo-4,5-dihydro-tetrazole-1-carboxylic acid (2-cyano-ethyl)-cyclopropyl-amide;
   4-(3-Fluoro-phenyl)-5-oxo-4,5-dihydro-tetrazole-1-carboxylic acid methyl-pyridin-2-yl-amide;
   4-(3-Fluoro-phenyl)-5-oxo-4,5-dihydro-tetrazole-1-carboxylic acid methyl-pyridin-3-yl-amide;
   1-(3-Fluoro-phenyl)-4-(2-methoxymethyl-piperidine-1-carbonyl)-1,4-dihydro-tetrazol-5-one;
   4-Cyclopropyl-5-oxo-4,5-dihydro-tetrazole-1-carboxylic acid methyl-pyridin-3-yl-amide;
   5-Oxo-4-propyl-4,5-dihydro-tetrazole-1-carboxylic acid methyl-pyridin-3-yl-amide;
   4-Isobutyl-5-oxo-4,5-dihydro-tetrazole-1-carboxylic acid methyl-pyridin-3-yl-amide;
   1-(4-Acetyl-piperazine-1-carbonyl)-4-(3-fluoro-phenyl)-1,4-dihydro-tetrazol-5-one;
   1-(3-Fluoro-phenyl)-4-(4-propionyl-piperazine-1-carbonyl)-1,4-dihydro-tetrazol-5-one;
   4-[4-(3-Fluoro-phenyl)-5-oxo-4,5-dihydro-tetrazole-1-carbonyl]-piperazine-1-carboxylic acid diethylamide;
   1-(3-Fluoro-phenyl)-4-(3-phenyl-pyrrolidine-1-carbonyl)-1,4-dihydro-tetrazol-5-one;
   N-{(S)-1-[4-(3-Fluoro-phenyl)-5-oxo-4,5-dihydro-tetrazole-1-carbonyl]-pyrrolidin-3-yl}-acetamide;
   N-{(R)-1-[4-(3-Fluoro-phenyl)-5-oxo-4,5-dihydro-tetrazole-1-carbonyl]-pyrrolidin-3-yl}-acetamide;
   N-Ethyl-N-{1-[4-(3-fluoro-phenyl)-5-oxo-4,5-dihydro-tetrazole-1-carbonyl]-pyrrolidin-3-yl}-acetamide;
   1-(3,3-Difluoro-pyrrolidine-1-carbonyl)-4-(3-fluoro-phenyl)-1,4-dihydro-tetrazol-5-one;
   1-(2,2-Dimethyl-pyrrolidine-l-carbonyl)-4-(3-fluoro-phenyl)-1,4-dihydro-tetrazol-5-one;
   1-(3,3-Dimethyl-pyrrolidine-1-carbonyl)-4-(3-fluoro-phenyl)-1,4-dihydro-tetrazol-5-one;
   1-(3-Fluoro-phenyl)-4-(2-methyl-pyrrolidine-1-carbonyl)-1,4-dihydro-tetrazol-5-one;
   N-{1-[4-(3-Fluoro-phenyl)-5-oxo-4,5-dihydro-tetrazole-1-carbonyl]-pyrrolidin-3-yl}-N-methyl-acetamide;
   4-[4-(3-Fluoro-phenyl)-5-oxo-4,5-dihydro-tetrazole-1-carbonyl]-piperazine-1-carboxylic acid ethyl ester;
   (S)-1-[4-(3-Fluoro-phenyl)-5-oxo-4,5-dihydro-tetrazole-1-carbonyl]-pyrrolidine-2-carbonitrile;
   (R)-1-[4-(3-Fluoro-phenyl)-5-oxo-4,5-dihydro-tetrazole-1-carbonyl]-pyrrolidine-2-carbonitrile;
   4-(6-Chloro-pyridin-3-yl)-5-oxo-4,5-dihydro-tetrazole-1-carboxylic acid benzyl-ethyl-amide;
   4-(3-Fluoro-phenyl)-5-oxo-4,5-dihydro-tetrazole-1-carboxylic acid (2-methoxyethyl)-methyl-amide;
   4-(3-Fluoro-phenyl)-5-oxo-4,5-dihydro-tetrazole-1-carboxylic acid isopropyl-(2-methoxy-ethyl)-amide;
   4-(3-Fluoro-phenyl)-5-oxo-4,5-dihydro-tetrazole-1-carboxylic acid (2-methoxyethyl)-propyl-amide;
   4-(3-Fluoro-phenyl)-5-oxo-4,5-dihydro-tetrazole-1-carboxylic acid bis-(2-methoxyethyl)-amide;
   1-(2,6-Dimethyl-morpholine-4-carbonyl)-4-(3-fluoro-phenyl)-1,4-dihydro-tetrazol-5-one;
   1-((2S,6R)-2,6-Dimethyl-morpholine-4-carbonyl)-4-(3-fluoro-phenyl)-1,4-dihydro-tetrazol-5-one;
   1-(3-Fluoro-phenyl)-4-(4-methoxymethyl-piperidine-1-carbonyl)-1,4-dihydro-tetrazol-5-one;
   1-[4-(3-Fluoro-phenyl)-5-oxo-4,5-dihydro-tetrazole-1-carbonyl]-piperidine-4-carbonitrile;
   4-tert-Butoxycarbonylamino-1-[4-(3-fluoro-phenyl)-5-oxo-4,5-dihydro-tetrazole-1-carbonyl]-piperidine-4-carboxylic acid methyl ester;
   1-(3,3-Dimethyl-piperidine-1-carbonyl)-4-(3-fluoro-phenyl)-1,4-dihydro-tetrazol-5-one;
   1-(3-Fluoro-phenyl)-4-(2-oxa-8-aza-spiro[4.5]decane-8-carbonyl)-1,4-dihydro-tetrazol-5-one;
   N-{(R)-1-[4-(3-Chloro-phenyl)-5-oxo-4,5-dihydro-tetrazole-1-carbonyl]-pyrrolidin-3-yl}-acetamide;
   N-{(R)-1-[5-Oxo-4-(3-trifluoromethoxy-phenyl)-4,5-dihydro-tetrazole-1-carbonyl]-pyrrolidin-3-yl}-acetamide;
   N-{(R)-1-[4-(4-Chloro-phenyl)-5-oxo-4,5-dihydro-tetrazole-1-carbonyl]-pyrrolidin-3-yl}-acetamide;
   N-{(S)-1-[4-(3-Chloro-phenyl)-5-oxo-4,5-dihydro-tetrazole-1-carbonyl]-pyrrolidin-3-yl}-acetamide;
   N-{(S)-1-[5-Oxo-4-(3-trifluoromethoxy-phenyl)-4,5-dihydro-tetrazole-1-carbonyl]-pyrrolidin-3-yl}-acetamide;
   N-{(S)-1-[4-(4-Chloro-phenyl)-5-oxo-4,5-dihydro-tetrazole-1-carbonyl]-pyrrolidin-3-yl}-acetamide;
   N-{1-[4-(3-Chloro-phenyl)-5-oxo-4,5-dihydro-tetrazole-1-carbonyl]-pyrrolidin-3-yl}-N-ethyl-acetamide;
   N-Ethyl-N-{1-[5-oxo-4-(3-trifluoromethoxy-phenyl)-4,5-dihydro-tetrazole-1-carbonyl]-pyrrolidin-3-yl}-acetamide;
   N-{1-[4-(4-Chloro-phenyl)-5-oxo-4,5-dihydro-tetrazole-1-carbonyl]-pyrrolidin-3-yl}-N-ethyl-acetamide;
   1-(3-Chloro-phenyl)-4-[2-(3-methyl-isoxazol-5-yl)-pyrrolidine-1-carbonyl]-1,4-dihydro-tetrazol-5-one;
   1-[2-(3-Methyl-isoxazol-5-yl)-pyrrolidine-1-carbonyl]-4-(3-trifluoromethoxyphenyl)-1,4-dihydro-tetrazol-5-one;
   1-(4-Chloro-phenyl)-4-[2-(3-methyl-isoxazol-5-yl)-pyrrolidine-1-carbonyl]-1,4-dihydro-tetrazol-5-one;
   1-(3-Chloro-phenyl)-4-(4,4-dimethyl-piperidine-1-carbonyl)-1,4-dihydro-tetrazol-5-one;
   1-(4,4-Dimethyl-piperidine-1-carbonyl)-4-(3-trifluoromethoxy-phenyl)-1,4-dihydro-tetrazol-5-one;
   1-(4-Chloro-phenyl)-4-(4,4-dimethyl-piperidine-1-carbonyl)-1,4-dihydro-tetrazol-5-one;
   4-(3-Chloro-phenyl)-5-oxo-4,5-dihydro-tetrazole-1-carboxylic acid ethyl-(2-methoxy-ethyl)-amide;
   5-Oxo-4-(3-trifluoromethoxy-phenyl)-4,5-dihydro-tetrazole-1-carboxylic acid ethyl-(2-methoxy-ethyl)-amide;
   4-(4-Chloro-phenyl)-5-oxo-4,5-dihydro-tetrazole-1-carboxylic acid ethyl-(2-methoxy-ethyl)-amide;
   4-(3-Chloro-phenyl)-5-oxo-4,5-dihydro-tetrazole-1-carboxylic acid isopropyl-(2-methoxy-ethyl)-amide;
   5-Oxo-4-(3-trifluoromethoxy-phenyl)-4,5-dihydro-tetrazole-1-carboxylic acid isopropyl-(2-methoxy-ethyl)-amide;
   4-(4-Chloro-phenyl)-5-oxo-4,5-dihydro-tetrazole-1-carboxylic acid isopropyl-(2-methoxy-ethyl)-amide;
   4-(3-Chloro-phenyl)-5-oxo-4,5-dihydro-tetrazole-1-carboxylic acid (3-methoxypropyl)-methyl-amide;
   5-Oxo-4-(3-trifluoromethoxy-phenyl)-4,5-dihydro-tetrazole-1-carboxylic acid (3-methoxy-propyl)-methyl-amide;
   4-(4-Chloro-phenyl)-5-oxo-4,5-dihydro-tetrazole-1-carboxylic acid (3-methoxypropyl)-methyl-amide;
   1-(3-Chloro-phenyl)-4-[4-(2-morpholin-4-yl-2-oxo-ethyl)-piperazine-1-carbonyl]-1,4-dihydro-tetrazol-5-one;
   1-[4-(2-Morpholin-4-yl-2-oxo-ethyl)-piperazine-1-carbonyl]-4-(3-trifluoromethoxyphenyl)-1,4-dihydro-tetrazol-5-one;
   1-(4-Chloro-phenyl)-4-[4-(2-morpholin-4-yl-2-oxo-ethyl)-piperazine-1-carbonyl]-1,4-dihydro-tetrazol-5-one;
   2-{4-[4-(3-Chloro-phenyl)-5-oxo-4,5-dihydro-tetrazole-1-carbonyl]-piperazin-1-yl}-N,N-dimethyl-acetamide;
   N,N-Dimethyl-2-{4-[5-oxo-4-(3-trifluoromethoxy-phenyl)-4,5-dihydro-tetrazole-1-carbonyl]-piperazin-1-yl}-acetamide;
   2-{4-[4-(4-Chloro-phenyl)-5-oxo-4,5-dihydro-tetrazole-1-carbonyl]-piperazin-1-yl}-N,N-dimethyl-acetamide;
   1-(3-Chloro-phenyl)-4-[4-(2-oxo-2-pyrrolidin-1-yl-ethyl)-piperazine-1-carbonyl]-1,4-dihydro-tetrazol-5-one;
   1-[4-(2-Oxo-2-pyrrolidin-1-yl-ethyl)-piperazine-1-carbonyl]-4-(3-trifluoromethoxyphenyl)-1,4-dihydro-tetrazol-5-one;
   1-(4-Chloro-phenyl)-4-[4-(2-oxo-2-pyrrolidin-1-yl-ethyl)-piperazine-1-carbonyl]-1,4-dihydro-tetrazol-5-one;
   4-(3-Chloro-phenyl)-5-oxo-4,5-dihydro-tetrazole-1-carboxylic acid (2-ethoxy-ethyl)-ethyl-amide;
   5-Oxo-4-(3-trifluoromethoxy-phenyl)-4,5-dihydro-tetrazole-1-carboxylic acid (2-ethoxy-ethyl)-ethyl-amide;
   4-(4-Chloro-phenyl)-5-oxo-4,5-dihydro-tetrazole-1-carboxylic acid (2-ethoxy-ethyl)-ethyl-amide;
   1-(3-Chloro-phenyl)-4-(morpholine-4-carbonyl)-1,4-dihydro-tetrazol-5-one
   1-(Morpholine-4-carbonyl)-4-(3-trifluoromethoxy-phenyl)-1,4-dihydro-tetrazol-5-one
   1-(4-Chloro-phenyl)-4-(morpholine-4-carbonyl)-1,4-dihydro-tetrazol-5-one;
   2-{4-[4-(3-Chloro-phenyl)-5-oxo-4,5-dihydro-tetrazole-1-carbonyl]-piperazin-1-yl}-N-ethyl-acetamide;
   N-Ethyl-2-{4-[5-oxo-4-(3-trifluoromethoxy-phenyl)-4,5-dihydro-tetrazole-1-carbonyl]-piperazin-1-yl}-acetamide;
   2-{4-[4-(4-Chloro-phenyl)-5-oxo-4,5-dihydro-tetrazole-1-carbonyl]-piperazin-1-yl}-N-ethyl-acetamide;
   N-Butyl-2-{4-[4-(3-chloro-phenyl)-5-oxo-4,5-dihydro-tetrazole-1-carbonyl]-piperazin-1-yl}-acetamide;
   N-Butyl-2-{4-[5-oxo-4-(3-trifluoromethoxy-phenyl)-4,5-dihydro-tetrazole-1-carbonyl]-piperazin-1-yl}-acetamide;
   N-Butyl-2-{4-[4-(4-chloro-phenyl)-5-oxo-4,5-dihydro-tetrazole-1-carbonyl]-piperazin-1-yl}-acetamide;
   4-(3-Fluoro-phenyl)-5-oxo-4,5-dihydro-tetrazole-1-carboxylic acid (2-methoxyethyl)-amide;
   4-(3-Chloro-phenyl)-5-oxo-4,5-dihydro-tetrazole-1-carboxylic acid (2-ethoxy-ethyl)-methyl-amide;
   5-Oxo-4-(3-trifluoromethoxy-phenyl)-4,5-dihydro-tetrazole-1-carboxylic acid (2-ethoxy-ethyl)-methyl-amide;
   4-(4-Chloro-phenyl)-5-oxo-4,5-dihydro-tetrazole-1-carboxylic acid (2-ethoxy-ethyl)-methyl-amide;
   [4-(3-Chloro-phenyl)-5-oxo-4,5-dihydro-tetrazol-1-yl]-acetic acid ethyl ester;
   1-(3-Chloro-phenyl)-4-(2-morpholin-4-yl-2-oxo-ethyl)-1,4-dihydro-tetrazol-5-one;
   1-(3-Chloro-phenyl)-4-[4-(1-methyl-2-oxo-2-pyrrolidin-1-yl-ethyl)-piperazine-1-carbonyl]-1,4-dihydro-tetrazol-5-one;
   1-[4-(1-Methyl-2-oxo-2-pyrrolidin-1-yl-ethyl)-piperazine-1-carbonyl]-4-(3-trifluoromethoxy-phenyl)-1,4-dihydro-tetrazol-5-one;
   1-(4-Chloro-phenyl)-4-[4-(1-methyl-2-oxo-2-pyrrolidin-1-yl-ethyl)-piperazine-1-carbonyl]-1,4-dihydro-tetrazol-5-one;
   1-(morpholine-4-carbonyl)-4-[2-(trifluoromethyl)phenyl]tetrazol-5-one;
   1-(morpholine-4-carbonyl)-4-[3-(trifluoromethyl)phenyl]tetrazol-5-one;
   1-(morpholine-4-carbonyl)-4-[4-(trifluoromethyl)phenyl]tetrazol-5-one;
   1-(2,6-difluorophenyl)-4-(morpholine-4-carbonyl)tetrazol-5-one;
   1-(2,4-difluorophenyl)-4-(morpholine-4-carbonyl)tetrazol-5-one;
   1-(2,5-difluorophenyl)-4-(morpholine-4-carbonyl)tetrazol-5-one;
   1-(3,4-difluorophenyl)-4-(morpholine-4-carbonyl)tetrazol-5-one;
   1-(3,5-difluorophenyl)-4-(morpholine-4-carbonyl)tetrazol-5-one;
   1-(3-methylphenyl)-4-(morpholine-4-carbonyl)tetrazol-5-one;
   1-(4-methylphenyl)-4-(morpholine-4-carbonyl)tetrazol-5-one;
   1-(3-methoxyphenyl)-4-(morpholine-4-carbonyl)tetrazol-5-one;
   1-(4-methoxyphenyl)-4-(morpholine-4-carbonyl)tetrazol-5-one;
   1-(2,3-dichlorophenyl)-4-(morpholine-4-carbonyl)tetrazol-5-one;
   1-(1-methylindol-4-yl)-4-(morpholine-4-carbonyl)tetrazol-5-one;
   1-(1-methylindol-5-yl)-4-(morpholine-4-carbonyl)tetrazol-5-one;
   1-(1-methylindol-6-yl)-4-(morpholine-4-carbonyl)tetrazol-5-one;
   1-(3-bromophenyl)-4-(morpholine-4-carbonyl)tetrazol-5-one;
   1-(3-chlorophenyl)-4-[(2R,6S)-2,6-dimethylmorpholine-4-carbonyl]tetrazol-5-one;
   1-(3-chlorophenyl)-4-(2,6-dimethylmorpholine-4-carbonyl)tetrazol-5-one; and 1-(3-chlorophenyl)-4-(3,3-dimethylpyrrolidine-1-carbonyl)tetrazol-5-one.

The invention further relates to a compound of formula (I) selected from:
4-(3-Fluoro-phenyl)-5-oxo-4,5-dihydro-tetrazole-1-carboxylic acid [2-(3,4-dimethoxy-phenyl)-ethyl] -methyl-amide;
4-(3-Fluoro-phenyl)-5-oxo-4,5-dihydro-tetrazole-1-carboxylic acid methylphenethyl-amide;
4-(3-Fluoro-phenyl)-5-oxo-4,5-dihydro-tetrazole-1-carboxylic acid ethyl-pyridin-4-ylmethyl-amide;
4-(3-Fluoro-phenyl)-5-oxo-4,5-dihydro-tetrazole-1-carboxylic acid benzyl-ethyl-amide;
1-(4,4-Dimethyl-piperidine-1-carbonyl)-4-(3-fluoro-phenyl)-1,4-dihydro-tetrazol-5-one;
N-{(R)-1-[4-(3-Fluoro-phenyl)-5-oxo-4,5-dihydro-tetrazole-1-carbonyl]-pyrrolidin-3-yl}-acetamide;
1-(3-Chloro-phenyl)-4-(4,4-dimethyl-piperidine-1-carbonyl)-1,4-dihydro-tetrazol-5-one;
1-(4-Chloro-phenyl)-4-(4,4-dimethyl-piperidine-1-carbonyl)-1,4-dihydro-tetrazol-5-one;
4-(3-Chloro-phenyl)-5-oxo-4,5-dihydro-tetrazole-1-carboxylic acid (3-methoxypropyl)-methyl-amide; and
1-(3-Chloro-phenyl)-4-(morpholine-4-carbonyl)-1,4-dihydro-tetrazol-5-one.

The preparation of compounds of formula (I) of the present invention may be carried out in sequential or convergent synthetic routes. Syntheses of the compounds of the invention are shown in the following schemes. The skills required for carrying out the reactions and purifications of the resulting products are known to those skilled in the art. The substituents and indices used in the following description of the processes have the significance given herein before unless indicated to the contrary. In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 2nd Edition, Richard C. Larock. John Wiley & Sons, New York, NY. 1999). We found it convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 h to several days will usually suffice to yield the described intermediates and compounds. The reaction sequence is not limited to the one displayed in the scheme, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art. 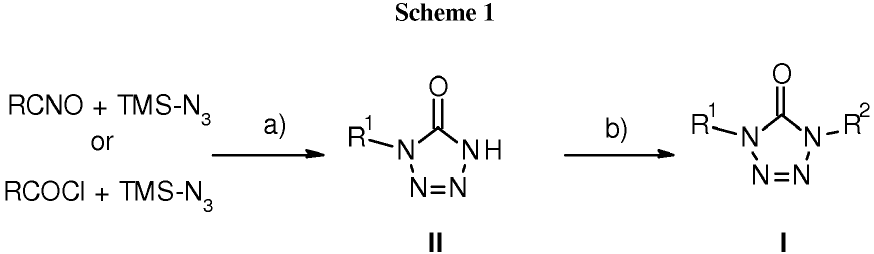
a) Tetrazolones **II** are either commercially available or can be synthesized according to methods known in the art. Conveniently, isocyanate derivatives are reacted with azido trimethyl silane (in analogy to: J. Org. Chem. 1980, 45, 5130-5136) or acid chloride derivatives are reacted with azido trimethyl silane (in analogy to: J. Org. Chem. 1995, 60, 7641-7645) to access tetrazolone derivatives **II**.
b) In case R² is R⁵, tetrazolones **II** are conveniently reacted with an electrophile under basic conditions (e.g. Cs₂CO₃) to yield tetrazolone derivatives **I.** These derivatives are either final compounds or conveniently further reacted to access final tetrazolones **I.** In case: R² is -C(O)NR³R⁴, tetrazolones **II** are conveniently reacted with a C1-equivalent (e.g. phosgene or diphosgene) optionally in the presence of a base and subsequently with a suitable amine derivative or optionally with a suitable carbamoyl chloride in the presence of a base to access tetrazolone derivatives **I.** These derivatives are either final compounds or conveniently further reacted to access final tetrazolones **I.**

The invention also relates to a process for the preparation of a compound of formula (I), comprising the reaction of a compound of formula (A) 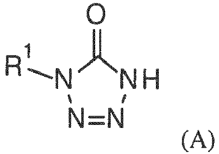
(a) in the presence of R⁵-X and a base; or
(b) in the presence of phosgene, diphosgene or triphosgene followed by reaction in the presence of HNR³R⁴ and a base;
wherein X is an electron withdrawing group and wherein R¹, R³, R⁴ and R⁵ are as defined above.

X is for example chloride or bromide.

In step (a), the base is for example DMAP or K₂CO₃.

In step (b), the base is for example Cs₂CO₃ or K₂CO₃.

The invention also relates to a compound of formula (I) when manufactured according to the above process.

The invention also relates in particular to a compound of formula (I) for use in the treatment or prophylaxis of pain, atherosclerosis, age-related macular degeneration, diabetic retinopathy, glaucoma, retinal vein occlusion, retinopathy of prematurity, ocular ischemic syndrome, geographic atrophy, diabetes mellitus, inflammation, inflammatory bowel disease, ischemia-reperfusion injury, acute liver failure, liver fibrosis, lung fibrosis, kidney fibrosis, systemic fibrosis, acute allograft rejection, chronic allograft nephropathy, diabetic nephropathy, glomerulonephropathy, cardiomyopathy, heart failure, myocardial ischemia, myocardial infarction, systemic sclerosis, thermal injury, burning, hypertrophic scars, keloids, gingivitis pyrexia, liver cirrhosis or tumors, regulation of bone mass, amyotrophic lateral sclerosis, multiple sclerosis, Alzheimer's disease, Parkinson's disease, stroke, transient ischemic attack or uveitis; and

The invention particularly relates to a compound of formula (I) for use in the treatment or prophylaxis of ischemia, reperfusion injury, liver fibrosis or kidney fibrosis, in particular ischemia or reperfusion injury.

The invention further particularly relates to a compound of formula (I) for use in the treatment or prophylaxis of age-related macular degeneration, diabetic retinopathy, glaucoma, retinal vein occlusion, retinopathy of prematurity, ocular ischemic syndrome, geographic atrophy or uveitis.

The invention further particularly relates to a compound of formula (I) for use in the treatment or prophylaxis of amyotrophic lateral sclerosis or multiple sclerosis.

The invention is further directed to a compound of formula (I), when manufactured according to a process according to the invention.

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula (I) may be formulated by mixing at ambient temperature at the appropriate pH, and at the desired degree of purity, with physiologically acceptable carriers, i.e., carriers that are nontoxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compound of formula (I) is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The invention will now be illustrated by the following examples which have no limiting character.

### Examples

### Example 1

### 4-(3-Fluoro-phenyl)-5-oxo-4,5-dihydro-tetrazole-1-carboxylic acid [2-(3,4-dimethoxyphenyl)-ethyl]-methyl-amide

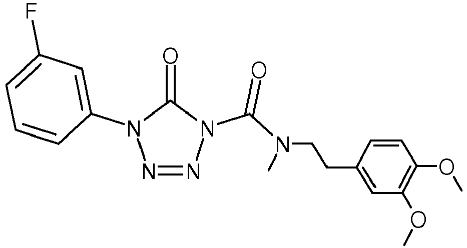

### a) 1-(3-Fluoro-phenyl)-1,4-dihydro-tetrazol-5-one

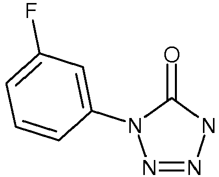

A mixture of 1-fluoro-3-isocyanatobenzene (2 g, 14.6 mmol) and TMS-N₃ (2.52 g, 2.9 ml, 21.9 mmol) were heated to 100 °C for 18 h. Excess TMS-N₃ was removed under vacuum and the residue taken up in 1 mL wet MeOH. The precipitate was filtered off, washed with water and dried under high vacuum to yield 2.42 g (92 %) of the title compound as white crystals. MS(m/e): 222.1 (MH⁺+CH₃CN).

### b) 4-(3-Fluoro-phenyl)-5-oxo-4,5-dihydro-tetrazole-1-carboxylic acid [2-(3,4-dimethoxyphenyl)-ethyl]-methyl-amide

A mixture of 1-(3-fluorophenyl)-1H-tetrazol-5(4H)-one (30 mg, 167 µmol) and DMAP (50.9 mg, 416 µmol) in DCM (8 mL) was treated at 0 °C with trichloromethyl carbonochloridate (39.5 mg, 24.7 µl, 200 µmol) and shaken for 30 min. 2-(3,4-dimethoxyphenyl)-N-methylethanamine (51.9 mg, 266 µmol) was added and shaken at room temperature over -night. The volatiles were removed and DMF and formic acid was added and the mixture was subjected to purification by preparative HPLC on reversed phase eluting with a gradient formed from acetonitrile, water and formic acid. The product containing fractions were evaporated to yield 18 mg (27 %) of the title compound. MS(m/e): 402.3 (MH⁺).

### Example 2

### 4-(3-Fluoro-phenyl)-5-oxo-4,5-dihydro-tetrazole-1-carboxylic acid (2-cyano-ethyl)-methyl-amide

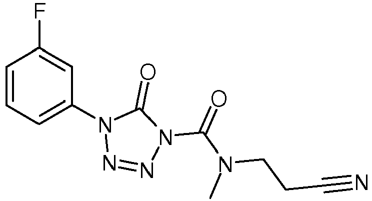

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3-(methylamino)propanenitrile. MS(m/e): 291.2 (MH⁺).

### Example 3

### 1-[4-(3-Fluoro-phenyl)-5-oxo-4,5-dihydro-tetrazole-1-carbonyl]-piperidine-3-carboxylic acid amide

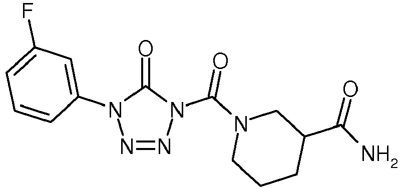

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and piperidine-3-carboxamide. MS(m/e): 334.2 (MH⁺).

### Example 4

### 1-(3-Fluoro-phenyl)-4-(morpholine-4-carbonyl)-1,4-dihydro-tetrazol-5-one

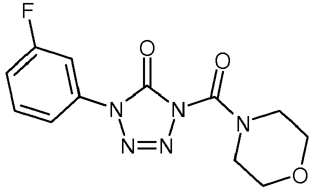

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and morpholine. MS(m/e): 294.2 (MH⁺).

### Example 5

### 4-(3-Fluoro-phenyl)-5-oxo-4,5-dihydro-tetrazole-1-carboxylic acid methyl-phenethyl-amide

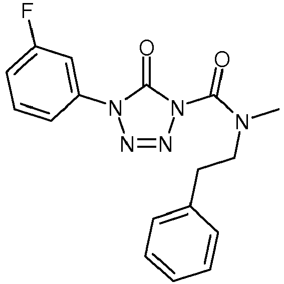

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-methyl-2-phenylethanamine. MS(m/e): 342.2 (MH⁺).

### Example 6

### 4-(3-Fluoro-phenyl)-5-oxo-4,5-dihydro-tetrazole-1-carboxylic acid ethyl-pyridin-4-ylmethyl-amide

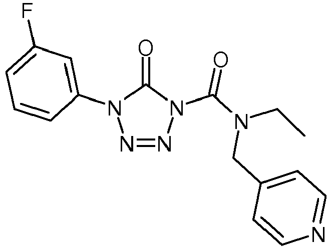

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-(pyridin-4-ylmethyl)ethanamine. MS(m/e): 342.2 (MH⁺).

### Example 7

### 4-(3-Fluoro-phenyl)-5-oxo-4,5-dihydro-tetrazole-1-carboxylic acid ethyl-(2-methoxyethyl)-amide

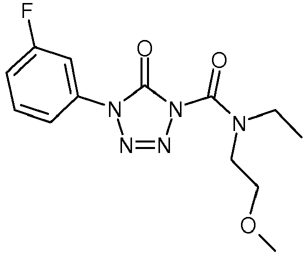

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-ethyl-2-methoxyethanamine. MS(m/e): 310.2 (MH⁺).

### Example 8

### 4-(3-Fluoro-phenyl)-5-oxo-4,5-dihydro-tetrazole-1-carboxylic acid methyl-(3-phenylpropyl)-amide

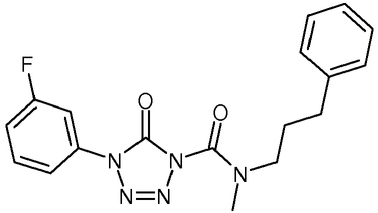

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-methyl-3-phenylpropan-1-amine. MS(m/e): 356.3 (MH⁺).

### Example 9

### 1-(2-Benzyl-pyrrolidine-1-carbonyl)-4-(3-fluoro-phenyl)-1,4-dihydro-tetrazol-5-one

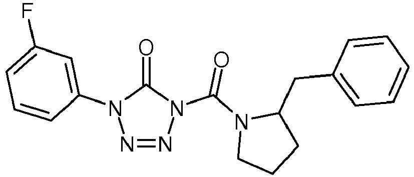

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-benzylpyrrolidine. MS(m/e): 368.3 (MH⁺).

### Example 10

### 1-(3-Fluoro-phenyl)-4-[2-(4-fluoro-phenyl)-pyrrolidine-1-carbonyl]-1,4-dihydro-tetrazol-5-one

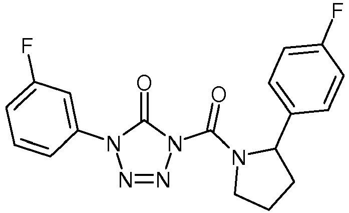

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-(4-fluorophenyl)pyrrolidine. MS(m/e): 372.2 (MH⁺).

### Example 11

### 1-(6-Fluoro-3,4-dihydro-1H-isoquinoline-2-carbonyl)-4-(3-fluoro-phenyl)-1,4-dihydro-tetrazol-5-one

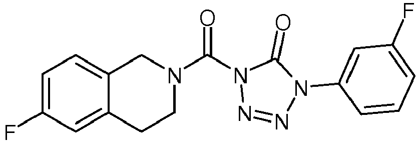

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 6-fluoro-1,2,3,4-tetrahydroisoquinoline. MS(m/e): 358.2 (MH⁺).

### Example 12

### 4-(3-Fluoro-phenyl)-5-oxo-4,5-dihydro-tetrazole-1-carboxylic acid benzyl-ethyl-amide

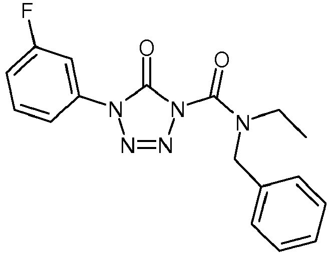

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-benzylethanamine. MS(m/e): 342.2 (MH⁺).

### Example 13

### (2S,3S)-2-{[4-(3-Fluoro-phenyl)-5-oxo-4,5-dihydro-tetrazole-1-carbonyl]-methyl-amino}-3-methyl-pentanoic acid methyl ester

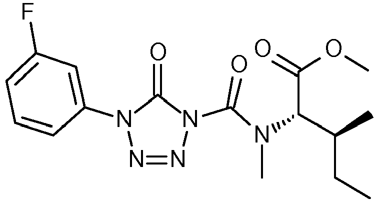

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (2S,3S)-methyl 3-methyl-2-(methylamino)pentanoate hydrochloride. MS(m/e): 366.3 (MH⁺).

### Example 14

### 1-(4-Fluoro-benzyl)-4-(3-fluoro-phenyl)-1,4-dihydro-tetrazol-5-one

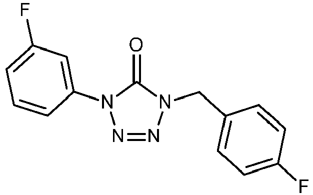

A mixture of 1-(3-fluorophenyl)-1H-tetrazol-5(4H)-one (30 mg, 167 µmol) and Cs₂CO₃ (57.0 mg, 175 µmol) in DMF (2 mL) was treated with 1-(bromomethyl)-4-fluorobenzene (32.4 mg, 21.1 µl, 172 µmol) at room temperature. The mixture was diluted with water and the suspension filtered. The precipitate was washed with water and dried under high vacuum to yield 38 mg (78 %) of the title compound as white powder. MS(m/e): 288 (MH⁺).

### Example 15

### 4-[4-(3-Fluoro-phenyl)-5-oxo-4,5-dihydro-tetrazol-1-yl]-piperidine-1-carboxylic acid tert-butyl ester

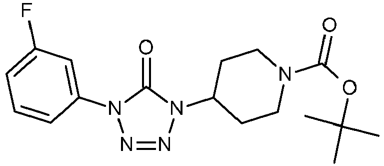

A mixture of 1-(3-fluorophenyl)-1H-tetrazol-5(4H)-one (55 mg, 305 µmol), DMAP (39.2 mg, 321 µmol) and tert-butyl 4-bromopiperidine-1-carboxylate (80.7 mg, 305 µmol) in DMF (1 mL) was shaken at room temperature for 24 h. Cs₂CO₃ (99.5 mg, 305 µmol) was added and the mixture shaken at 85 °C for 16h. After cooling to room temperature the mixture subjected to preparative HPLC separation on reversed phase eluting with a gradient formed from acetonitrile, water and NEt₃. Evaporation of the product containing fractions yielded 13.9 mg (12 %) of the title compound as amorphous solid. MS(m/e): 364 (MH⁺).

### Example 16

### 2-[4-(3-Fluoro-phenyl)-5-oxo-4,5-dihydro-tetrazol-1-yl]-2-methyl-propionic acid ethyl ester

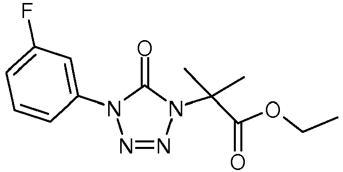

A mixture of 1-(3-fluorophenyl)-1H-tetrazol-5(4H)-one (80 mg, 444 µmol) and Cs₂CO₃ (289 mg, 888 µmol) in DMF (2 mL) was shaken at room temperature for 15 min and subsequently treated with ethyl 2-bromo-2-methylpropanoate (95.3 mg, 72.5 µl, 489 µmol) and reacted at room temperature. The precipitate was filtered off and the filtrate subjected to purification by preparative HPLC eluting with a gradient formed from acetonitrile, water and NEt₃. The product containing fractions were evaporated to yield 28 mg (21 %) of the title compound as colorless liquid. MS(m/e): 295.2 (MH⁺).

### Example 17

### 1-(3-Fluoro-phenyl)-4-(5-methanesulfonyl-4-methyl-4H-[1,2,4]triazol-3-ylmethyl)-1,4-dihydro-tetrazol-5-one

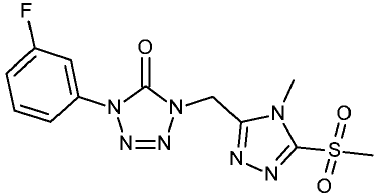

A mixture of 1-(3-fluorophenyl)-1H-tetrazol-5(4H)-one (15.2 mg, 84.4 µmol) and K₂CO₃ (23.3 mg, 169 µmol) in DMF (1000 µL) was reacted with 3-(iodomethyl)-4-methyl-5-(methylsulfonyl)-4H-1,2,4-triazole (25.4 mg, 84.4 µmol) and stirred at room temperature overnight. The mixture was filtered and subjected to purification by preparative HPLC eluting with a gradient formed from acetonitrile, water and NEt₃. The product containing fractions were evaporated to yield 18 mg (62 %) of the title compound as colorless liquid. MS(m/e): 354.3 (MH⁺).

### Example 18

### 1-(3-Fluoro-phenyl)-4-[2-(2-methyl-2H-pyrazol-3-yl)-pyrrolidine-1-carbonyl]-1,4-dihydro-tetrazol-5-one

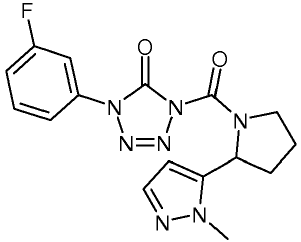

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 1-methyl-5-(pyrrolidin-2-yl)-1H-pyrazole. MS(m/e): 358.3 (MH⁺).

### Example 19

### 1-(3-Fluoro-phenyl)-4-[2-(1-methyl-1H-pyrazol-3-yl)-pyrrolidine-1-carbonyl]-1,4-dihydro-tetrazol-5-one

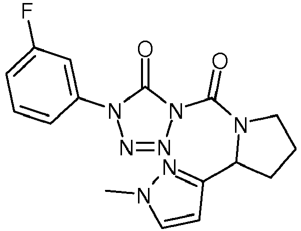

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 1-methyl-3-(pyrrolidin-2-yl)-1H-pyrazole. MS(m/e): 358.2 (MH⁺).

### Example 20

### 1-(3-Fluoro-phenyl)-4-[2-(3-methyl-isoxazol-5-yl)-pyrrolidine-1-carbonyl]-1,4-dihydro-tetrazol-5-one

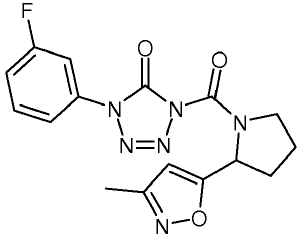

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3-methyl-5-(pyrrolidin-2-yl)isoxazole. MS(m/e): 359.2 (MH⁺).

### Example 21

### 1-(3-Fluoro-phenyl)-4-((S)-2-methoxymethyl-pyrrolidine-1-carbonyl)-1,4-dihydro-tetrazol-5-one

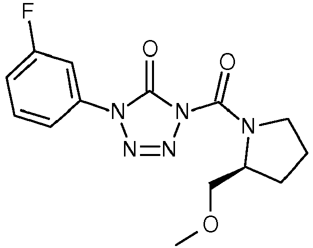

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (S)-2-(methoxymethyl)pyrrolidine. MS(m/e): 322.1 (MH⁺).

### Example 22

### (S)-1-[4-(3-Fluoro-phenyl)-5-oxo-4,5-dihydro-tetrazole-1-carbonyl]-pyrrolidine-2-carboxylic acid amide

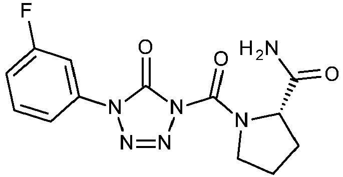

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (S)-pyrrolidine-2-carboxamide.

### Example 23

### N-{1-[4-(3-Fluoro-phenyl)-5-oxo-4,5-dihydro-tetrazole-1-carbonyl]-pyrrolidin-3-yl}-acetamide

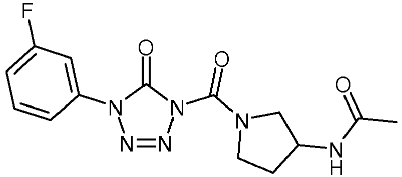

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-(pyrrolidin-3-yl)acetamide. MS(m/e): 335.2 (MH⁺).

### Example 24

### 1-[4-(3-Fluoro-phenyl)-5-oxo-4,5-dihydro-tetrazole-1-carbonyl]-azetidine-3-carboxylic acid methyl ester

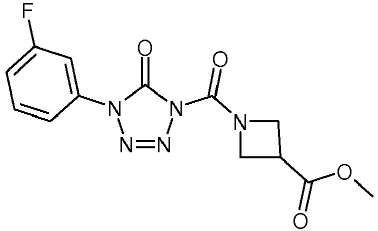

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and methyl azetidine-3-carboxylate. MS(m/e): 322.1 (MH⁺).

### Example 25

### 1-(3-Fluoro-phenyl)-4-[2-(3-methyl-[1,2,4]oxadiazol-5-yl)-pyrrolidine-1-carbonyl]-1,4-dihydro-tetrazol-5-one

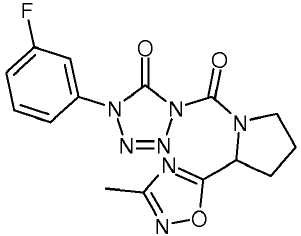

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3-methyl-5-(pyrrolidin-2-yl)-1,2,4-oxadiazole. MS(m/e): 360.2 (MH⁺).

### Example 26

### N-{1-[4-(3-Fluoro-phenyl)-5-oxo-4,5-dihydro-tetrazole-1-carbonyl]-piperidin-4-yl}-acetamide

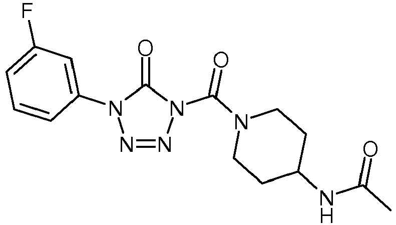

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-(piperidin-4-yl)acetamide. MS(m/e): 349.3 (MH⁺).

### Example 27

### 1-(3-Fluoro-phenyl)-4-[4-(2-oxo-2-pyrrolidin-1-yl-ethyl)-piperazine-1-carbonyl]-1,4-dihydro-tetrazol-5-one

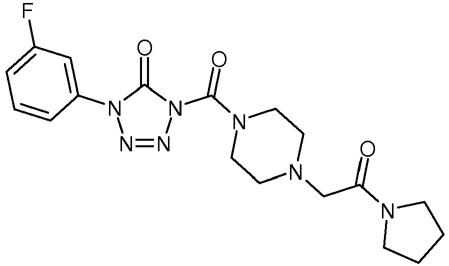

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-(piperazin-1-yl)-1-(pyrrolidin-1-yl)ethanone. MS(m/e): 404.3 (MH⁺).

### Example 28

### 1-(4,4-Dimethyl-piperidine-1-carbonyl)-4-(3-fluoro-phenyl)-1,4-dihydro-tetrazol-5-one

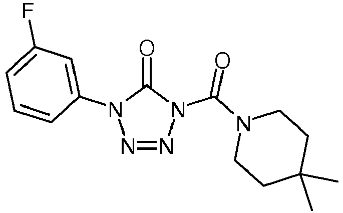

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 4,4-dimethylpiperidine. MS(m/e): 320.2 (MH⁺).

### Example 29

### 1-(1,1-Dioxo-1λ6-thiomorpholine-4-carbonyl)-4-(3-fluoro-phenyl)-1,4-dihydro-tetrazol-5-one

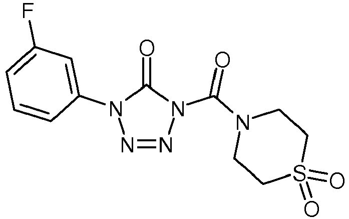

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and thiomorpholine 1,1-dioxide. MS(m/e): 359.0 (MH⁺+NH₄).

### Example 30

### 1-(3-Fluoro-phenyl)-4-(2,3,5,6-tetrahydro-[1,2']bipyrazinyl-4-carbonyl)-1,4-dihydro-tetrazol-5-one

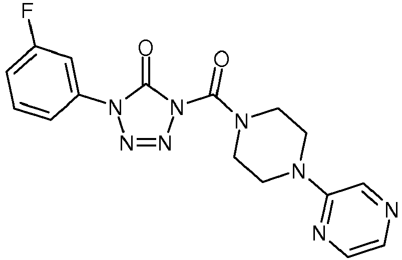

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-(piperazin-1-yl)pyrazine. MS(m/e): 371.2 (MH⁺).

### Example 31

### 4-(3-Fluoro-phenyl)-5-oxo-4,5-dihydro-tetrazole-1-carboxylic acid (2-cyano-ethyl)-cyclopropyl-amide

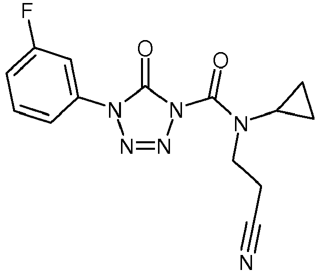

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3-(cyclopropylamino)propanenitrile. MS(m/e): 317.1 (MH⁺).

### Example 32

### 4-(3-Fluoro-phenyl)-5-oxo-4,5-dihydro-tetrazole-1-carboxylic acid methyl-pyridin-2-yl-amide

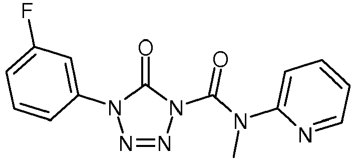

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-methylpyridin-2-amine. MS(m/e): 315.1 (MH⁺).

### Example 33

### 4-(3-Fluoro-phenyl)-5-oxo-4,5-dihydro-tetrazole-1-carboxylic acid methyl-pyridin-3-yl-amide

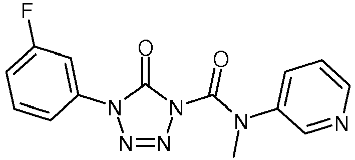

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-methylpyridin-3-amine. MS(m/e): 315.1 (MH⁺).

### Example 34

### 1-(3-Fluoro-phenyl)-4-(2-methoxymethyl-piperidine-1-carbonyl)-1,4-dihydro-tetrazol-5-one

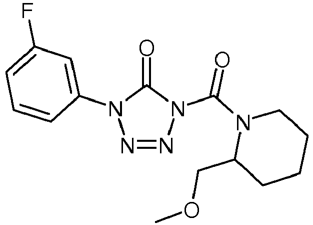

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-(methoxymethyl)piperidine. MS(m/e): 336.2 (MH⁺).

### Example 35

### 4-Cyclopropyl-5-oxo-4,5-dihydro-tetrazole-1-carboxylic acid methyl-pyridin-3-yl-amide

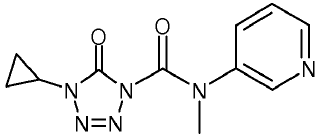

### a) 1-Cyclopropyl-1,4-dihydro-tetrazol-5-one

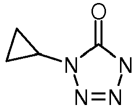

A mixture of cyclopropanecarbonyl chloride (100 mg, 957 µmol) and azidotrimethylsilane (661 mg, 760 µl, 5.74 mmol) are reacted at 0 °C for 1 h. Subsequently, the mixture was warmed to room temperature and heated to 60 °C for 1 h and to 90 °C overnight. Removal of all volatiles yielded the crude title compound which was used without further purification in the subsequent step.

### b) 4-Cyclopropyl-5-oxo-4,5-dihydro-tetrazole-1-carboxylic acid methyl-pyridin-3-yl-amide

A mixture of 1-cyclopropyl-1H-tetrazol-5(4H)-one (27.1 mg, 215 µmol), K₂CO₃ (35.6 mg, 258 µmol) and DMAP (31.5 mg, 258 µmol) in acetonitrile (1 mL) was shaken for 15 min at room temperature. The mixture was treated with methyl(pyridin-3-yl)carbamic chloride (36.7 mg, 215 µmol)and shaken at 60 °C overnight. The mixture was evaporated and the residue was taken up in DMF and formic acid and subjected to purification via preparative HPLC on reversed phase eluting with a gradient formed from acetonitrile, water and NEt₃. The product containing fractions were evaporated to yield 16 mg (29 %) of the title compound as brown solid. MS(m/e): 261.1 (MH⁺).

### Example 36

### 5-Oxo-4-propyl-4,5-dihydro-tetrazole-1-carboxylic acid methyl-pyridin-3-yl-amide

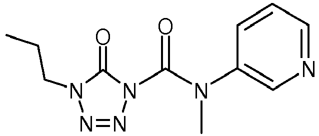

### a) 1-Propyl-1,4-dihydro-tetrazol-5-one

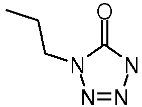

In analogy to the procedure described for the synthesis of 1-Cyclopropyl-1,4-dihydro-tetrazol-5-one (example 35) the title compound was prepared from butyryl chloride and azidotrimethylsilane as light yellow oil and used without further purification in the subsequent step.

### b) 5-Oxo-4-propyl-4,5-dihydro-tetrazole-1-carboxylic acid methyl-pyridin-3-yl-amide

In analogy to the procedure described for the synthesis of 4-Cyclopropyl-5-oxo-4,5-dihydro-tetrazole-1-carboxylic acid methyl-pyridin-3-yl-amide (example 35) the title compound was prepared from 1-Propyl-1,4-dihydro-tetrazol-5-ol and methyl(pyridin-3-yl)carbamic chloride as brown solid. MS(m/e): 263.2 (MH⁺).

### Example 37

### 4-Isobutyl-5-oxo-4,5-dihydro-tetrazole-1-carboxylic acid methyl-pyridin-3-yl-amide

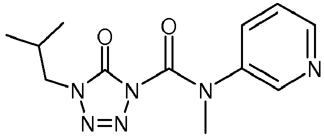

### a) 1-Isobutyl-1,4-dihydro-tetrazol-5-one

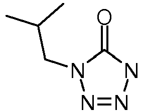

In analogy to the procedure described for the synthesis of 1-Cyclopropyl-1,4-dihydro-tetrazol-5-one (example 35) the title compound was prepared from 3-methylbutanoyl chloride and azidotrimethylsilane as light yellow oil and used without further purification in the subsequent step.

### b) 4-Isobutyl-5-oxo-4,5-dihydro-tetrazole-1-carboxylic acid methyl-pyridin-3-yl-amide

In analogy to the procedure described for the synthesis of 4-Cyclopropyl-5-oxo-4,5-dihydro-tetrazole-1-carboxylic acid methyl-pyridin-3-yl-amide (example 35) the title compound was prepared from 1-Isobutyl-1,4-dihydro-tetrazol-5-one and methyl(pyridin-3-yl)carbamic chloride as brown solid. MS(m/e): 277.2 (MH⁺).

### Example 38

### 1-(4-Acetyl-piperazine-1-carbonyl)-4-(3-fluoro-phenyl)-1,4-dihydro-tetrazol-5-one

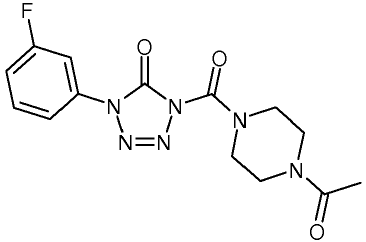

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 1-(piperazin-1-yl)ethanone. MS(m/e): 335.1 (MH⁺).

### Example 39

### 1-(3-Fluoro-phenyl)-4-(4-propionyl-piperazine-1-carbonyl)-1,4-dihydro-tetrazol-5-one

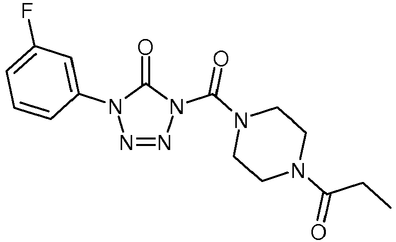

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 1-(piperazin-1-yl)propan-1-one. MS(m/e): 349.2 (MH⁺).

### Example 40

### 4-[4-(3-Fluoro-phenyl)-5-oxo-4,5-dihydro-tetrazole-1-carbonyl]-piperazine-1-carboxylic acid diethylamide

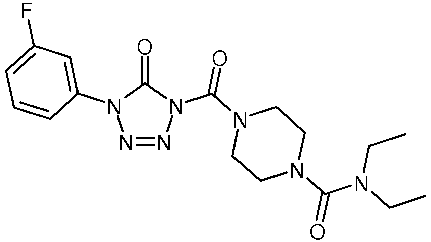

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N,N-diethylpiperazine-1-carboxamide. MS(m/e): 392.3 (MH⁺).

### Example 41

### 1-(3-Fluoro-phenyl)-4-(3-phenyl-pyrrolidine-1-carbonyl)-1,4-dihydro-tetrazol-5-one

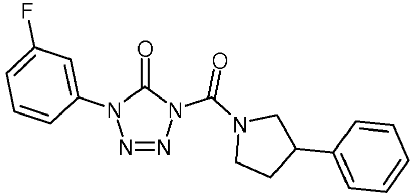

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3-phenylpyrrolidine. MS(m/e): 354.2 (MH⁺).

### Example 42

### N-{(S)-1-[4-(3-Fluoro-phenyl)-5-oxo-4,5-dihydro-tetrazole-1-carbonyl]-pyrrolidin-3-yl}-acetamide

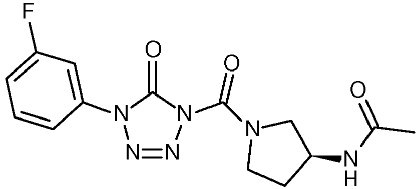

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (S)-N-(pyrrolidin-3-yl)acetamide. MS(m/e): 335.2 (MH⁺).

### Example 43

### N-{(R)-1-[4-(3-Fluoro-phenyl)-5-oxo-4,5-dihydro-tetrazole-1-carbonyl]-pyrrolidin-3-yl}-acetamide

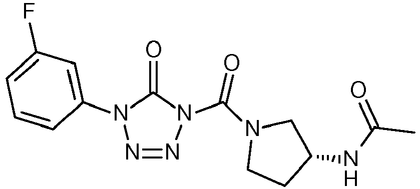

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (R)-N-(pyrrolidin-3-yl)acetamide. MS(m/e): 335.2 (MH⁺).

### Example 44

### N-Ethyl-IN-{1-[4-(3-fluoro-phenyl)-5-oxo-4,5-dihydro-tetrazole-1-carbonyl]-pyrrolidin-3-yl}-acetamide

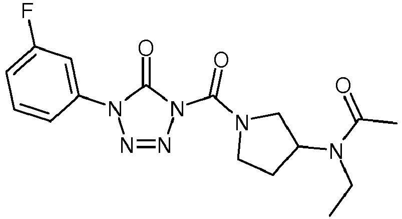

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-ethyl-N-(pyrrolidin-3-yl)acetamide. MS(m/e): 363.2 (MH⁺).

### Example 45

### 1-(3,3-Difluoro-pyrrolidine-1-carbonyl)-4-(3-fluoro-phenyl)-1,4-dihydro-tetrazol-5-one

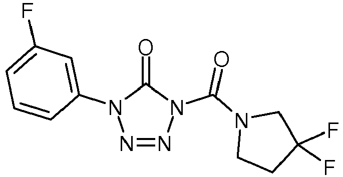

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3,3-difluoropyrrolidine. MS(m/e): 314 (MH⁺).

### Example 46

### 1-(2,2-Dimethyl-pyrrolidine-1-carbonyl)-4-(3-fluoro-phenyl)-1,4-dihydro-tetrazol-5-one

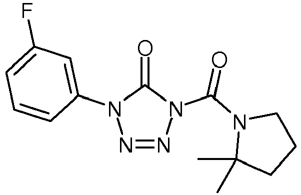

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2,2-dimethylpyrrolidine. MS(m/e): 306.2 (MH⁺).

### Example 47

### 1-(3,3-Dimethyl-pyrrolidine-1-carbonyl)-4-(3-fluoro-phenyl)-1,4-dihydro-tetrazol-5-one

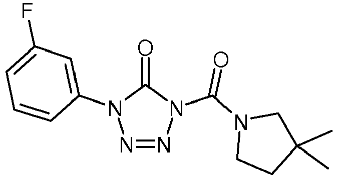

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3,3-dimethylpyrrolidine. MS(m/e): 306.2 (MH⁺).

### Example 48

### 1-(3-Fluoro-phenyl)-4-(2-methyl-pyrrolidine-1-carbonyl)-1,4-dihydro-tetrazol-5-one

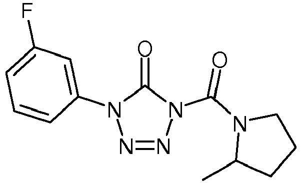

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-methylpyrrolidine. MS(m/e): 292.2 (MH⁺).

### Example 49

### N-{1-[4-(3-Fluoro-phenyl)-5-oxo-4,5-dihydro-tetrazole-1-carbonyl]-pyrrolidin-3-yl}-N-methyl-acetamide

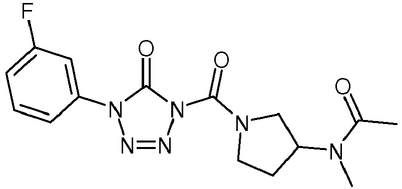

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-methyl-N-(pyrrolidin-3-yl)acetamide. MS(m/e): 349.2 (MH⁺).

### Example 50

### 4-[4-(3-Fluoro-phenyl)-5-oxo-4,5-dihydro-tetrazole-1-carbonyl]-piperazine-1-carboxylic acid ethyl ester

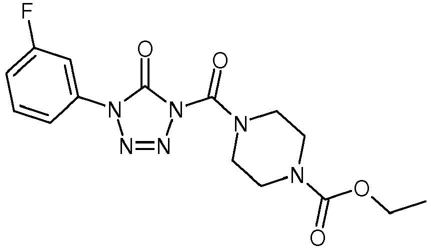

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and ethyl piperazine-1-carboxylate. MS(m/e): 365.1 (MH⁺).

### Example 51

### (S)-1-[4-(3-Fluoro-phenyl)-5-oxo-4,5-dihydro-tetrazole-1-carbonyl]-pyrrolidine-2-carbonitrile

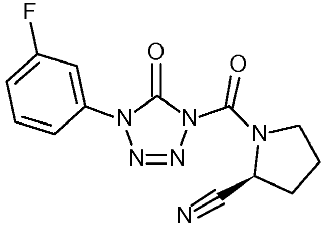

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (R)-pyrrolidine-2-carbonitrile. MS(m/e): 303.2 (MH⁺).

### Example 52

### (R)-1-[4-(3-Fluoro-phenyl)-5-oxo-4,5-dihydro-tetrazole-1-carbonyl]-pyrrolidine-2-carbonitrile

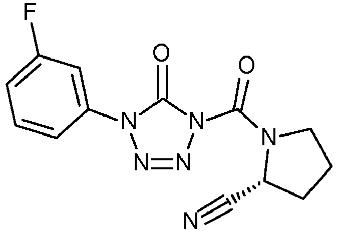

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (S)-pyrrolidine-2-carbonitrile. MS(m/e): 303.2 (MH⁺).

### Example 53

### 4-(6-Chloro-pyridin-3-yl)-5-oxo-4,5-dihydro-tetrazole-1-carboxylic acid benzyl-ethyl-amide

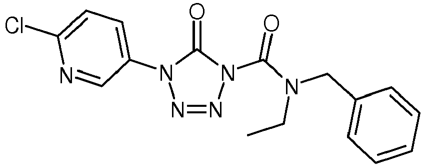

### a) 1-(6-Chloro-pyridin-3-yl)-1,4-dihydro-tetrazol-5-one

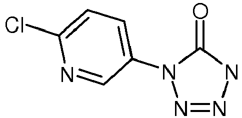

In analogy to the procedure described for the synthesis of 1-Cyclopropyl-1,4-dihydro-tetrazol-5-one (example 35) the title compound was prepared from 6-chloronicotinoyl chloride and azidotrimethylsilane as off-white crystal and used without further purification in the subsequent step.

### b) 4-(6-Chloro-pyridin-3-yl)-5-oxo-4,5-dihydro-tetrazole-1-carboxylic acid benzyl-ethyl-amide

In analogy to the procedure described for the synthesis of 4-Cyclopropyl-5-oxo-4,5-dihydro-tetrazole-1-carboxylic acid methyl-pyridin-3-yl-amide (example 35) the title compound was prepared from 1-(6-Chloro-pyridin-3-yl)-1,4-dihydro-tetrazol-5-one and benzyl(ethyl)carbamic chloride as colorless viscous oil. MS(m/e): 400.3 (MH⁺).

### Example 54

### 4-(3-Fluoro-phenyl)-5-oxo-4,5-dihydro-tetrazole-1-carboxylic acid (2-methoxy-ethyl)-methyl-amide

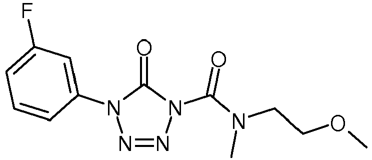

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-methoxy-N-methylethanamine. MS(m/e): 296.2 (MH⁺).

### Example 55

### 4-(3-Fluoro-phenyl)-5-oxo-4,5-dihydro-tetrazole-1-carboxylic acid isopropyl-(2-methoxy-ethyl)-amide

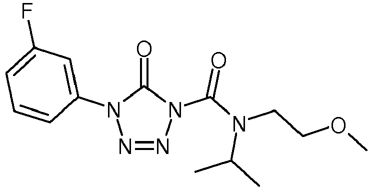

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-(2-methoxyethyl)propan-2-amine. MS(m/e): 324.2 (MH⁺).

### Example 56

### 4-(3-Fluoro-phenyl)-5-oxo-4,5-dihydro-tetrazole-1-carboxylic acid (2-methoxy-ethyl)-propyl-amide

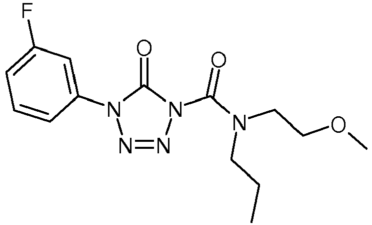

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and N-(2-methoxyethyl)propan-1-amine. MS(m/e): 324.2 (MH⁺).

### Example 57

### 4-(3-Fluoro-phenyl)-5-oxo-4,5-dihydro-tetrazole-1-carboxylic acid bis-(2-methoxy-ethyl)-amide

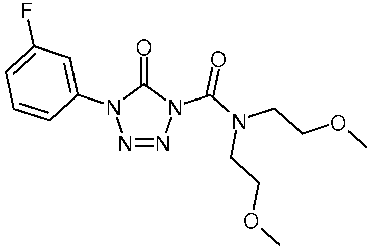

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and bis(2-methoxyethyl)amine. MS(m/e): 340.2 (MH⁺).

### Example 58

### 1-(2,6-Dimethyl-morpholine-4-carbonyl)-4-(3-fluoro-phenyl)-1,4-dihydro-tetrazol-5-one

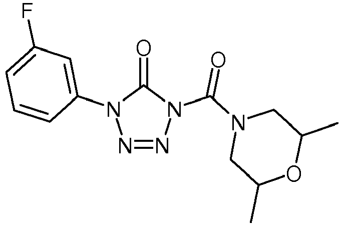

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2,6-dimethylmorpholine. MS(m/e): 322.2 (MH⁺).

### Example 59

### 1-((2S,6R)-2,6-Dimethyl-morpholine-4-carbonyl)-4-(3-fluoro-phenyl)-1,4-dihydro-tetrazol-5-one

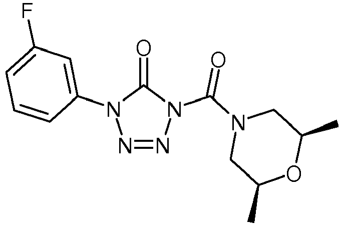

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and (2R,6S)-2,6-dimethylmorpholine. MS(m/e): 322.3 (MH⁺).

### Example 60

### 1-(3-Fluoro-phenyl)-4-(4-methoxymethyl-piperidine-1-carbonyl)-1,4-dihydro-tetrazol-5-one

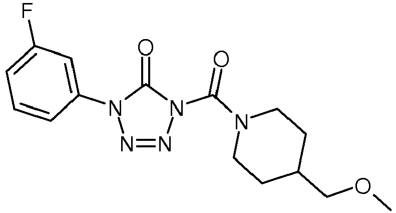

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 4-(methoxymethyl)piperidine. MS(m/e): 336.2 (MH⁺).

### Example 61

### 1-[4-(3-Fluoro-phenyl)-5-oxo-4,5-dihydro-tetrazole-1-carbonyl]-piperidine-4-carbonitrile

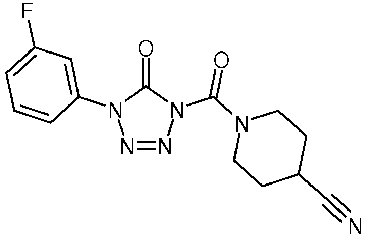

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and piperidine-4-carbonitrile. MS(m/e): 317.3 (MH⁺).

### Example 62

### 4-tert-Butoxycarbonylamino-1-[4-(3-fluoro-phenyl)-5-oxo-4,5-dihydro-tetrazole-1-carbonyl]-piperidine-4-carboxylic acid methyl ester

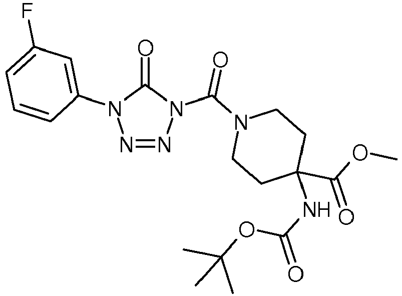

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and methyl 4-(tert-butoxycarbonylamino)piperidine-4-carboxylate. MS(m/e): 465.4 (MH⁺).

### Example 63

### 1-(3,3-Dimethyl-piperidine-1-carbonyl)-4-(3-fluoro-phenyl)-1,4-dihydro-tetrazol-5-one

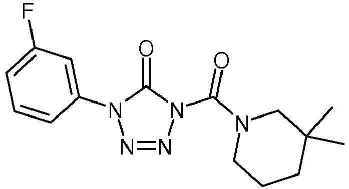

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 3,3-dimethylpiperidine. MS(m/e): 320.2 (MH⁺).

### Example 64

### 1-(3-Fluoro-phenyl)-4-(2-oxa-8-aza-spiro[4.5]decane-8-carbonyl)-1,4-dihydro-tetrazol-5-one

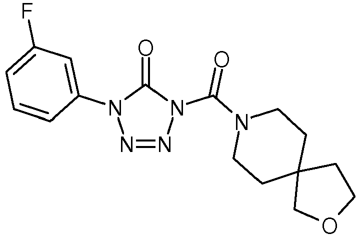

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-Fluoro-phenyl)-1,4-dihydro-tetrazol-5-one and 2-oxa-8-azaspiro[4.5]decane. MS(m/e): 348.2 (MH⁺).

### Example 65

### N-{(R)-1-[4-(3-Chloro-phenyl)-5-oxo-4,5-dihydro-tetrazole-1-carbonyl]-pyrrolidin-3-yl}-acetamide

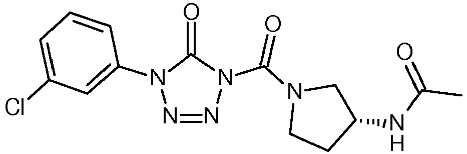

### a) 1-(3-Chloro-phenyl)-1,4-dihydro-tetrazol-5-one

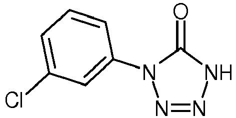

In analogy to the procedure described for the synthesis of 1-Cyclopropyl-1,4-dihydro-tetrazol-5-one (example 35) the title compound was prepared from 3-chlorobenzoyl chloride and azidotrimethylsilane as white solid.

### b) N-{(R)-1-[4-(3-Chloro-phenyl)-5-oxo-4,5-dihydro-tetrazole-1-carbonyl]-pyrrolidin-3-yl}-acetamide

In analogy to the procedure described for the synthesis of 4-Cyclopropyl-5-oxo-4,5-dihydro-tetrazole-1-carboxylic acid methyl-pyridin-3-yl-amide (example 35) the title compound was prepared from 1-(3-Chloro-phenyl)-1,4-dihydro-tetrazol-5-one and (R)-N-(pyrrolidin-3-yl)acetamide. MS(m/e): 351.4 (MH⁺).

### Example 66

### N-{(R)-1-[5-Oxo-4-(3-trifluoromethoxy-phenyl)-4,5-dihydro-tetrazole-1-carbonyl]-pyrrolidin-3-yl}-acetamide

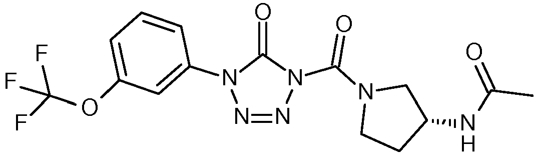

### a) 1-(3-Trifluoromethoxy-phenyl)-1,4-dihydro-tetrazol-5-one

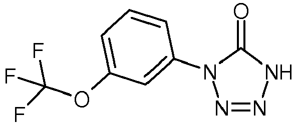

In analogy to the procedure described for the synthesis of 1-Cyclopropyl-1,4-dihydro-tetrazol-5-one (example 35) the title compound was prepared from 3-(trifluoromethoxy)benzoyl chloride and azidotrimethylsilane as white solid.

### b) N-{(R)-1-[5-Oxo-4-(3-trifluoromethoxy-phenyl)-4,5-dihydro-tetrazole-1-carbonyl]-pyrrolidin-3-yl}-acetamide

In analogy to the procedure described for the synthesis of 4-Cyclopropyl-5-oxo-4,5-dihydro-tetrazole-1-carboxylic acid methyl-pyridin-3-yl-amide (example 35) the title compound was prepared from 1-(3-Trifluoromethoxy-phenyl)-1,4-dihydro-tetrazol-5-one and (R)-N-(pyrrolidin-3-yl)acetamide. MS(m/e): 401.4 (MH⁺).

### Example 67

### N-{(R)-1-[4-(4-Chloro-phenyl)-5-oxo-4,5-dihydro-tetrazole-1-carbonyl]-pyrrolidin-3-yl}-acetamide

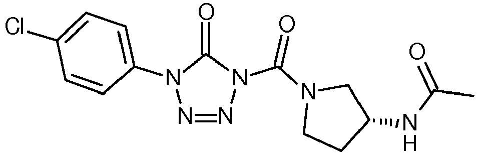

### a) 1-(4-Chloro-phenyl)-1,4-dihydro-tetrazol-5-one

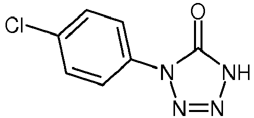

In analogy to the procedure described for the synthesis of 1-Cyclopropyl-1,4-dihydro-tetrazol-5-one (example 35) the title compound was prepared from 4-chlorobenzoyl chloride and azidotrimethylsilane as white solid.

### b) N-{(R)-1-[4-(4-Chloro-phenyl)-5-oxo-4,5-dihydro-tetrazole-1-carbonyl]-pyrrolidin-3-yl}-acetamide

In analogy to the procedure described for the synthesis of 4-Cyclopropyl-5-oxo-4,5-dihydro-tetrazole-1-carboxylic acid methyl-pyridin-3-yl-amide (example 35) the title compound was prepared from 1-(4-Chloro-phenyl)-1,4-dihydro-tetrazol-5-one and (R)-N-(pyrrolidin-3-yl)acetamide. MS(m/e): 351.4 (MH⁺).

### Example 68

### N-{(S)-1-[4-(3-Chloro-phenyl)-5-oxo-4,5-dihydro-tetrazole-1-carbonyl]-pyrrolidin-3-yl}-acetamide

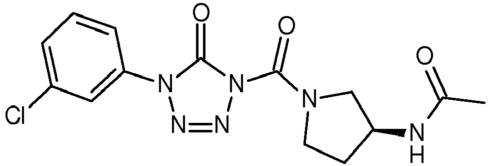

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and (S)-N-(pyrrolidin-3-yl)acetamide. MS(m/e): 351.4 (MH⁺).

### Example 69

### N-{(S)-1-[5-Oxo-4-(3-trifluoromethoxy-phenyl)-4,5-dihydro-tetrazole-1-carbonyl]-pyrrolidin-3-yl}-acetamide

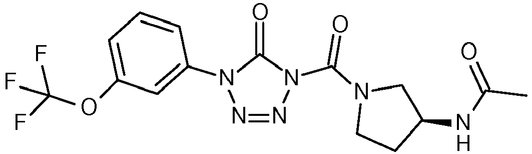

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and (S)-N-(pyrrolidin-3-yl)acetamide. MS(m/e): 401.4 (MH⁺).

### Example 70

### N-{(S)-1-[4-(4-Chloro-phenyl)-5-oxo-4,5-dihydro-tetrazole-1-carbonyl]-pyrrolidin-3-yl}-acetamide

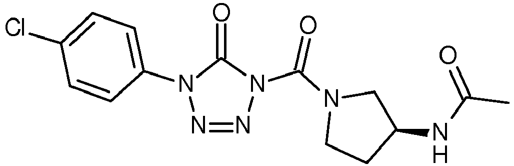

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and (S)-N-(pyrrolidin-3-yl)acetamide. MS(m/e): 351.4 (MH⁺).

### Example 71

### N-{1-[4-(3-Chloro-phenyl)-5-oxo-4,5-dihydro-tetrazole-1-carbonyl]-pyrrolidin-3-yl}-N-ethyl-acetamide

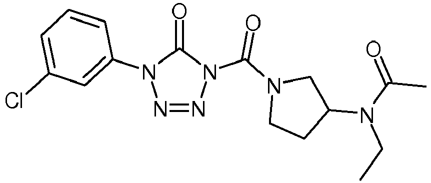

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and N-ethyl-N-(pyrrolidin-3-yl)acetamide. MS(m/e): 379.4 (MH⁺).

### Example 72

### N-Ethyl-N-{1-[5-oxo-4-(3-trifluoromethoxy-phenyl)-4,5-dihydro-tetrazole-1-carbonyl]-pyrrolidin-3-yl}-acetamide

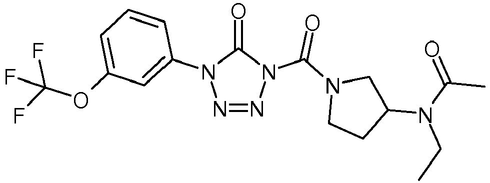

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and N-ethyl-N-(pyrrolidin-3-yl)acetamide. MS(m/e): 429.4 (MH⁺).

### Example 73

### N-{1-[4-(4-Chloro-phenyl)-5-oxo-4,5-dihydro-tetrazole-1-carbonyl]-pyrrolidin-3-yl}-N-ethyl-acetamide

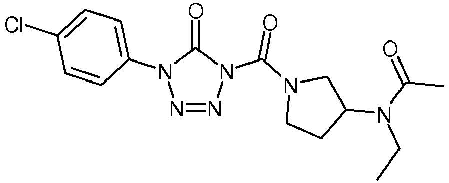

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and N-ethyl-N-(pyrrolidin-3-yl)acetamide. MS(m/e): 379.4 (MH⁺).

### Example 74

### 1-(3-Chloro-phenyl)-4-[2-(3-methyl-isoxazol-5-yl)-pyrrolidine-1-carbonyl]-1,4-dihydro-tetrazol-5-one

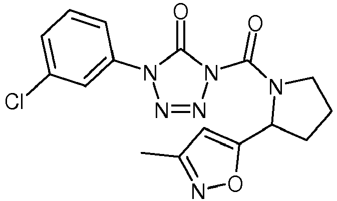

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and 3-methyl-5-(pyrrolidin-2-yl)isoxazole. MS(m/e): 375.4 (MH⁺).

### Example 75

### 1-[2-(3-Methyl-isoxazol-5-yl)-pyrrolidine-1-carbonyl]-4-(3-trifluoromethoxy-phenyl)-1,4-dihydro-tetrazol-5-one

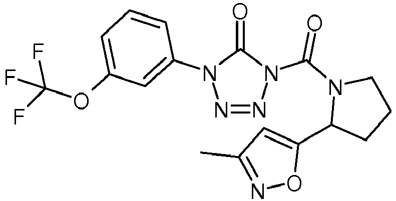

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 3-methyl-5-(pyrrolidin-2-yl)isoxazole. MS(m/e): 425.4 (MH⁺).

### Example 76

### 1-(4-Chloro-phenyl)-4-[2-(3-methyl-isoxazol-5-yl)-pyrrolidine-1-carbonyl]-1,4-dihydro-tetrazol-5-one

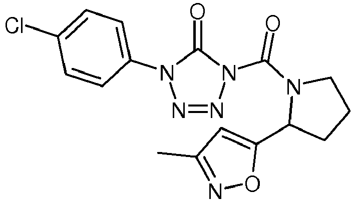

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 3-methyl-5-(pyrrolidin-2-yl)isoxazole. MS(m/e): 375.4 (MH⁺).

### Example 77

### 1-(3-Chloro-phenyl)-4-(4,4-dimethyl-piperidine-1-carbonyl)-1,4-dihydro-tetrazol-5-one

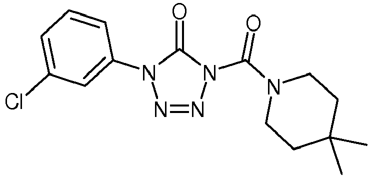

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and 4,4-dimethylpiperidine. MS(m/e): 336.4 (MH⁺).

### Example 78

### 1-(4,4-Dimethyl-piperidine-1-carbonyl)-4-(3-trifluoromethoxy-phenyl)-1,4-dihydro-tetrazol-5-one

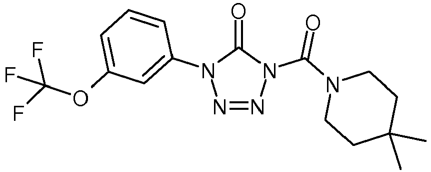

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 4,4-dimethylpiperidine. MS(m/e): 386. (MH⁺).

### Example 79

### 1-(4-Chloro-phenyl)-4-(4,4-dimethyl-piperidine-1-carbonyl)-1,4-dihydro-tetrazol-5-one

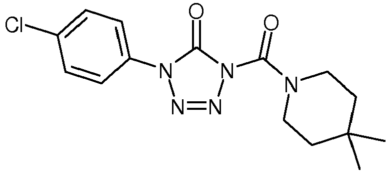

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 4,4-dimethylpiperidine. MS(m/e): 336.4 (MH⁺).

### Example 80

### 4-(3-Chloro-phenyl)-5-oxo-4,5-dihydro-tetrazole-1-carboxylic acid ethyl-(2-methoxyethyl)-amide

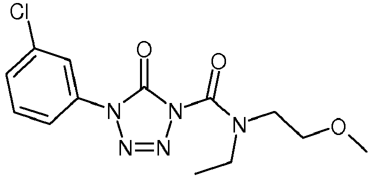

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and N-ethyl-2-methoxyethanamine. MS(m/e): 326.4 (MH⁺).

### Example 81

### 5-Oxo-4-(3-trifluoromethoxy-phenyl)-4,5-dihydro-tetrazole-1-carboxylic acid ethyl-(2-methoxy-ethyl)-amide

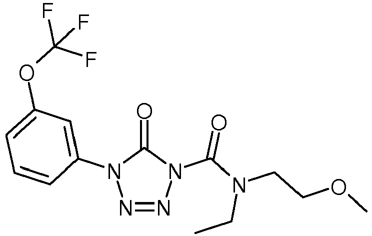

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and N-ethyl-2-methoxyethanamine. MS(m/e): 376.3 (MH⁺).

### Example 82

### 4-(4-Chloro-phenyl)-5-oxo-4,5-dihydro-tetrazole-1-carboxylic acid ethyl-(2-methoxyethyl)-amide

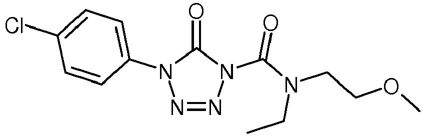

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and N-ethyl-2-methoxyethanamine. MS(m/e): 326.4 (MH⁺).

### Example 83

### 4-(3-Chloro-phenyl)-5-oxo-4,5-dihydro-tetrazole-1-carboxylic acid isopropyl-(2-methoxy-ethyl)-amide

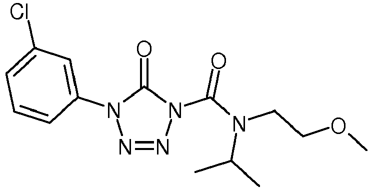

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and N-(2-methoxyethyl)propan-2-amine. MS(m/e): 340.5 (MH⁺).

### Example 84

### 5-Oxo-4-(3-trifluoromethoxy-phenyl)-4,5-dihydro-tetrazole-1-carboxylic acid isopropyl-(2-methoxy-ethyl)-amide

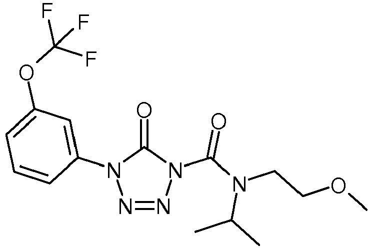

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and N-(2-methoxyethyl)propan-2-amine. MS(m/e): 390.4 (MH⁺).

### Example 85

### 4-(4-Chloro-phenyl)-5-oxo-4,5-dihydro-tetrazole-1-carboxylic acid isopropyl-(2-methoxy-ethyl)-amide

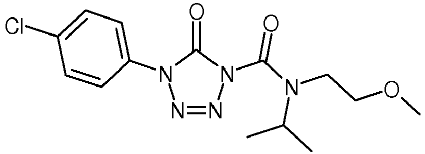

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and N-(2-methoxyethyl)propan-2-amine. MS(m/e): 340.4 (MH⁺).

### Example 86

### 4-(3-Chloro-phenyl)-5-oxo-4,5-dihydro-tetrazole-1-carboxylic acid (3-methoxypropyl)-methyl-amide

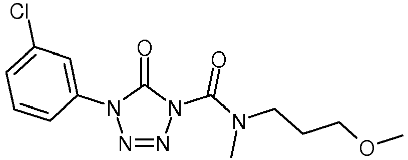

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and 3-methoxy-N-methylpropan-l-amine. MS(m/e): 326.4 (MH⁺).

### Example 87

### 5-Oxo-4-(3-trifluoromethoxy-phenyl)-4,5-dihydro-tetrazole-l-carboxylic acid (3-ethoxy-propyl)-methyl-amide

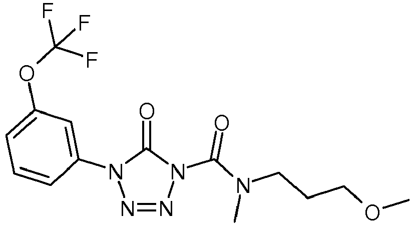

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 3-methoxy-N-methylpropan-1-amine. MS(m/e): 376.5 (MH⁺).

### Example 88

### 4-(4-Chloro-phenyl)-5-oxo-4,5-dihydro-tetrazole-1-carboxylic acid (3-methoxy-propyl)-methyl-amide

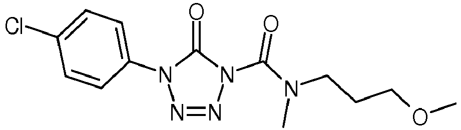

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 3-methoxy-N-methylpropan-1-amine. MS(m/e): 326.4 (MH⁺).

### Example 89

### 1-(3-Chloro-phenyl)-4-[4-(2-morpholin-4-yl-2-oxo-ethyl)-piperazine-1-carbonyl]-1,4-dihydro-tetrazol-5-one

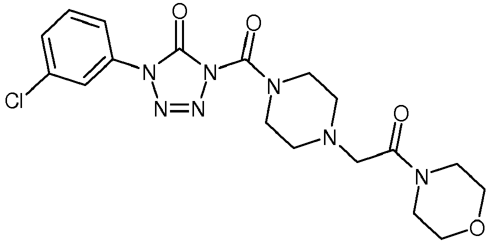

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and l-morpholino-2-(piperazin-l-yl)ethanone. MS(m/e): 436.4 (MH⁺).

### Example 90

### 1-[4-(2-Morpholin-4-yl-2-oxo-ethyl)-piperazine-1-carbonyl]-4-(3-trifluoromethoxy-phenyl)-1,4-dihydro-tetrazol-5-one

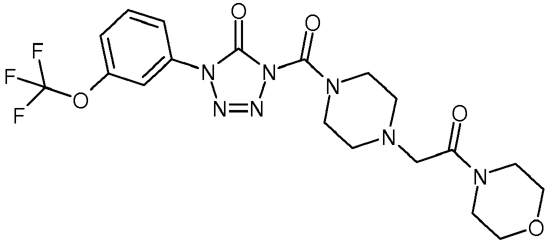

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 1-morpholino-2-(piperazin-1-yl)ethanone. MS(m/e): 486.4 (MH⁺).

### Example 91

### 1-(4-Chloro-phenyl)-4-[4-(2-morpholin-4-yl-2-oxo-ethyl)-piperazine-1-carbonyl]-1,4-dihydro-tetrazol-5-one

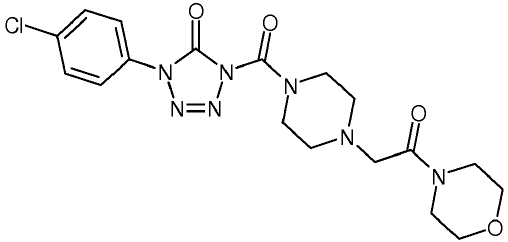

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 1-morpholino-2-(piperazin-1-yl)ethanone. MS(m/e): 436.4 (MH⁺).

### Example 92

### 2-{4-[4-(3-Chloro-phenyl)-5-oxo-4,5-dihydro-tetrazole-1-carbonyl]-piperazin-1-yl}-N,N-dimethyl-acetamide

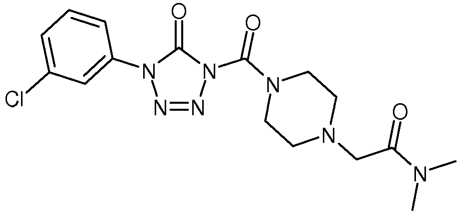

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and N,N-dimethyl-2-(piperazin-1-yl)acetamide. MS(m/e): 394.5 (MH⁺).

### Example 93

### N,N-Dimethyl-2-{4-[5-oxo-4-(3-trifluoromethoxy-phenyl)-4,5-dihydro-tetrazole-1-carbonyl]-piperazin-1-yl}-acetamide

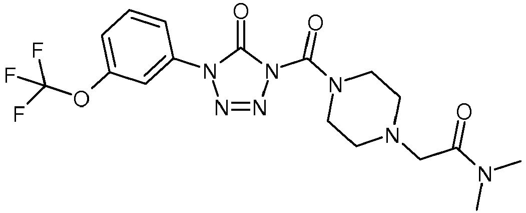

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and N,N-dimethyl-2-(piperazin-1-yl)acetamide. MS(m/e): 444.4 (MH⁺).

### Example 94

### 2-{4-[4-(4-Chloro-phenyl)-5-oxo-4,5-dihydro-tetrazole-1-carbonyl]-piperazin-1-yl}-N,N-dimethyl-acetamide

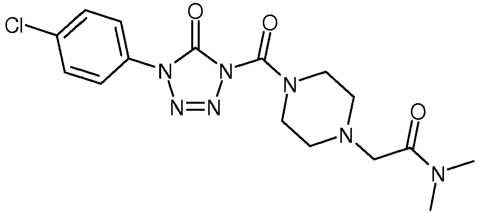

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and N,N-dimethyl-2-(piperazin-1-yl)acetamide. MS(m/e): 394.5 (MH⁺).

### Example 95

### 1-(3-Chloro-phenyl)-4-[4-(2-oxo-2-pyrrolidin-1-yl-ethyl)-piperazine-1-carbonyl]-1,4-dihydro-tetrazol-5-one

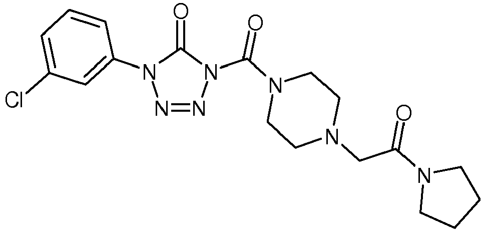

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and 2-(piperazin-1-yl)-1-(pyrrolidin-1-yl)ethanone. MS(m/e): 420.5 (MH⁺).

### Example 96

### 1-[4-(2-Oxo-2-pyrrolidin-1-yl-ethyl)-piperazine-1-carbonyl]-4-(3-trifluoromethoxy-phenyl)-1,4-dihydro-tetrazol-5-one

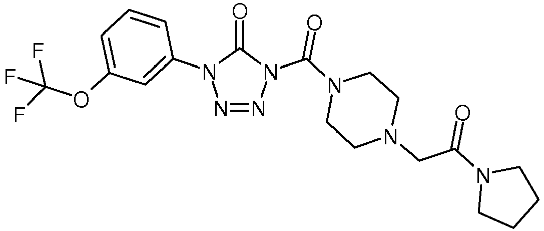

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 2-(piperazin-1-yl)-1-(pyrrolidin-1-yl)ethanone. MS(m/e): 470.4 (MH⁺).

### Example 97

### 1-(4-Chloro-phenyl)-4-[4-(2-oxo-2-pyrrolidin-1-yl-ethyl)-piperazine-1-carbonyl]-1,4-dihydro-tetrazol-5-one

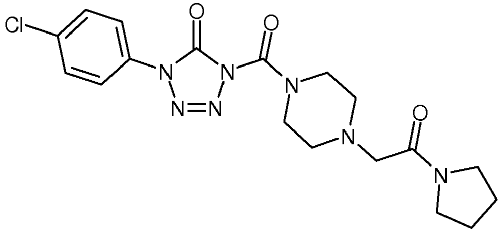

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 2-(piperazin-1-yl)-1-(pyrrolidin-1-yl)ethanone. MS(m/e): 420.5 (MH⁺).

### Example 98

### 4-(3-Chloro-phenyl)-5-oxo-4,5-dihydro-tetrazole-1-carboxylic acid (2-ethoxy-ethyl)-ethyl-amide

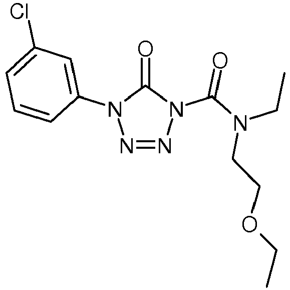

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and 2-ethoxy-N-ethylethanamine. MS(m/e): 340.4 (MH⁺).

### Example 99

### 5-Oxo-4-(3-trifluoromethoxy-phenyl)-4,5-dihydro-tetrazole-1-carboxylic acid (2-ethoxy-ethyl)-ethyl-amide

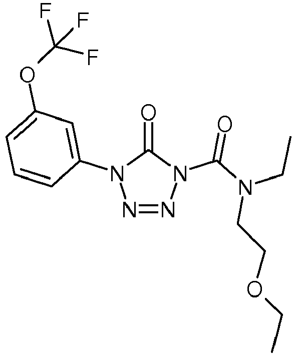

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 2-ethoxy-N-ethylethanamine. MS(m/e): 390.2 (MH⁺).

### Example 100

### 4-(4-Chloro-phenyl)-5-oxo-4,5-dihydro-tetrazole-1-carboxylic acid (2-ethoxy-ethyl)-ethyl-amide

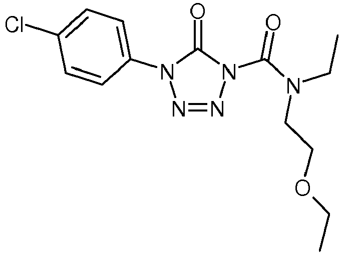

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 2-ethoxy-N-ethylethanamine. MS(m/e): 340.2 (MH⁺).

### Example 101

### 1-(3-Chloro-phenyl)-4-(morpholine-4-carbonyl)-1,4-dihydro-tetrazol-5-one

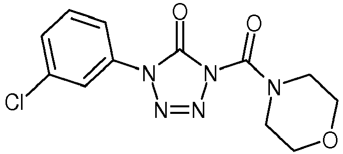

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and morpholine. MS(m/e): 309.9 (MH⁺).

### Example 102

### 1-(Morpholine-4-carbonyl)-4-(3-trifluoromethoxy-phenyl)-1,4-dihydro-tetrazol-5-one

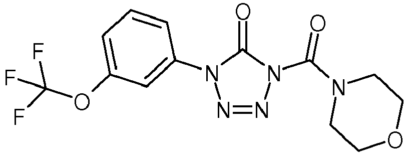

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and morpholine. MS(m/e): 360.3 (MH⁺).

### Example 103

### 1-(4-Chloro-phenyl)-4-(morpholine-4-carbonyl)-1,4-dihydro-tetrazol-5-one

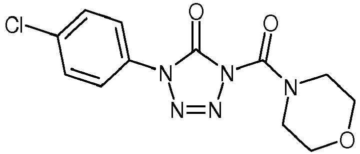

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and morpholine. MS(m/e): 310.1 (MH⁺).

### Example 104

### 2-{4-[4-(3-Chloro-phenyl)-5-oxo-4,5-dihydro-tetrazole-1-carbonyl]-piperazin-1-yl}-N-ethyl-acetamide

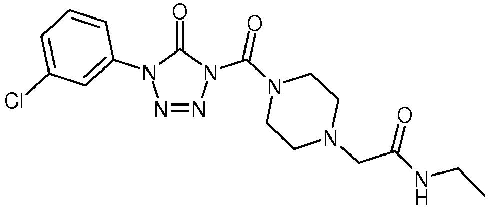

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and N-ethyl-2-(piperazin-1-yl)acetamide. MS(m/e): 394.2 (MH⁺).

### Example 105

### N-Ethyl-2-{4-[5-oxo-4-(3-trifluoromethoxy-phenyl)-4,5-dihydro-tetrazole-1-carbonyl]-piperazin-1-yl}-acetamide

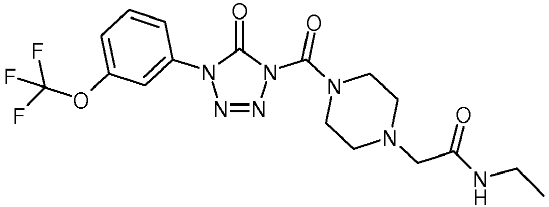

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and N-ethyl-2-(piperazin-1-yl)acetamide. MS(m/e): 444.4 (MH⁺).

### Example 106

### 2-{4-[4-(4-Chloro-phenyl)-5-oxo-4,5-dihydro-tetrazole-1-carbonyl]-piperazin-1-yl}-N-ethyl-acetamide

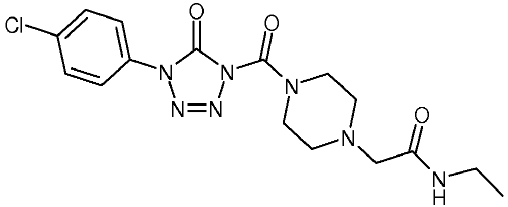

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and N-ethyl-2-(piperazin-1-yl)acetamide. MS(m/e): 394.2 (MH⁺).

### Example 107

### N-Butyl-2-{4-[4-(3-chloro-phenyl)-5-oxo-4,5-dihydro-tetrazole-1-carbonyl]-piperazin-1-yl}-acetamide

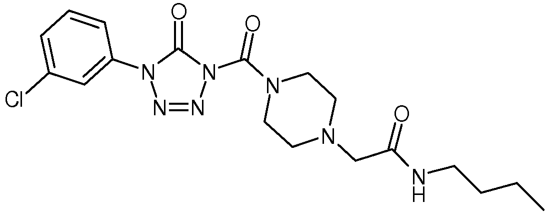

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and N-butyl-2-(piperazin-1-yl)acetamide. MS(m/e): 422.3 (MH⁺).

### Example 108

### N-Butyl-2-{4-[5-oxo-4-(3-trifluoromethoxy-phenyl)-4,5-dihydro-tetrazole-1-carbonyl]-piperazin-1-yl}-acetamide

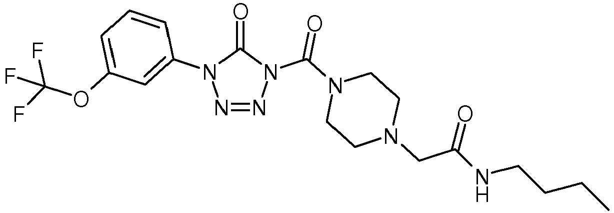

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and N-butyl-2-(piperazin-1-yl)acetamide. MS(m/e): 472.3 (MH⁺).

### Example 109

### N-Butyl-2-{4-[4-(4-chloro-phenyl)-5-oxo-4,5-dihydro-tetrazole-1-carbonyl]-piperazin-1-yl}-acetamide

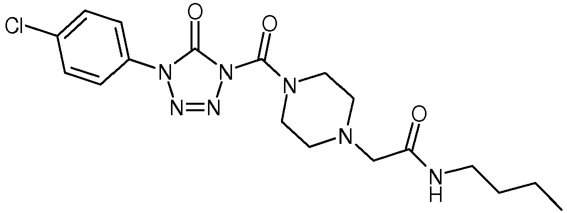

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and N-butyl-2-(piperazin-1-yl)acetamide. MS(m/e): 422.3 (MH⁺).

### Example 110

### 4-(3-Fluoro-phenyl)-5-oxo-4,5-dihydro-tetrazole-1-carboxylic acid (2-methoxy-ethyl)-amide

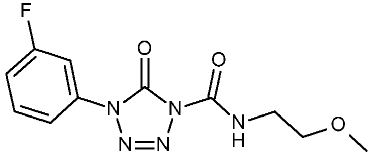

A mixture of 1-(3-fluorophenyl)-1H-tetrazol-5(4H)-one (222.2 mg, 1.23 mmol) and DMAP (301 mg, 2.47 mmol) in DCM (12.3 mL) was treated at 0 °C with diphosgene (254 mg, 155 µl, 1.28 mmol) and subsequently with 2-methoxyethanamine (139 mg, 159 µl, 1.85 mmol). The mixture was stirred at room temperature overnight and evaporated. The residue was taken up in DMF (5 mL) and 300 uL HCOOH and subjected to purification by preparative HPLC eluting with a gradient formed from acetonitrile, water and HCOOH. The product containing fractions were evaporated to yield 145 mg (40 %) of the title compound as white solid. MS(m/e): 282.1(MH⁺).

### Example 111

### 4-(3-Chloro-phenyl)-5-oxo-4,5-dihydro-tetrazole-1-carboxylic acid (2-ethoxy-ethyl)-methyl-amide

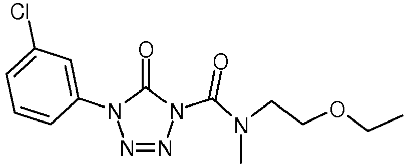

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and 2-ethoxy-N-methylethanamine hydrochloride. MS(m/e): 326.1 (MH⁺).

### Example 112

### 5-Oxo-4-(3-trifluoromethoxy-phenyl)-4,5-dihydro-tetrazole-1-carboxylic acid (2-ethoxy-ethyl)-methyl-amide

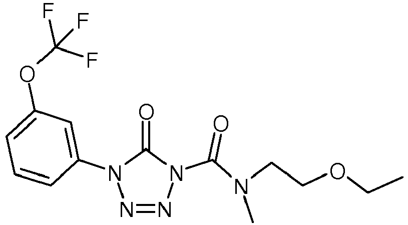

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 2-ethoxy-N-methylethanamine hydrochloride. MS(m/e): 376.2 (MH⁺).

### Example 113

### 4-(4-Chloro-phenyl)-5-oxo-4,5-dihydro-tetrazole-1-carboxylic acid (2-ethoxy-ethyl)-methyl-amide

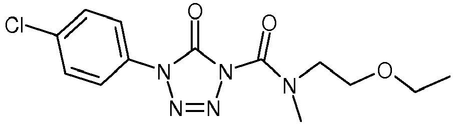

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 2-ethoxy-N-methylethanamine hydrochloride. MS(m/e): 326.1 (MH⁺).

### Example 114

### [4-(3-Chloro-phenyl)-5-oxo-4,5-dihydro-tetrazol-1-yl]-acetic acid ethyl ester

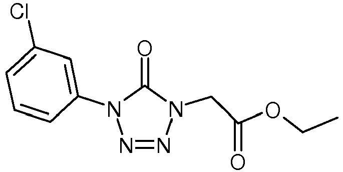

In analogy to the procedure described for the synthesis of 2-[4-(3-Fluoro-phenyl)-5-oxo-4,5-dihydro-tetrazol-l-yl]-2-methyl-propionic acid ethyl ester (example 16) the title compound was prepared from 1-(3-chloro-phenyl)-1,4-dihydro-tetrazol-5-one and ethyl 2-bromoacetate and isolated as white solid. MS(m/e): 283.1 (MH⁺).

### Example 115

### 1-(3-Chloro-phenyl)-4-(2-morpholin-4-yl-2-oxo-ethyl)-1,4-dihydro-tetrazol-5-one

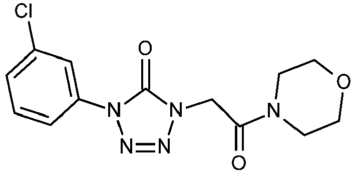

A mixture of ethyl 2-(4-(3-chlorophenyl)-5-oxo-4,5-dihydro-1H-tetrazol-1-yl)acetate (57.4 mg, 203 µmol) and LiOH (monohydrat) (15 mg, 357 µmol) in water (2 mL), MeOH (2 mL) and THF (0.5 mL) was stirred at room temperature for 2 h. The mixture was evaporated and water and HCl (1 M aq) was 42 mg (81 %) of the acid as white crystals.

31.2 mg (123 µmol) of the acid and TBTU (43.3 mg, 135 µmol) in DMF (1.5 mL) were treated wih morpholine (21.3 mg, 21.3 µl, 245 µmol) and shaken at room temperature for 1.5 h. HCOOH was added and the mixture was subjected to purification by preparative HPLC eluting with a gradient formed from acetonitrile, water and HCOOH. The product containing fractions were evaporated to yield 28 mg (72 %) of the title compound as white solid. MS(m/e): 324.1(MH⁺).

### Example 116

### 1-(3-Chloro-phenyl)-4-[4-(1-methyl-2-oxo-2-pyrrolidin-1-yl-ethyl)-piperazine-1-carbonyl]-1,4-dihydro-tetrazol-5-one

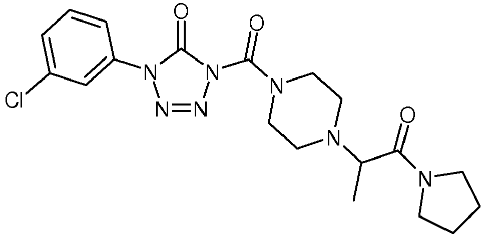

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-chloro-phenyl)-1,4-dihydro-tetrazol-5-one and 2-(piperazin-1-yl)-1-(pyrrolidin-1-yl)propan-1-one. MS(m/e): 434.4 (MH⁺).

### Example 117

### 1-[4-(1-Methyl-2-oxo-2-pyrrolidin-1-yl-ethyl)-piperazine-1-carbonyl]-4-(3-trifluoromethoxy-phenyl)-1,4-dihydro-tetrazol-5-one

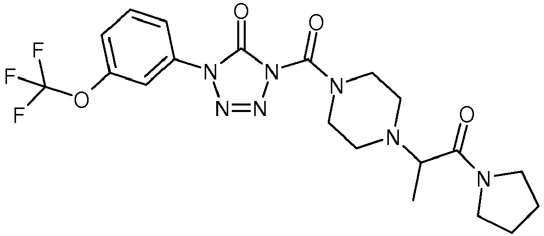

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(3-(trifluoromethoxy)phenyl)-1H-tetrazol-5(4H)-one and 2-(piperazin-1-yl)-1-(pyrrolidin-1-yl)propan-1-one. MS(m/e): 484.4 (MH⁺).

### Example 118

### 1-(4-Chloro-phenyl)-4-[4-(1-methyl-2-oxo-2-pyrrolidin-1-yl-ethyl)-piperazine-1-carbonyl]-1,4-dihydro-tetrazol-5-one

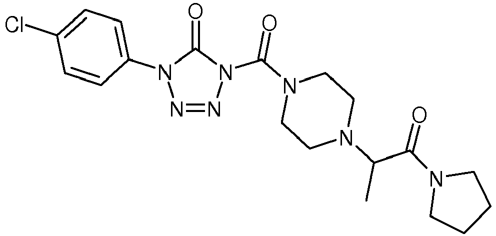

In analogy to the procedure described for the synthesis of 4-(3-Fluoro-phenyl)-5-oxo-4,5-dihydro-tetrazole-1-carboxylic acid [2-(3,4-dimethoxy-phenyl)-ethyl]-methyl-amide (example 1) the title compound was prepared from 1-(4-chloro-phenyl)-1,4-dihydro-tetrazol-5-one and 2-(piperazin-1-yl)-1-(pyrrolidin-1-yl)propan-1-one. MS(m/e): 434.4 (MH⁺).

### Example 119

### 1-(morpholine-4-carbonyl)-4-[2-(trifluoromethyl)phenyl]tetrazol-5-one

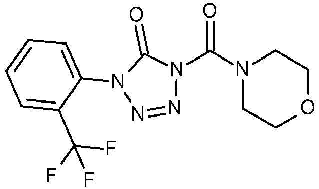

### a) 1-(2-Trifluoromethyl-phenyl)-1,4-dihydro-tetrazol-5-one

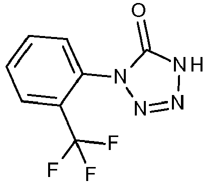

In analogy to the procedure described for the synthesis of 1-Cyclopropyl-1,4-dihydro-tetrazol-5-one (example 35) the title compound was prepared from 2-(trifluoromethyl)benzoyl chloride and azidotrimethylsilane as white solid and used in the subsequent step.

### b) 1-(morpholine-4-carbonyl)-4-[2-(trifluoromethyl)phenyl]tetrazol-5-one

In analogy to the procedure described for the synthesis of 4-Cyclopropyl-5-oxo-4,5-dihydro-tetrazole-1-carboxylic acid methyl-pyridin-3-yl-amide (example 35) the title compound was prepared from 1-(2-Trifluoromethyl-phenyl)-1,4-dihydro-tetrazol-5-one and morpholine-4-carbonyl chloride. MS(m/e): 344.2 (MH⁺).

### Example 120

### 1-(morpholine-4-carbonyl)-4-[3-(trifluoromethyl)phenyl]tetrazol-5-one

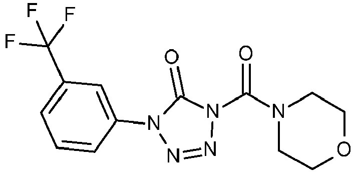

In analogy to the procedure described for the synthesis of 1-(morpholine-4-carbonyl)-4-[2-(trifluoromethyl)phenyl]tetrazol-5-one (example 119) the title compound was prepared from 3-(trifluoromethyl)benzoyl chloride, azidotrimethylsilane and morpholine-4-carbonyl chloride. MS(m/e): 344.3 (MH⁺).

### Example 121

### 1-(morpholine-4-carbonyl)-4-[4-(trifluoromethyl)phenyl]tetrazol-5-one

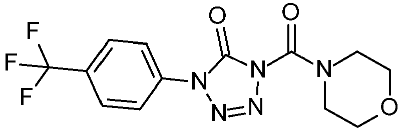

In analogy to the procedure described for the synthesis of 1-(morpholine-4-carbonyl)-4-[2-(trifluoromethyl)phenyl]tetrazol-5-one (example 119) the title compound was prepared from 4-(trifluoromethyl)benzoyl chloride, azidotrimethylsilane and morpholine-4-carbonyl chloride. MS(m/e): 344.2 (MH⁺).

### Example 122

### 1-(2,6-difluorophenyl)-4-(morpholine-4-carbonyl)tetrazol-5-one

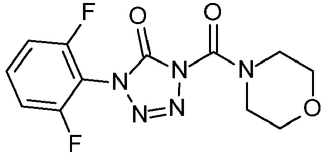

### a) 1-(2,6-Difluoro-phenyl)-1,4-dihydro-tetrazol-5-one

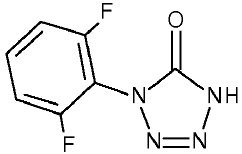

In analogy to the procedure described for the synthesis of 1-(3-Fluoro-phenyl)-1,4-dihydro-tetrazol-5-one (example 1) the title compound was prepared from 1,3-difluoro-2-isocyanatobenzene and azidotrimethylsilane. MS(m/e): 240.1 (MH⁺).

### b) 1-(2,6-difluorophenyl)-4-(morpholine-4-carbonyl)tetrazol-5-one

In analogy to the procedure described for the synthesis of 1-(morpholine-4-carbonyl)-4-[2-(trifluoromethyl)phenyl]tetrazol-5-one (example 119) the title compound was prepared from 1-(2,6-Difluoro-phenyl)-1,4-dihydro-tetrazol-5-one and morpholine-4-carbonyl chloride. MS(m/e): 312.3 (MH⁺).

### Example 123

### 1-(2,4-difluorophenyl)-4-(morpholine-4-carbonyl)tetrazol-5-one

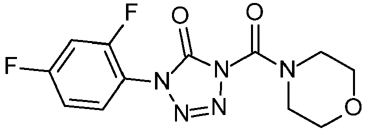

### a) 1-(2,4-Difluoro-phenyl)-1,4-dihydro-tetrazol-5-one

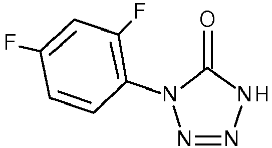

In analogy to the procedure described for the synthesis of 1-(3-Fluoro-phenyl)-1,4-dihydro-tetrazol-5-one (example 1) the title compound was prepared from 2,4-difluoro-2-isocyanatobenzene and azidotrimethylsilane. MS(m/e): 240.1 (MH⁺).

### b) 1-(2,4-difluorophenyl)-4-(morpholine-4-carbonyl)tetrazol-5-one

In analogy to the procedure described for the synthesis of 1-(morpholine-4-carbonyl)-4-[2-(trifluoromethyl)phenyl]tetrazol-5-one (example 119) the title compound was prepared from 1-(2,4-Difluoro-phenyl)-1,4-dihydro-tetrazol-5-one and morpholine-4-carbonyl chloride. MS(m/e): 312.3 (MH⁺).

### Example 124

### 1-(2,5-difluorophenyl)-4-(morpholine-4-carbonyl)tetrazol-5-one

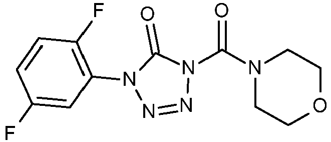

In analogy to the procedure described for the synthesis of 1-(2,6-difluorophenyl)-4-(morpholine-4-carbonyl)tetrazol-5-one (example 122) the title compound was prepared from 1,4-difluoro-2-isocyanatobenzene, azidotrimethylsilane and morpholine-4-carbonyl chloride. MS(m/e): 312.2 (MH⁺).

### Example 125

### 1-(3,4-difluorophenyl)-4-(morpholine-4-carbonyl)tetrazol-5-one

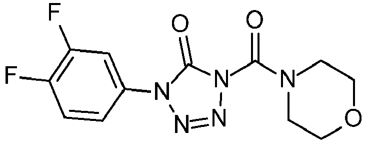

In analogy to the procedure described for the synthesis of 1-(2,6-difluorophenyl)-4-(morpholine-4-carbonyl)tetrazol-5-one (example 122) the title compound was prepared from 1,2-Difluoro-4-isocyanato-benzene, azidotrimethylsilane and morpholine-4-carbonyl chloride. MS(m/e): 312.2 (MH⁺).

### Example 126

### 1-(3,5-difluorophenyl)-4-(morpholine-4-carbonyl)tetrazol-5-one

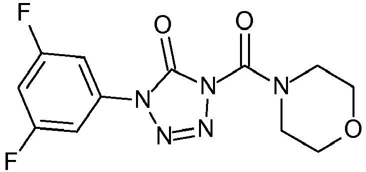

In analogy to the procedure described for the synthesis of 1-(2,6-difluorophenyl)-4-(morpholine-4-carbonyl)tetrazol-5-one (example 122) the title compound was prepared from 1,3-difluoro-5-isocyanatobenzene, azidotrimethylsilane and morpholine-4-carbonyl chloride. MS(m/e): 312.1 (MH⁺).

### Example 127

### 1-(3-methylphenyl)-4-(morpholine-4-carbonyl)tetrazol-5-one

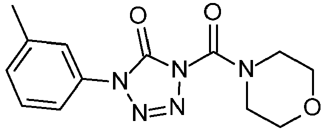

In analogy to the procedure described for the synthesis of 1-(2,6-difluorophenyl)-4-(morpholine-4-carbonyl)tetrazol-5-one (example 122) the title compound was prepared from 1-isocyanato-3-methylbenzene, azidotrimethylsilane and morpholine-4-carbonyl chloride. MS(m/e): 290.2 (MH⁺).

### Example 128

### 1-(4-methylphenyl)-4-(morpholine-4-carbonyl)tetrazol-5-one

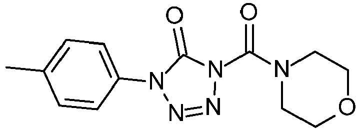

In analogy to the procedure described for the synthesis of 1-(2,6-difluorophenyl)-4-(morpholine-4-carbonyl)tetrazol-5-one (example 122) the title compound was prepared from 1-isocyanato-4-methylbenzene, azidotrimethylsilane and morpholine-4-carbonyl chloride. MS(m/e): 290.1 (MH⁺).

### Example 129

### 1-(3-methoxyphenyl)-4-(morpholine-4-carbonyl)tetrazol-5-one

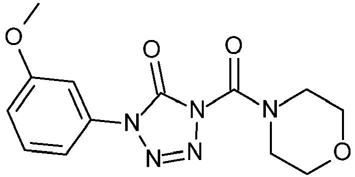

In analogy to the procedure described for the synthesis of 1-(2,6-difluorophenyl)-4-(morpholine-4-carbonyl)tetrazol-5-one (example 122) the title compound was prepared from 1-isocyanato-3-methoxybenzene, azidotrimethylsilane and morpholine-4-carbonyl chloride. MS(m/e): 306.2 (MH⁺).

### Example 130

### 1-(4-methoxyphenyl)-4-(morpholine-4-carbonyl)tetrazol-5-one

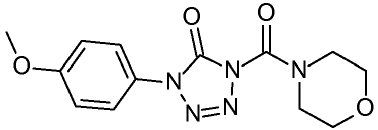

In analogy to the procedure described for the synthesis of 1-(2,6-difluorophenyl)-4-(morpholine-4-carbonyl)tetrazol-5-one (example 122) the title compound was prepared from 1-isocyanato-4-methoxybenzene, azidotrimethylsilane and morpholine-4-carbonyl chloride. MS(m/e): 306.2 (MH⁺).

### Example 131

### 1-(2,3-dichlorophenyl)-4-(morpholine-4-carbonyl)tetrazol-5-one

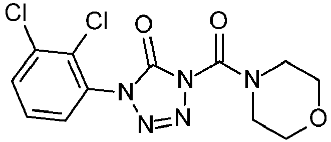

In analogy to the procedure described for the synthesis of 1-(2,6-difluorophenyl)-4-(morpholine-4-carbonyl)tetrazol-5-one (example 122) the title compound was prepared from 1,2-dichloro-3-isocyanatobenzene, azidotrimethylsilane and morpholine-4-carbonyl chloride. MS(m/e): 344.1 (MH⁺).

### Example 132

### 1-(1-methylindol-4-yl)-4-(morpholine-4-carbonyl)tetrazol-5-one

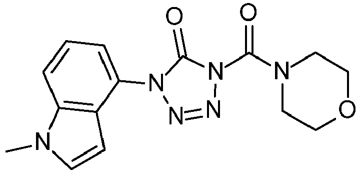

In analogy to the procedure described for the synthesis of 1-(2,6-difluorophenyl)-4-(morpholine-4-carbonyl)tetrazol-5-one (example 122) the title compound was prepared from 4-isocyanato-1-methyl-1H-indole, azidotrimethylsilane and morpholine-4-carbonyl chloride. MS(m/e): 329.2 (MH⁺).

### Example 133

### 1-(1-methylindol-5-yl)-4-(morpholine-4-carbonyl)tetrazol-5-one

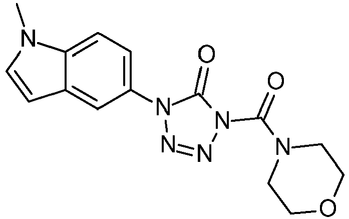

In analogy to the procedure described for the synthesis of 1-(2,6-difluorophenyl)-4-(morpholine-4-carbonyl)tetrazol-5-one (example 122) the title compound was prepared from 5-isocyanato-1-methyl-1H-indole, azidotrimethylsilane and morpholine-4-carbonyl chloride. MS(m/e): 329.2 (MH⁺).

### Example 134

### 1-(1-methylindol-6-yl)-4-(morpholine-4-carbonyl)tetrazol-5-one

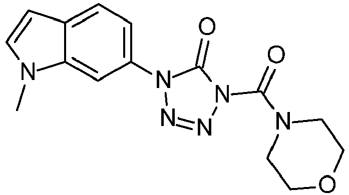

In analogy to the procedure described for the synthesis of 1-(2,6-difluorophenyl)-4-(morpholine-4-carbonyl)tetrazol-5-one (example 122) the title compound was prepared from 6-isocyanato-1-methyl-1H-indole, azidotrimethylsilane and morpholine-4-carbonyl chloride. MS(m/e): 329.3 (MH⁺).

### Example 135

### 1-(3-bromophenyl)-4-(morpholine-4-carbonyl)tetrazol-5-one

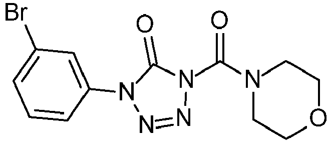

In analogy to the procedure described for the synthesis of 1-(morpholine-4-carbonyl)-4-[2-(trifluoromethyl)phenyl]tetrazol-5-one (example 119) the title compound was prepared from 3-bromobenzoyl chloride, azidotrimethylsilane and morpholine-4-carbonyl chloride. MS(m/e): 354.2 (MH⁺).

### Example 136

### 1-(3-chlorophenyl)-4-[(2R,6S)-2,6-dimethylmorpholine-4-carbonyl]tetrazol-5-one

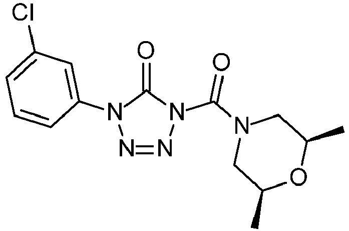

In analogy to the procedure described for the synthesis of 4-(3-Fluoro-phenyl)-5-oxo-4,5-dihydro-tetrazole-1-carboxylic acid (2-methoxy-ethyl)-amide (example 110) the title compound was prepared from 1-(3-chlorophenyl)-1H-tetrazol-5(4H)-one and (2R,6S)-2,6-dimethylmorpholine (cis-2,6-). MS(m/e): 338.3 (MH⁺).

### Example 137

### 1-(3-chlorophenyl)-4-(2,6-dimethylmorpholine-4-carbonyl)tetrazol-5-one

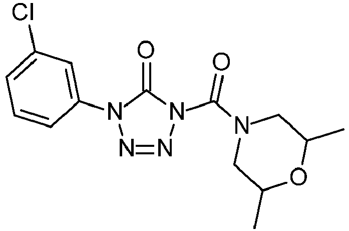

In analogy to the procedure described for the synthesis of 4-(3-Fluoro-phenyl)-5-oxo-4,5-dihydro-tetrazole-1-carboxylic acid (2-methoxy-ethyl)-amide (example 110) the title compound was prepared from 1-(3-chlorophenyl)-1H-tetrazol-5(4H)-one and 2,6-dimethylmorpholine. MS(m/e): 338.3 (MH⁺).

### Example 138

### 1-(3-chlorophenyl)-4-(3,3-dimethylpyrrolidine-1-carbonyl)tetrazol-5-one

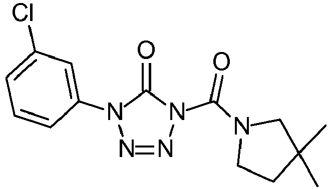

In analogy to the procedure described for the synthesis of 4-(3-Fluoro-phenyl)-5-oxo-4,5-dihydro-tetrazole-1-carboxylic acid (2-methoxy-ethyl)-amide (example 110) the title compound was prepared from 1-(3-chlorophenyl)-1H-tetrazol-5(4H)-one and 3,3-dimethylpyrrolidine. MS(m/e): 322.2 (MH⁺).

### Example 139

### Pharmacological tests

The following tests were carried out in order to determine the activity of the compounds of formula (I):

### Radioligand binding assay

The affinity of the compounds of the invention for cannabinoid CB1 receptors was determined using recommended amounts of membrane preparations (PerkinElmer) of human embryonic kidney (HEK) cells expressing the human CNR1 or CNR2 receptors in conjunction with 1.5 or 2.6 nM [3H]-CP-55,940 (Perkin Elmer) as radioligand, respectively. Binding was performed in binding buffer (50 mM Tris, 5 mM MgCl2, 2.5 mM EDTA, and 0.5% (wt/vol) fatty acid free BSA, pH 7.4 for CB1 receptor and 50 mM Tris, 5 mM MgCl₂, 2.5 mM EGTA, and 0.1% (wt/vol) fatty acid free BSA, pH 7.4 for CB2 receptor) in a total volume of 0.2 ml for 1h at 30 °C shaking. The reaction was terminated by rapid filtration through microfiltration plates coated with 0.5% polyethylenimine (UniFilter GF/B filter plate; Packard). Bound radioactivity was analyzed for Ki using nonlinear regression analysis (Activity Base, ID Business Solution, Limited), with the Kd values for [3H]CP55,940 determined from saturation experiments. The compounds of formula (I) show an excellent affinity for the CB2 receptor with affinities below 10 µM, more particularly of 1 nM to 3µM and most particularly of 1 nM to 100 nM.

### cAMP Assay

CHO cells expressing human CB1 or CB2 receptors are seeded 17-24 hours prior to the experiment 50.000 cells per well in a black 96 well plate with flat clear bottom (Corning Costar #3904) in DMEM (Invitrogen No. 31331), 1x HT supplement, with 10 % fetal calf serum and incubated at 5% CO₂ and 37 °C in a humidified incubator. The growth medium was exchanged with Krebs Ringer Bicarbonate buffer with 1 mM IBMX and incubated at 30 °C for 30 min. Compounds were added to a final assay volume of 100 µl and incubated for 30 min at 30 °C. Using the cAMP-Nano-TRF detection kit the assay (Roche Diagnostics) was stopped by the addition of 50 µl lysis reagent (Tris, NaCl, 1.5% Triton X100, 2.5% NP40, 10% NaN₃) and 50 µl detection solutions (20 µM mAb Alexa700-cAMP 1:1, and 48 µM Ruthenium-2-AHA-cAMP) and shaken for 2h at room temperature. The time-resolved energy transfer is measured by a TRF reader (Evotec Technologies GmbH), equipped with a ND:YAG laser as excitation source. The plate is measured twice with the excitation at 355 nm and at the emission with a delay of 100 ns and a gate of 100 ns, total exposure time 10s at 730 (bandwidth30 nm) or 645 nm (bandwidth 75 nm), respectively. The FRET signal is calculated as follows: FRET = T730-Alexa730-P(T645-B645) with P = Ru730-B730/Ru645-B645, where T730 is the test well measured at 730 nM, T645 is the test well measured at 645 nm, B730 and B645 are the buffer controls at 730 nm and 645 nm, respectively. cAMP content is determined from the function of a standard curve spanning from 10 µM to 0.13 nM cAMP.

EC₅₀ values were determined using Activity Base analysis (ID Business Solution, Limited). The EC₅₀ values for a wide range of cannabinoid agonists generated from this assay were in agreement with the values published in the scientific literature.

The compounds of the invention are CB2 receptor agonists with EC₅₀ below 1 µM and selectivity versus CB1 in the corresponding assay of at least 10 fold. Particular compound of the invention are CB2 receptor agonists with EC₅₀ below 0.05 µM and selectivity versus CB1 in the corresponding assay of at least 500 fold.

For example, the following compounds showed the following human EC₅₀ values (uM) in the functional cAMP assay described above:

| **Example** | **Ec50:CB2 - hu cAMP** | **Ec50:CB1** - **hu cAMP** | | **Example** | **Ec50:CB2 - hu cAMP** | **Ec50:CB1** - **hu cAMP** |
|---|---|---|---|---|---|---|
| **1** | 0.0006 | >10 | | **70** | 0.0321 | >10 |
| **2** | 0.0115 | >10 | | **71** | 0.0167 | >10 |
| **3** | 0.0159 | >10 | | **72** | 0.027 | >10 |
| **4** | 0.003 | >10 | | **73** | 0.0108 | >10 |
| **5** | 0.0008 | >10 | | **74** | 0.0097 | >10 |
| **6** | 0.0008 | >10 | | **75** | 0.0285 | >10 |
| **7** | 0.0027 | >10 | | **76** | 0.0074 | >10 |
| **8** | 0.0012 | >10 | | **77** | 0.0006 | >10 |
| **9** | 0.0041 | >10 | | **78** | 0.0015 | >10 |
| **10** | 0.0059 | >10 | | **79** | 0.0009 | >10 |
| **11** | 0.0789 | >10 | | **80** | 0.0024 | >10 |
| **12** | 0.0006 | >10 | | **81** | 0.004 | >10 |
| **13** | 0.0112 | >10 | | **82** | 0.0033 | >10 |
| **14** | 1.1837 | >10 | | **83** | 0.0053 | >10 |
| **15** | 2.9981 | >10 | | **84** | 0.0095 | >10 |
| **16** | 0.3266 | >10 | | **85** | 0.0079 | >10 |
| **17** | 0.8982 | >10 | | **86** | 0.001 | >10 |
| **18** | 0.2192 | >10 | | **87** | 0.0036 | >10 |
| **19** | 0.025 | >10 | | **88** | 0.0019 | >10 |
| **20** | 0.0069 | >10 | | **89** | 0.0169 | >10 |
| **21** | 0.0975 | >10 | | **90** | 0.067 | >10 |
| **22** | 1.9192 | >10 | | **91** | 0.0428 | >10 |
| **23** | 0.0314 | >10 | | **92** | 0.0164 | >10 |
| **24** | 0.1218 | >10 | | **93** | 0.0242 | >10 |
| **25** | 0.026 | >10 | | **94** | 0.0211 | >10 |
| **26** | 0.0178 | >10 | | **95** | 0.0047 | >10 |
| **27** | 0.0071 | >10 | | **96** | 0.0182 | >10 |
| **28** | 0.0008 | >10 | | **97** | 0.0129 | >10 |
| **29** | 0.0416 | >10 | | **98** | 0.0018 | >10 |
| **30** | 0.2052 | >10 | | **99** | 0.0125 | >10 |
| **31** | 0.1657 | >10 | | **100** | 0.0017 | >10 |
| **32** | 0.1517 | >10 | | **101** | 0.0032 | >10 |
| **33** | 0.025 | >10 | | **102** | 0.0044 | >10 |
| **34** | 0.019 | >10 | | **103** | 0.0045 | >10 |
| **35** | 0.1611 | >10 | | **104** | 0.0127 | >10 |
| **36** | 0.8742 | >10 | | **105** | 0.0201 | >10 |
| **37** | 0.4069 | >10 | | **106** | 0.0106 | >10 |
| **38** | 0.092 | >10 | | **107** | 0.0029 | >10 |
| **39** | 0.4974 | >10 | | **108** | 0.0139 | >10 |
| **40** | 0.0528 | >10 | | **109** | 0.0042 | >10 |
| **41** | 0.0032 | >10 | | **110** | 0.9677 | >10 |
| **42** | 0.0911 | >10 | | **111** | 0.0015 | >10 |
| **43** | 0.0258 | >10 | | **112** | 0.0041 | >10 |
| **44** | 0.0138 | >10 | | **113** | 0.0014 | >10 |
| **45** | 0.7974 | >10 | | **114** | 1.9916 | >10 |
| **46** | 0.0902 | >10 | | **115** | 2.0158 | >10 |
| **47** | 0.0027 | >10 | | **116** | 0.1219 | >10 |
| **48** | 0.0067 | >10 | | **117** | 0.1064 | >10 |
| **49** | 0.0143 | >10 | | **118** | 0.1142 | >10 |
| **50** | 0.5672 | >10 | | **119** | 0.386 | >10 |
| **51** | 0.1535 | >10 | | **120** | 0.0092 | >10 |
| **52** | 0.1291 | >10 | | **121** | 0.016 | >10 |
| **53** | 0.001 | >10 | | **122** | 0.019 | >10 |
| **54** | 0.0013 | >10 | | **123** | 0.012 | >10 |
| **55** | 0.0041 | >10 | | **124** | 0.019 | >10 |
| **56** | 0.017 | >10 | | **125** | 0.007 | >10 |
| **57** | 0.0085 | >10 | | **126** | 0.013 | >10 |
| **58** | 0.0454 | >10 | | **127** | 0.0084 | >10 |
| **59** | 0.9879 | >10 | | **128** | 0.0066 | >10 |
| **60** | 0.0013 | >10 | | **129** | 0.0091 | >10 |
| **61** | 0.0068 | >10 | | **130** | 0.0067 | >10 |
| **62** | 0.0614 | >10 | | **131** | 0.016 | >10 |
| **63** | 0.0018 | >10 | | **132** | 0.0076 | >10 |
| **64** | 0.0025 | >10 | | **133** | 0.018 | >10 |
| **65** | 0.015 | >10 | | **134** | 0.008 | >10 |
| **66** | 0.0204 | >10 | | **135** | 0.007 | >10 |
| **67** | 0.0146 | >10 | | **136** | 0.507 | >10 |
| **68** | 0.0395 | >10 | | **137** | 0.13 | >10 |
| **69** | 0.0182 | >10 | | **138** | 0.005 | >10 |

### Example A

Film coated tablets containing the following ingredients can be manufactured in a conventional manner:

| Ingredients | Per tablet | |
|---|---|---|
| **Kernel:** | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |

| **Film Coat:** | | |
|---|---|---|
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titan dioxide | 0.8 mg | 1.6 mg |

The active ingredient is sieved and mixed with microcrystalline cellulose and the mixture is granulated with a solution of polyvinylpyrrolidone in water. The granulate is then mixed with sodium starch glycolate and magnesium stearate and compressed to yield kernels of 120 or 350 mg respectively. The kernels are lacquered with an aq. solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Polyethylene glycol 400 | 150.0 mg |
| Acetic acid | q.s. ad pH 5.0 |
| Water for injection solutions | ad 1.0 ml |

The active ingredient is dissolved in a mixture of Polyethylene glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

## Claims

1. A compound of formula (I) 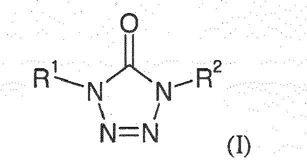 wherein
R¹ is n-propyl, iso-butyl, cyclopropyl, 3-fluorophenyl, 6-chloropyridin-3-yl, 3-chlorophenyl, 4-chlorophenyl, 3-trifluoromethoxyphenyl, 2-(trifluoromethyl)phenyl, 3-(trifluoromethyl)phenyl, 4-(trifluoromethyl)phenyl, 2,6-difluorophenyl, 2,5-difluorophenyl, 3,4-difluorophenyl, 3,5-difluorophenyl, 3-methylphenyl, 4-methylphenyl, 3-methoxyphenyl, 4-methoxyphenyl, 2,3-dichlorophenyl, 1-methylindolyl or 3-bromophenyl;
R² is -C(O)NR³R⁴ or R⁵;
one of R³ and R⁴ is hydrogen, alkyl, cycloalkyl or alkoxyalkyl and the other one is -(CH₂)ₙ-R⁶;
or R³ and R⁴ together with the nitrogen atom to which they are attached form heterocyclyl or substituted heterocyclyl, wherein heterocyclyl is piperidinyl, morpholinyl, pyrrolidinyl, 3,4-dihydro-1H-isoquinolinyl, azetidinyl, piperazinyl, 1,1-dioxothiomorpholinyl or 2-oxa-8-aza-spiro[4.5]decyl, and wherein substituted heterocyclyl is heterocyclyl substituted with one substituent selected from halogen, alkyl, cyano, alkoxyalkyl, aminocarbonyl, dialkylaminocarbonyl, dialkylaminocarbonylalkyl, alkylaminocarbonylalkyl, phenyl, halophenyl, phenylalkyl, halophenylalkyl, methylpyrazolyl, methylisoxazolyl, alkoxyalkyl, alkylcarbonylamino, alkylcarbonyl, alkoxycarbonyl, alkyl-[1,2,4]oxadiazolyl, pyrrolidinylcarbonylalkyl, pyrazinyl, (alkyl)(alkylcarbonyl)amino, alkylisoxazolyl and morpholinylcarbonylalkyl, or with two substituents independently selected from alkyl, halogen, alkoxycarbonyl and alkoxycarbonylamino;
R⁵ is halophenylalkyl, alkoxycarbonylpiperidinyl, alkoxycarbonylalkyl, (alkylsulfonyl)(alkyl)[1,2,4]triazolylalkyl or morpholinylcarbonylalkyl;
R⁶ is alkoxy, dialkoxyphenyl, cyano, phenyl, pyridinyl or alkoxycarbonylalkyl; and
n is 0, 1, 2 or 3;
or a pharmaceutically acceptable salt therof;
provided that
1-Cyclopropyl-4-(pyrrolidine-1-carbonyl)-1,4-dihydro-tetrazol-5-one;
1-Cyclopropyl-4-(2-methyl-pyrrolidine-1-carbonyl)-1,4-dihydro-tetrazol-5-one;
1-Cyclopropyl-4-(2,5-dimethyl-pyrrolidine-1-carbonyl)-1,4-dihydro-tetrazol-5-one;
1-Cyclopropyl-4-(2,6-dimethyl-piperidine-1-carbonyl)-1,4-dihydro-tetrazol-5-one;
4-Cyclopropyl-5-oxo-4,5-dihydro-tetrazole-1-carboxylic acid benzyl-isopropylamide;
[4-(4-Chloro-phenyl)-5-oxo-4,5-dihydro-tetrazol-1-yl]-acetic acid ethyl ester;
2-[4-(4-Chloro-phenyl)-5-oxo-4,5-dihydro-tetrazol-1-yl]-propionic acid ethyl ester;
3-[4-(3-Chloro-phenyl)-5-oxo-4,5-dihydro-tetrazol-1-yl]-propionic acid methyl ester;
1-(2,3-Dichloro-phenyl)-4-(2-methyl-piperidine-1-carbonyl)-1,4-dihydro-tetrazol-5-one;
1-(2,6-difluorophenyl)-4-(3,4-dihydro-1H-isoquinoline-2-carbonyl)tetrazol-5-one;
4-(2,6-difluorophenyl)-5-oxo-N-phenyl-N-propyl-tetrazole-1-carboxamide;
4-(2,6-difluorophenyl)-N-ethyl-5-oxo-N-phenyl-tetrazole-1-carboxamide;
4-(2,6-difluorophenyl)-N-methyl-5-oxo-N-phenyl-tetrazole-1-carboxamide;
4-(2,6-difluorophenyl)-N-isopropyl-5-oxo-N-phenyl-tetrazole-1-carboxamide;
N-cyclohexyl-4-(2,6-difluorophenyl)-5-oxo-N-phenyl-tetrazole-1-carboxamide;
4-(2,6-difluorophenyl)-N-methyl-5-oxo-N-(2-pyridyl)tetrazole-1-carboxamide;
N,4-dicyclopropyl-5-oxo-N-phenyl-tetrazole-1-carboxamide;
N-butyl-4-cyclopropyl-5-oxo-N-phenyl-tetrazole-1-carboxamide;
4-cyclopropyl-N-isobutyl-5-oxo-N-phenyl-tetrazole-1-carboxamide;
4-cyclopropyl-N-isopropyl-5-oxo-N-phenyl-tetrazole-1-carboxamide;
4-cyclopropyl-5-oxo-N-phenyl-N-propyl-tetrazole-1-carboxamide;
4-cyclopropyl-N-methyl-5-oxo-N-phenyl-tetrazole-1-carboxamide;
4-cyclopropyl-N-ethyl-5-oxo-N-phenyl-tetrazole-1-carboxamide;
4-cyclopropyl-5-oxo-N-phenyl-N-sec-butyl-tetrazole-1-carboxamide;
N-(1-benzyl-2-methyl-propyl)-4-cyclopropyl-5-oxo-N-phenyl-tetrazole-1-carboxamide;
1-(2-methylpiperidine-1-carbonyl)-4-(p-tolyl)tetrazol-5-one;
1-(2-methylpiperidine-1-carbonyl)-4-(m-tolyl)tetrazol-5-one;
N-isopropyl-5-oxo-N-phenyl-4-propyl-tetrazole-1-carboxamide;
1-(4-chlorophenyl)-4-[(2-fluorophenyl)methyl]tetrazol-5-one;
1-(4-chlorophenyl)-4-[(3-fluorophenyl)methyl]tetrazol-5-one;
1-(4-chlorophenyl)-4-[(3-chlorophenyl)methyl]tetrazol-5-one;
1-[(4-bromophenyl)methyl]-4-(4-chlorophenyl)tetrazol-5-one; and
methyl 3-[5-oxo-4-(p-tolyl)tetrazol-1-yl]propanoate;
are excluded;
wherein
"alkyl", alone or in combination, signifies a straight-chain or branched-chain alkyl group with 1 to 8 carbon atoms;
"cycloalkyl", alone or in combination, signifies a cycloalkyl ring with 3 to 8 carbon atoms; and
"alkoxy", alone or in combination, signifies a group of the formula alkyl-O- in which the term "alkyl" has the previously given significance.

2. A compound according to claim 1, wherein R¹ is 3-fluorophenyl, 3-chlorophenyl or 4-chlorophenyl.

3. A compound according to claim 1 or 2, wherein one of R³ and R⁴ is alkyl and the other one is -(CH₂)ₙ-R⁶.

4. A compound according to any one of claims 1 to 3, wherein one of R³ and R⁴ is methyl or ethyl and the other one is -(CH₂)ₙ-R⁶.

5. A compound according to any one of claims 1 and 2, wherein R³ and R⁴ together with the nitrogen atom to which they are attached form heterocyclyl or substituted heterocyclyl, wherein heterocyclyl is piperidinyl, pyrrolidinyl or morpholinyl, and wherein substituted heterocyclyl is heterocyclyl substituted with one substitutent selected from alkyl and alkylcarbonylamino, or with two substituents independently selected from alkyl.

6. A compound according to any one of claims 1, 2 and 5, wherein R³ and R⁴ together with the nitrogen atom to which they are attached form morpholinyl, dimethylpiperidinyl or methylcarbonylaminopyrrolidinyl.

7. A compound according to any one of claims 1 and 2, wherein R⁵ is tert.-butyloxycarbonylpiperidinyl, fluorophenylmethyl, ethoxycarbonylmethyl, ethoxycarbonyl(dimethyl)methyl, (methylsulfonyl)(methyl)[1,2,4]triazolyl or morpholinylcarbonylmethyl.

8. A compound according to any one of claims 1 to 4, wherein R⁶ is alkoxy, dialkoxyphenyl, phenyl or pyridinyl.

9. A compound according to any one of claims 1 to 4 and 8, wherein R⁶ is methoxy, dimethoxyphenyl, phenyl or pyridinyl.

10. A compound according to any one of claims 1 to 4, 8 and 9, wherein n is 1, 2 or 3.

11. A compound according to any one of claims 1 to 10 selected from
4-(3-Fluoro-phenyl)-5-oxo-4,5-dihydro-tetrazole-1-carboxylic acid [2-(3,4-dimethoxy-phenyl)-ethyl]-methyl-amide;
4-(3-Fluoro-phenyl)-5-oxo-4,5-dihydro-tetrazole-l-carboxylic acid (2-cyano-ethyl)-methyl-amide;
1-[4-(3-Fluoro-phenyl)-5-oxo-4,5-dihydro-tetrazole-1-carbonyl]-piperidine-3-carboxylic acid amide;
1-(3-Fluoro-phenyl)-4-(morpholine-4-carbonyl)-1,4-dihydro-tetrazol-5-one;
4-(3-Fluoro-phenyl)-5-oxo-4,5-dihydro-tetrazole-1-carboxylic acid methylphenethyl-amide;
4-(3-Fluoro-phenyl)-5-oxo-4,5-dihydro-tetrazole-l-carboxylic acid ethyl-pyridin-4-ylmethyl-amide;
4-(3-Fluoro-phenyl)-5-oxo-4,5-dihydro-tetrazole-1-carboxylic acid ethyl-(2-methoxy-ethyl)-amide;
4-(3-Fluoro-phenyl)-5-oxo-4,5-dihydro-tetrazole-1-carboxylic acid methyl-(3-phenyl-propyl)-amide;
1-(2-Benzyl-pyrrolidine-1-carbonyl)-4-(3-fluoro-phenyl)-1,4-dihydro-tetrazol-5-one;
1-(3-Fluoro-phenyl)-4-[2-(4-fluoro-phenyl)-pyrrolidine-1-carbonyl]-1,4-dihydro-tetrazol-5-one;
1-(6-Fluoro-3,4-dihydro-1H-isoquinoline-2-carbonyl)-4-(3-fluoro-phenyl)-1,4-dihydro-tetrazol-5-one;
4-(3-Fluoro-phenyl)-5-oxo-4,5-dihydro-tetrazole-1-carboxylic acid benzyl-ethylamide;
(2S,3S)-2-{[4-(3-Fluoro-phenyl)-5-oxo-4,5-dihydro-tetrazole-1-carbonyl]-methyl-amino}-3-methyl-pentanoic acid methyl ester;
1-(4-Fluoro-benzyl)-4-(3-fluoro-phenyl)-1,4-dihydro-tetrazol-5-one;
4-[4-(3-Fluoro-phenyl)-5-oxo-4,5-dihydro-tetrazol-1-yl]-piperidine-1-carboxylic acid tert-butyl ester;
2-[4-(3-Fluoro-phenyl)-5-oxo-4,5-dihydro-tetrazol-1-yl]-2-methyl-propionic acid ethyl ester;
1-(3-Fluoro-phenyl)-4-(5-methanesulfonyl-4-methyl-4H-[1,2,4]triazol-3-ylmethyl)-1,4-dihydro-tetrazol-5-one;
1-(3-Fluoro-phenyl)-4-[2-(2-methyl-2H-pyrazol-3-yl)-pyrrolidine-1-carbonyl]-1,4-dihydro-tetrazol-5-one;
1-(3-Fluoro-phenyl)-4-[2-(1-methyl-1H-pyrazol-3-yl)-pyrrolidine-1-carbonyl]-1,4-dihydro-tetrazol-5-one;
1-(3-Fluoro-phenyl)-4-[2-(3-methyl-isoxazol-5-yl)-pyrrolidine-1-carbonyl]-1,4-dihydro-tetrazol-5-one;
1-(3-Fluoro-phenyl)-4-((S)-2-methoxymethyl-pyrrolidine-1-carbonyl)-1,4-dihydro-tetrazol-5-one;
(S)-1-[4-(3-Fluoro-phenyl)-5-oxo-4,5-dihydro-tetrazole-1-carbonyl]-pyrrolidine-2-carboxylic acid amide;
N-{1-[4-(3-Fluoro-phenyl)-5-oxo-4,5-dihydro-tetrazole-1-carbonyl]-pyrrolidin-3-yl}-acetamide;
1-[4-(3-Fluoro-phenyl)-5-oxo-4,5-dihydro-tetrazole-1-carbonyl]-azetidine-3-carboxylic acid methyl ester;
1-(3-Fluoro-phenyl)-4-[2-(3-methyl-[1,2,4]oxadiazol-5-yl)-pyrrolidine-1-carbonyl]-1,4-dihydro-tetrazol-5-one;
N-{1-[4-(3-Fluoro-phenyl)-5-oxo-4,5-dihydro-tetrazole-1-carbonyl]-piperidin-4-yl}-acetamide;
1-(3-Fluoro-phenyl)-4-[4-(2-oxo-2-pyrrolidin-1-yl-ethyl)-piperazine-1-carbonyl]-1,4-dihydro-tetrazol-5-one;
1-(4,4-Dimethyl-piperidine-1-carbonyl)-4-(3-fluoro-phenyl)-1,4-dihydro-tetrazol-5-one;
1-(1,1-Dioxo-1λ6-thiomorpholine-4-carbonyl)-4-(3-fluoro-phenyl)-1,4-dihydro-tetrazol-5-one;
1-(3-Fluoro-phenyl)-4-(2,3,5,6-tetrahydro-[1,2']bipyrazinyl-4-carbonyl)-1,4-dihydro-tetrazol-5-one;
4-(3-Fluoro-phenyl)-5-oxo-4,5-dihydro-tetrazole-1-carboxylic acid (2-cyano-ethyl)-cyclopropyl-amide;
4-(3-Fluoro-phenyl)-5-oxo-4,5-dihydro-tetrazole-1-carboxylic acid methyl-pyridin-2-yl-amide;
4-(3-Fluoro-phenyl)-5-oxo-4,5-dihydro-tetrazole-1-carboxylic acid methyl-pyridin-3-yl-amide;
1-(3-Fluoro-phenyl)-4-(2-methoxymethyl-piperidine-1-carbonyl)-1,4-dihydro-tetrazol-5-one;
4-Cyclopropyl-5-oxo-4,5-dihydro-tetrazole-1-carboxylic acid methyl-pyridin-3-yl-amide;
5-Oxo-4-propyl-4,5-dihydro-tetrazole-1-carboxylic acid methyl-pyridin-3-yl-amide;
4-Isobutyl-5-oxo-4,5-dihydro-tetrazole-1-carboxylic acid methyl-pyridin-3-yl-amide;
1-(4-Acetyl-piperazine-1-carbonyl)-4-(3-fluoro-phenyl)-1,4-dihydro-tetrazol-5-one;
1-(3-Fluoro-phenyl)-4-(4-propionyl-piperazine-1-carbonyl)-1,4-dihydro-tetrazol-5-one;
4-[4-(3-Fluoro-phenyl)-5-oxo-4,5-dihydro-tetrazole-1-carbonyl]-piperazine-1-carboxylic acid diethylamide;
1-(3-Fluoro-phenyl)-4-(3-phenyl-pyrrolidine-1-carbonyl)-1,4-dihydro-tetrazol-5-one;
N-{(S)-1-[4-(3-Fluoro-phenyl)-5-oxo-4,5-dihydro-tetrazole-1-carbonyl]-pyrrolidin-3-yl}-acetamide;
N-{(R)-1-[4-(3-Fluoro-phenyl)-5-oxo-4,5-dihydro-tetrazole-1-carbonyl]-pyrrolidin-3-yl}-acetamide;
N-Ethyl-N-{1-[4-(3-fluoro-phenyl)-5-oxo-4,5-dihydro-tetrazole-1-carbonyl]-pyrrolidin-3-yl}-acetamide;
1-(3,3-Difluoro-pyrrolidine-1-carbonyl)-4-(3-fluoro-phenyl)-1,4-dihydro-tetrazol-5-one;
1-(2,2-Dimethyl-pyrrolidine-1-carbonyl)-4-(3-fluoro-phenyl)-1,4-dihydro-tetrazol-5-one;
1-(3,3-Dimethyl-pyrrolidine-1-carbonyl)-4-(3-fluoro-phenyl)-1,4-dihydro-tetrazol-5-one;
1-(3-Fluoro-phenyl)-4-(2-methyl-pyrrolidine-1-carbonyl)-1,4-dihydro-tetrazol-5-one;
N-{1-[4-(3-Fluoro-phenyl)-5-oxo-4,5-dihydro-tetrazole-1-carbonyl]-pyrrolidin-3-yl}-N-methyl-acetamide;
4-[4-(3-Fluoro-phenyl)-5-oxo-4,5-dihydro-tetrazole-1-carbonyl]-piperazine-1-carboxylic acid ethyl ester;
(S)-1-[4-(3-Fluoro-phenyl)-5-oxo-4,5-dihydro-tetrazole-1-carbonyl]-pyrrolidine-2-carbonitrile;
(R)-1-[4-(3-Fluoro-phenyl)-5-oxo-4,5-dihydro-tetrazole-1-carbonyl]-pyrrolidine-2-carbonitrile;
4-(6-Chloro-pyridin-3-yl)-5-oxo-4,5-dihydro-tetrazole-1-carboxylic acid benzyl-ethyl-amide;
4-(3-Fluoro-phenyl)-5-oxo-4,5-dihydro-tetrazole-1-carboxylic acid (2-methoxyethyl)-methyl-amide;
4-(3-Fluoro-phenyl)-5-oxo-4,5-dihydro-tetrazole-1-carboxyl acid isopropyl-(2-methoxy-ethyl)-amide;
4-(3-Fluoro-phenyl)-5-oxo-4,5-dihydro-tetrazole-1-carboxylic acid (2-methoxyethyl)-propyl-amide;
4-(3-Fluoro-phenyl)-5-oxo-4,5-dihydro-tetrazole-1-carboxylic acid bis-(2-methoxyethyl)-amide;
1-(2,6-Dimethyl-moipholine-4-carbonyl)-4-(3-fluoro-phenyl)-1,4-dihydro-tetrazol-5-one;
1-((2S,6R)-2,6-Dimethyl-morpholine-4-carbonyl)-4-(3-fluoro-phenyl)-1,4-dihydro-tetrazol-5-one;
1-(3-Fluoro-phenyl)-4-(4-methoxymethyl-piperidine-1-carbonyl)-1,4-dihydro-tetrazol-5-one;
1-[4-(3-Fluoro-phenyl)-5-oxo-4,5-dihydro-tetrazole-1-carbonyl]-piperidine-4-carbonitrile;
4-tert-Butoxycarbonylamino-1-[4-(3-fluoro-phenyl)-5-oxo-4,5-dihydro-tetrazole-1-carbonyl]-piperidine-4-carboxylic acid methyl ester;
1-(3,3-Dimethyl-piperidine-1-carbonyl)-4-(3-fluoro-phenyl)-1,4-dihydro-tetrazol-5-one;
1-(3-Fluoro-phenyl)-4-(2-oxa-8-aza-spiro[4.5]decane-8-carbonyl)-1,4-dihydro-tetrazol-5-one;
N-{(R)-1-[4-(3-Chloro-phenyl)-5-oxo-4,5-dihydro-tetrazole-1-carbonyl]-pyrrolidin-3-yl}-acetamide;
N-{(R)-1-[5-Oxo-4-(3-trifluoromethoxy-phenyl)-4,5-dihydro-tetrazole-1-carbonyl]-pyrrolidin-3-yl}-acetamide;
N-{(R)-1-[4-(4-Chloro-phenyl)-5-oxo-4,5-dihydro-tetrazole-1-carbonyl]-pyrrolidin-3-yl}-acetamide;
N-{(S)-1-[4-(3-Chloro-phenyl)-5-oxo-4,5-dihydro-tetrazole-1-carbonyl]-pyrrolidin-3-yl}-acetamide;
N-{(S)-1-[5-Oxo-4-(3-trifluoromethoxy-phenyl)-4,5-dihydro-tetrazole-1-carbonyl]-pyrrolidin-3-yl}-acetamide;
N-{(S)-1-[4-(4-Chloro-phenyl)-5-oxo-4,5-dihydro-tetrazole-1-carbonyl]-pyrrolidin-3-yl}-acetamide;
N-{1-[4-(3-Chloro-phenyl)-5-oxo-4,5-dihydro-tetrazole-1-carbonyl]-pyrrolidin-3-yl}-N-ethyl-acetamide;
N-Ethyl-N-{1-[5-oxo-4-(3-trifluoromethoxy-phenyl)-4,5-dihydro-tetrazole-1-carbonyl]-pyrrolidin-3-yl}-acetamide;
N-{1-[4-(4-Chloro-phenyl)-5-oxo-4,5-dihydro-tetrazole-1-carbonyl]-pyrrolidin-3-yl}-N-ethyl-acetamide;
1-(3-Chloro-phenyl)-4-[2-(3-methyl-isoxazol-5-yl)-pyrrolidine-1-carbonyl]-1,4-dihydro-tetrazol-5-one;
1-[2-(3-Methyl-isoxazol-5-yl)-pyrrolidine-1-carbonyl]-4-(3-trifluoromethoxy-phenyl)-1,4-dihydro-tetrazol-5-one;
1-(4-Chloro-phenyl)-4-[2-(3-methyl-isoxazol-5-yl)-pyrrolidine-1-carbonyl]-1,4-dihydro-tetrazol-5-one;
1-(3-Chloro-phenyl)-4-(4,4-dimethyl-piperidine-1-carbonyl)-1,4-dihydro-tetrazol-5-one;
1-(4,4-Dimethyl-piperidine-1-carbonyl)-4-(3-trifluoromethoxy-phenyl)-1,4-dihydro-tetrazol-5-one;
1-(4-Chloro-phenyl)-4-(4,4-dimethyl-piperidine-1-carbonyl)-1,4-dihydro-tetrazol-5-one;
4-(3-Chloro-phenyl)-5-oxo-4,5-dihydro-tetrazole-1-carboxylic acid ethyl-(2-methoxy-ethyl)-amide;
5-Oxo-4-(3-trifluoromethoxy-phenyl)-4,5-dihydro-tetrazole-1-carboxylic acid ethyl-(2-methoxy-ethyl)-amide;
4-(4-Chloro-phenyl)-5-oxo-4,5-dihydro-tetrazole-1-carboxylic acid ethyl-(2-methoxy-ethyl)-amide;
4-(3-Chloro-phenyl)-5-oxo-4,5-dihydro-tetrazole-1-carboxylic acid isopropyl-(2-methoxy-ethyl)-amide;
5-Oxo-4-(3-trifluoromethoxy-phenyl)-4,5-dihydro-tetrazole-1-carboxylic acid isopropyl-(2-methoxy-ethyl)-amide;
4-(4-Chloro-phenyl)-5-oxo-4,5-dihydro-tetrazole-1-carboxylic acid isopropyl-(2-methoxy-ethyl)-amide;
4-(3-Chloro-phenyl)-5-oxo-4,5-dihydro-tetrazole-1-carboxylic acid (3-methoxypropyl)-methyl-amide;
5-Oxo-4-(3-trifluoromethoxy-phenyl)-4,5-dihydro-tetrazole-1-carboxylic acid (3-methoxy-propyl)-methyl-amide;
4-(4-Chloro-phenyl)-5-oxo-4,5-dihydro-tetrazole-1-carboxylic acid (3-methoxy-propyl)-methyl-amide;
1-(3-Chloro-phenyl)-4-[4-(2-morpholin-4-yl-2-oxo-ethyl)-piperazine-1-carbonyl]-1,4-dihydro-tetrazol-5-one;
1-[4-(2-Morpholin-4-yl-2-oxo-ethyl)-piperazine-1-carbonyl]-4-(3-trifluoromethoxy-phenyl)-1,4-dihydro-tetrazol-5-one;
1-(4-Chloro-phenyl)-4-[4-(2-morpholin-4-yl-2-oxo-ethyl)-piperazine-1-carbonyl]-1,4-dihydro-tetrazol-5-one;
2-{4-[4-(3-Chloro-phenyl)-5-oxo-4,5-dihydro-tetrazole-1-carbonyl]-piperazin-1-yl}-N,N-dimethyl-acetamide;
N,N-Dimethyl-2-{4-[5-oxo-4-(3-trifluoromethoxy-phenyl)-4,5-dihydro-tetrazole-1-carbonyl]-piperazin-1-yl}-acetamide;
2-{4-[4-(4-Chloro-phenyl)-5-oxo-4,5-dihydro-tetrazole-1-carbonyl]-piperazin-1-yl}-N,N-dimethyl-acetamide;
1-(3-Chloro-phenyl)-4-[4-(2-oxo-2-pyrrolidin-1-yl-ethyl)-piperazine-1-carbonyl]-1,4-dihydro-tetrazol-5-one;
1-[4-(2-Oxo-2-pyrrolidin-1-yl-ethyl)-piperazine-1-carbonyl]-4-(3-trifluoromethoxy-phenyl)-1,4-dihydro-tetrazol-5-one;
1-(4-Chloro-phenyl)-4-[4-(2-oxo-2-pyrrolidin-1-yl-ethyl)-piperazine-1-carbonyl]-1,4-dihydro-tetrazol-5-one;
4-(3-Chloro-phenyl)-5-oxo-4,5-dihydro-tetrazole-1-carboxylic acid (2-ethoxy-ethyl)-ethyl-amide;
5-Oxo-4-(3-trifluoromethoxy-phenyl)-4,5-dihydro-tetrazole-1-carboxylic acid (2-ethoxy-ethyl)-ethyl-amide;
4-(4-Chloro-phenyl)-5-oxo-4,5-dihydro-tetrazole-1-carboxylic acid (2-ethoxy-ethyl)-ethyl-amide;
1-(3-Chloro-phenyl)-4-(morpholine-4-carbonyl)-1,4-dihydro-tetrazol-5-one;
1-(Morpholine-4-carbonyl)-4-(3-trifluoromethoxy-phenyl)-1,4-dihydro-tetrazol-5-one;
1-(4-Chloro-phenyl)-4-(morpholine-4-carbonyl)-1,4-dihydro-tetrazol-5-one;
2-{4-[4-(3-Chloro-phenyl)-5-oxo-4,5-dihydro-tetrazole-1-carbonyl]-piperazin-1-yl}-N-ethyl-acetamide;
N-Ethyl-2-{4-[5-oxo-4-(3-trifluoromethoxy-phenyl)-4,5-dihydro-tetrazole-1-carbonyl]-piperazin-1-yl}-acetamide;
2-{4-[4-(4-Chloro-phenyl)-5-oxo-4,5-dihydro-tetrazole-1-carbonyl]-piperazin-1-yl}-N-ethyl-acetamide;
N-Butyl-2-{4-[4-(3-chloro-phenyl)-5-oxo-4,5-dihydro-tetrazole-1-carbonyl]-piperazin-1-yl}-acetamide;
N-Butyl-2-{4-[5-oxo-4-(3-trifluoromethoxy-phenyl)-4,5-dihydro-tetrazole-1-carbonyl]-piperazin-1-yl}-acetamide;
N-Butyl-2-{4-[4-(4-chloro-phenyl)-5-oxo-4,5-dihydro-tetrazole-1-carbonyl]-piperazin-1-yl}-acetamide;
4-(3-Fluoro-phenyl)-5-oxo-4,5-dihydro-tetrazole-1-carboxylic acid (2-methoxyethyl)-amide;
4-(3-Chloro-phenyl)-5-oxo-4,5-dihydro-tetrazole-1-carboxylic acid (2-ethoxy-ethyl)-methyl-amide;
5-Oxo-4-(3-trifluoromethoxy-phenyl)-4,5-dihydro-tetrazole-1-carboxylic acid (2-ethoxy-ethyl)-methyl-amide;
4-(4-Chloro-phenyl)-5-oxo-4,5-dihydro-tetrazole-1-carboxylic acid (2-ethoxy-ethyl)-methyl-amide;
[4-(3-Chloro-phenyl)-5-oxo-4,5-dihydro-tetrazol-1-yl]-acetic acid ethyl ester;
1-(3-Chloro-phenyl)-4-(2-morpholin-4-yl-2-oxo-ethyl)-1,4-dihydro-tetrazol-5-one;
1-(3-Chloro-phenyl)-4-[4-(1-methyl-2-oxo-2-pyrrolidin-1-yl-ethyl)-piperazine-1-carbonyl]-1,4-dihydro-tetrazol-5-one;
1-[4-(1-Methyl-2-oxo-2-pyrrolidin-1-yl-ethyl)-piperazine-1-carbonyl]-4-(3-trifluoromethoxy-phenyl)-1,4-dihydro-tetrazol-5-one;
1-(4-Chloro-phenyl)-4-[4-(1-methyl-2-oxo-2-pyrrolidin-1-yl-ethyl)-piperazine-1-carbonyl]-1,4-dihydro-tetrazol-5-one;
1-(morpholine-4-carbonyl)-4-[2-(trifluoromethyl)phenyl]tetrazol-5-one;
1-(morpholine-4-carbonyl)-4-[3-(trifluoromethyl)phenyl]tetrazol-5-one;
1-(morpholine-4-carbonyl)-4-[4-(trifluoromethyl)phenyl]tetrazol-5-one;
1-(2,6-difluorophenyl)-4-(morpholine-4-carbonyl)tetrazol-5-one;
1-(2,4-difluorophenyl)-4-(morpholine-4-carbonyl)tetrazol-5-one;
1-(2,5-difluorophenyl)-4-(morpholine-4-carbonyl)tetrazol-5-one;
1-(3,4-difluorophenyl)-4-(morpholine-4-carbonyl)tetrazol-5-one;
1-(3,5-difluorophenyl)-4-(morpholine-4-carbonyl)tetrazol-5-one;
1-(3-methylphenyl)-4-(morpholine-4-carbonyl)tetrazol-5-one;
1-(4-methylphenyl)-4-(morpholine-4-carbonyl)tetrazol-5-one;
1-(3-methoxyphenyl)-4-(morpholine-4-carbonyl)tetrazol-5-one;
1-(4-methoxyphenyl)-4-(morpholine-4-carbonyl)tetrazol-5-one;
1-(2,3-dichlorophenyl)-4-(morpholine-4-carbonyl)tetrazol-5-one;
1-(1-methylindol-4-yl)-4-(morpholine-4-carbonyl)tetrazol-5-one;
1-(1-methylindol-5-yl)-4-(morpholine-4-carbonyl)tetrazol-5-one;
1-(1-methylindol-6-yl)-4-(morpholine-4-carbonyl)tetrazol-5-one;
1-(3-bromophenyl)-4-(morpholine-4-carbonyl)tetrazol-5-one;
1-(3-chlorophenyl)-4-[(2R,6S)-2,6-dimethylmoipholine-4-carbonyl]tetrazol-5-one;
1-(3-chlorophenyl)-4-(2,6-dimethylmorpholine-4-carbonyl)tetrazol-5-one; and
1-(3-chlorophenyl)-4-(3,3-dimethylpyrrolidine-1-carbonyl)tetrazol-5-one.

12. A compound according to any one of claims 1 to 11 selected from
4-(3-Fluoro-phenyl)-5-oxo-4,5-dihydro-tetrazole-1-carboxylic acid [2-(3,4-dimethoxy-phenyl)-ethyl]-methyl-amide;
4-(3-Fluoro-phenyl)-5-oxo-4,5-dihydro-tetrazole-1-carboxylic acid methylphenethyl-amide;
4-(3-Fluoro-phenyl)-5-oxo-4,5-dihydro-tetrazole-1-carboxylic acid ethyl-pyridin-4-ylmethyl-amide;
4-(3-Fluoro-phenyl)-5-oxo-4,5-dihydro-tetrazole-1-carboxylic acid benzyl-ethylamide;
1-(4,4-Dimethyl-piperidine-1-carbonyl)-4-(3-fluoro-phenyl)-1,4-dihydro-tetrazol-5-one;
N-{(R)-1-[4-(3-Fluoro-phenyl)-5-oxo-4,5-dihydro-tetrazole-1-carbonyl]-pyrrolidin-3-yl}-acetamide;
1-(3-Chloro-phenyl)-4-(4,4-dimethyl-piperidine-1-carbonyl)-1,4-dihydro-tetrazol-5-one;
1-(4-Chloro-phenyl)-4-(4,4-dimethyl-piperidine-1-carbonyl)-1,4-dihydro-tetrazol-5-one;
4-(3-Chloro-phenyl)-5-oxo-4,5-dihydro-tetrazole-1-carboxylic acid (3-methoxypropyl)-methyl-amide; and
1-(3-Chloro-phenyl)-4-(morpholine-4-carbonyl)-1,4-dihydro-tetrazol-5-one.

13. A process for the preparation of a compound according to any one of claims 1 to 12, comprising the reaction of a compound of formula (A) 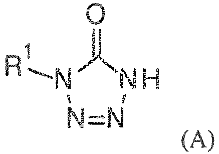
(a) in the presence of R⁵-X and a base; or
(b) in the presence of phosgene, diphosgene or triphosgene followed by reaction in the presence of HNR³R⁴ and a base;
wherein X is an electron withdrawing group and wherein R¹, R³ R⁴ and R⁵ are as defined in any one of claims 1 to 10.

14. A compound according to any one of claims 1 to 12 for use as therapeutically active substance.

15. A pharmaceutical composition comprising a compound in accordance with any one of claims 1 to 12 and a therapeutically inert carrier.

16. A compound according to any one of claims 1 to 12 for use in the treatment or prophylaxis of pain, atherosclerosis, age-related macular degeneration, diabetic retinopathy, glaucoma, retinal vein occlusion, retinopathy of prematurity, ocular ischemic syndrome, geographic atrophy, diabetes mellitus, inflammation, inflammatory bowel disease, ischemia-reperfusion injury, acute liver failure, liver fibrosis, lung fibrosis, kidney fibrosis, systemic fibrosis, acute allograft rejection, chronic allograft nephropathy, diabetic nephropathy, glomerulonephropathy, cardiomyopathy, heart failure, myocardial ischemia, myocardial infarction, systemic sclerosis, thermal injury, burning, hypertrophic scars, keloids, gingivitis pyrexia, liver cirrhosis or tumors, regulation of bone mass, amyotrophic lateral sclerosis, multiple sclerosis, Alzheimer's disease, Parkinson's disease, stroke, transient ischemic attack or uveitis.

## Patentansprüche

1. Eine Verbindung der Formel (I) 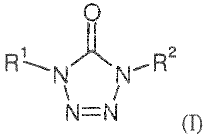 wobei
R¹ n-Propyl, iso-Butyl, Cyclopropyl, 3-Fluorphenyl, 6-Chlorpyridin-3-yl, 3-Chlorphenyl, 4-Chlorphenyl, 3-Trifluormethoxyphenyl, 2-(Trifluormethyl)phenyl, 3-(Trifluormethyl)phenyl, 4-(Trifluormethyl)phenyl, 2,6-Difluorphenyl, 2,5-Difluorphenyl, 3,4-Difluorphenyl, 3,5-Difluorphenyl, 3-Methylphenyl, 4-Methylphenyl, 3-Methoxyphenyl, 4-Methoxyphenyl, 2,3-Dichlorphenyl, 1-Methylindolyl oder 3-Bromphenyl ist;
R² -C(O)NR³R⁴ oder R⁵ ist;
eines von R³ und R⁴ Wasserstoff, Alkyl, Cycloalkyl oder Alkoxyalkyl ist und das andere -(CH₂)ₙ-R⁶ ist;
oder R³ und R⁴ zusammen mit dem Stickstoffatom, an das sie gebunden sind, Heterocyclyl oder substituiertes Heterocyclyl bilden, wobei Heterocyclyl gleich Piperidinyl, Morpholinyl, Pyrrolidinyl, 3,4-Dihydro-1H-isochinolinyl, Azetidinyl, Piperazinyl, 1,1-Dioxothiomorpholinyl oder 2-Oxa-8-aza-spiro[4.5]decyl ist, und wobei substituiertes Heterocyclyl ein Heterocyclyl ist, das mit einem Substituenten, ausgewählt aus Halogen, Alkyl, Cyano, Alkoxyalkyl, Aminocarbonyl, Dialkylaminocarbonyl, Dialkylaminocarbonylalkyl, Alkylaminocarbonylalkyl, Phenyl, Halogenphenyl, Phenylalkyl, Halogenphenylalkyl, Methylpyrazolyl, Methylisoxazolyl, Alkoxyalkyl, Alkylcarbonylamino, Alkylcarbonyl, Alkoxycarbonyl, Alkyl-[1,2,4]oxadiazolyl, Pyrrolidinylcarbonylalkyl, Pyrazinyl, (Alkyl)(alkylcarbonyl)amino, Alkylisoxazolyl und Morpholinylcarbonylalkyl substituiert ist, oder mit zwei Substituenten, unabhängig ausgewählt aus Alkyl, Halogen, Alkoxycarbonyl und Alkoxycarbonylamino, substituiert ist;
R⁵ Halogenphenylalkyl, Alkoxycarbonylpiperidinyl, Alkoxycarbonylalkyl, (Alkylsulfonyl)(alkyl)[1,2,4]triazolylalkyl oder Morpholinylcarbonylalkyl ist;
R⁶ Alkoxy, Dialkoxyphenyl, Cyano, Phenyl, Pyridinyl oder Alkoxycarbonylalkyl ist; und
n 0, 1, 2 oder 3 ist;
oder ein pharmazeutisch verträgliches Salz davon;
mit der Maßgabe, dass
1-Cyclopropyl-4-(pyrrolidin-1-carbonyl)-1,4-dihydro-tetrazol-5-on;
1-Cyclopropyl-4-(2-methyl-pyrrolidin-1-carbonyl)-1,4-dihydro-tetrazol-5-on;
1-Cyclopropyl-4-(2,5-dimethyl-pyrrolidin-1-carbonyl)-1,4-dihydro-tetrazol-5-on;
1-Cyclopropyl-4-(2,6-dimethyl-piperidin-1-carbonyl)-1,4-dihydro-tetrazol-5-on;
4-Cyclopropyl-5-oxo-4,5-dihydro-tetrazol-1-carbonsäure-benzyl-isopropyl-amid;
[4-(4-Chlor-phenyl)-5-oxo-4,5-dihydro-tetrazol-1-yl]-essigsäureethylester;
2-[4-(4-Chlor-phenyl)-5-oxo-4,5-dihydro-tetrazol-1-yl]-propionsäureethylester;
3-[4-(3-Chlor-phenyl)-5-oxo-4,5-dihydro-tetrazol-1-yl]-propionsäuremethylester;
1-(2,3-Dichlor-phenyl)-4-(2-methyl-piperidin-1-carbonyl)-1,4-dihydro-tetrazol-5-on;
1-(2,6-Difluorphenyl)-4-(3,4-dihydro-1H-isochinolin-2-carbonyl)tetrazol-5-on;
4-(2,6-Difluorphenyl)-5-oxo-N-phenyl-N-propyl-tetrazol-1-carboxamid;
4-(2,6-Difluorphenyl)-N-ethyl-5-oxo-N-phenyl-tetrazol-1-carboxamid;
4-(2,6-Difluorphenyl)-N-methyl-5-oxo-N-phenyl-tetrazol-1-carboxamid;
4-(2,6-Difluorphenyl)-N-isopropyl-5-oxo-N-phenyl-tetrazol-1-carboxamid;
N-Cyclohexyl-4-(2,6-difluorphenyl)-5-oxo-N-phenyl-tetrazol-1-carboxamid;
4-(2,6-Difluorphenyl)-N-methyl-5-oxo-N-(2-pyridyl)tetrazol-1-carboxamid;
N,4-Dicyclopropyl-5-oxo-N-phenyl-tetrazol-1-carboxamid;
N-Butyl-4-cyclopropyl-5-oxo-N-phenyl-tetrazol-1-carboxamid;
4-Cyclopropyl-N-isobutyl-5-oxo-N-phenyl-tetrazol-1-carboxamid;
4-Cyclopropyl-N-isopropyl-5-oxo-N-phenyl-tetrazol-1-carboxamid;
4-Cyclopropyl-5-oxo-N-phenyl-N-propyl-tetrazol-1-carboxamid;
4-Cyclopropyl-N-methyl-5-oxo-N-phenyl-tetrazol-1-carboxamid;
4-Cyclopropyl-N-ethyl-5-oxo-N-phenyl-tetrazol-1-carboxamid;
4-Cyclopropyl-5-oxo-N-phenyl-N-sec-butyl-tetrazol-1-carboxamid;
N-(1-Benzyl-2-methyl-propyl)-4-cyclopropyl-5-oxo-N-phenyl-tetrazol-1-carboxamid;
1-(2-Methylpiperidin-1-carbonyl)-4-(p-tolyl)tetrazol-5-on;
1-(2-Methylpiperidin-1-carbonyl)-4-(m-tolyl)tetrazol-5-on;
N-Isopropyl-5-oxo-N-phenyl-4-propyl-tetrazol-1-carboxamid;
1-(4-Chlorphenyl)-4-[(2-fluorphenyl)methyl]tetrazol-5-on;
1-(4-Chlorphenyl)-4-[(3-fluorphenyl)methyl]tetrazol-5-on;
1-(4-Chlorphenyl)-4-[(3-chlorphenyl)methyl]tetrazol-5-on;
1-[(4-Bromphenyl)methyl]-4-(4-chlorphenyl)tetrazol-5-on; und
3-[5-oxo-4-(p-tolyl)tetrazol-1-yl]propansäuremethylester;
ausgeschlossen sind;
wobei
"Alkyl", alleinstehend oder in Kombination, für eine geradkettige oder verzeigtkettige Alkylgruppe mit 1 bis 8 Kohlenstoffatomen steht;
"Cycloalkyl", alleinstehend oder in Kombination, für einen Cycloalkylring mit 3 bis 8 Kohlenstoffatomen steht; und
"Alkoxy", alleinstehend oder in Kombination, für eine Gruppe der Formel Alkyl-O-steht, in der der Ausdruck "Alkyl" die vorstehende Bedeutung aufweist.

2. Eine Verbindung gemäß Anspruch 1, wobei R¹ 3-Fluorphenyl, 3-Chlorphenyl oder 4-Chlorphenyl ist.

3. Eine Verbindung gemäß Anspruch 1 oder 2, wobei eines von R³ und R⁴ Alkyl ist und das andere -(CH₂)ₙ-R⁶ ist.

4. Eine Verbindung gemäß einem der Ansprüche 1 bis 3, wobei eines von R³ und R⁴ Methyl oder Ethyl ist und das andere -(CH₂)ₙ-R⁶ ist.

5. Eine Verbindung gemäß einem der Ansprüche 1 und 2, wobei R³ und R⁴ zusammen mit dem Stickstoffatom, an das sie gebunden sind, Heterocyclyl oder substituiertes Heterocyclyl bilden, wobei Heterocyclyl Piperidinyl, Pyrrolidinyl oder Morpholinyl ist, und wobei substituiertes Heterocyclyl ein Heterocyclyl ist, das substituiert ist mit einem Substituenten, ausgewählt aus Alkyl und Alkylcarbonylamino, oder mit zwei Substituenten, unabhängig ausgewählt aus Alkyl.

6. Eine Verbindung gemäß einem der Ansprüche 1, 2 und 5, wobei R³ und R⁴ zusammen mit dem Stickstoffatom, an das sie gebunden sind, Morpholinyl, Dimethylpiperidinyl oder Methylcarbonylaminopyrrolidinyl bilden.

7. Eine Verbindung gemäß einem der Ansprüche 1 und 2, wobei R⁵ tert.-Butyloxycarbonylpiperidinyl, Fluorphenylmethyl, Ethoxycarbonylmethyl, Ethoxycarbonyl(dimethyl)methyl, (Methylsulfonyl)(methyl)[1,2,4]triazolyl oder Morpholinylcarbonylmethyl ist.

8. Eine Verbindung gemäß einem der Ansprüche 1 bis 4, wobei R⁶ Alkoxy, Dialkoxyphenyl, Phenyl oder Pyridinyl ist.

9. Eine Verbindung gemäß einem der Ansprüche 1 bis 4 und 8, wobei R⁶ Methoxy, Dimethoxyphenyl, Phenyl oder Pyridinyl ist.

10. Eine Verbindung gemäß einem der Ansprüche 1 bis 4, 8 und 9, wobei n gleich 1, 2 oder 3 ist.

11. Eine Verbindung gemäß einem der Ansprüche 1 bis 10, ausgewählt aus:
4-(3-Fluor-phenyl)-5-oxo-4,5-dihydro-tetrazol-1-carbonsäure-[2-(3,4-dimethoxyphenyl)-ethyl]-methyl-amid;
4-(3-Fluor-phenyl)-5-oxo-4,5-dihydro-tetrazol-1-carbonsäure-(2-cyano-ethyl)-methyl-amid;
1-[4-(3-Fluor-phenyl)-5-oxo-4,5-dihydro-tetrazol-1-carbonyl]-piperidin-3-carbonsäureamid;
1-(3-Fluor-phenyl)-4-(morpholin-4-carbonyl)-1,4-dihydro-tetrazol-5-on;
4-(3-Fluor-phenyl)-5-oxo-4,5-dihydro-tetrazol-1-carbonsäure-methyl-phenethyl-amid;
4-(3-Fluor-phenyl)-5-oxo-4,5-dihydro-tetrazol-1-carbonsäure-ethyl-pyridin-4-ylmethyl-amid;
4-(3-Fluor-phenyl)-5-oxo-4,5-dihydro-tetrazol-1-carbonsäure-ethyl-(2-methoxy-ethyl)-amid;
4-(3-Fluor-phenyl)-5-oxo-4,5-dihydro-tetrazol-1-carbonsäure-methyl-(3-phenyl-propyl)-amid;
1-(2-Benzyl-pyrrolidin-1-carbonyl)-4-(3-fluor-phenyl)-1,4-dihydro-tetrazol-5-on;
1-(3-Fluor-phenyl)-4-[2-(4-fluor-phenyl)-pynolidin-1-carbonyl]-1,4-dihydro-tetrazol-5-on;
1-(6-Fluor-3,4-dihydro-1H-isochinolin-2-carbonyl)-4-(3-fluor-phenyl)-1,4-dihydro-tetrazol-5-on;
4-(3-Fluor-phenyl)-5-oxo-4,5-dihydro-tetrazol-1-carbonsäure-benzyl-ethyl-amid;
(2S,3S)-2-{[4-(3-Fluor-phenyl)-5-oxo-4,5-dihydro-tetrazol-1-carbonyl]-methyl-amino}-3-methyl-pentansäuremethylester;
1-(4-Fluor-benzyl)-4-(3-fluor-phenyl)-1,4-dihydro-tetrazol-5-on;
4-[4-(3-Fluor-phenyl)-5-oxo-4,5-dihydro-tetrazol-1-yl]-piperidin-1-carbonsäure-tert-butylester;
2-[4-(3-Fluor-phenyl)-5-oxo-4,5-dihydro-tetrazol-1-yl]-2-methyl-propionsäureethylester;
1-(3-Fluor-phenyl)-4-(5-methansulfonyl-4-methyl-4H-[1,2,4]triazol-3-ylmethyl)-1,4-dihydro-tetrazol-5-on;
1-(3-Fluor-phenyl)-4-[2-(2-methyl-2H-pyrazol-3-yl)-pyrrolidin-1-carbonyl]-1,4-dihydro-tetrazol-5-on;
1-(3-Fluor-phenyl)-4-[2-(1-methyl-1H-pyrazol-3-yl)-pyrrolidin-1-carbonyl]-1,4-dihydro-tetrazol-5-on;
1-(3-Fluor-phenyl)-4-[2-(3-methyl-isoxazol-5-yl)-pyrrolidin-1-carbonyl]-1,4-dihydro-tetrazol-5-on;
1-(3-Fluor-phenyl)-4-((S)-2-methoxymethyl-pyrrolidin-1-carbonyl)-1,4-dihydro-tetrazol-5-on;
(S)-1-[4-(3-Fluor-phenyl)-5-oxo-4,5-dihydro-tetrazol-1-carbonyl]-pyrrolidin-2-carbonsäure-amid;
N-{1-[4-(3-Fluor-phenyl)-5-oxo-4,5-dihydro-tetrazol-1-carbonyl]-pyrrolidin-3-yl}-acetamid;
1-[4-(3-Fluor-phenyl)-5-oxo-4,5-dihydro-tetrazol-1-carbonyl]-azetidin-3-carbonsäuremethylester;
1-(3-Fluor-phenyl)-4-[2-(3-methyl-[1,2,4]oxadiazol-5-yl)-pyrrolidin-1-carbonyl]-1,4-dihydro-tetrazol-5-on;
N-{1-[4-(3-Fluor-phenyl)-5-oxo-4,5-dihydro-tetrazol-1-carbonyl]-piperidin-4-yl}-acetamid;
1-(3-Fluor-phenyl)-4-[4-(2-oxo-2-pyrrolidin-1-yl-ethyl)-piperazin-1-carbonyl]-1,4-dihydro-tetrazol-5-on;
1-(4,4-Dimethyl-piperidin-1-carbonyl)-4-(3-fluor-phenyl)-1,4-dihydro-tetrazol-5-on;
1-(1,1-Dioxo-1λ6-thiomorpholin-4-carbonyl)-4-(3-fluor-phenyl)-1,4-dihydro-tetrazol-5-on;
1-(3-Fluor-phenyl)-4-(2,3,5,6-tetrahydro-[1,2']bipyrazinyl-4-carbonyl)-1,4-dihydro-tetrazol-5-on;
4-(3-Fluor-phenyl)-5-oxo-4,5-dihydro-tetrazol-1-carbonsäure-(2-cyano-ethyl)-cyclopropyl-amid;
4-(3-Fluor-phenyl)-5-oxo-4,5-dihydro-tetrazol-1-carbonsäure-methyl-pyridin-2-yl-amid;
4-(3-Fluor-phenyl)-5-oxo-4,5-dihydro-tetrazol-1-carbonsäure-methyl-pyridin-3-yl-amid;
1-(3-Fluor-phenyl)-4-(2-methoxymethyl-piperidin-1-carbonyl)-1,4-dihydro-tetrazol-5-on;
4-Cyclopropyl-5-oxo-4,5-dihydro-tetrazol-1-carbonsäure-methyl-pyridin-3-yl-amid;
5-Oxo-4-propyl-4,5-dihydro-tetrazol-1-carbonsäure-methyl-pyridin-3-yl-amid;
4-Isobutyl-5-oxo-4,5-dihydro-tetrazol-1-carbonsäure-methyl-pyridin-3-yl-amid;
1-(4-Acetyl-piperazin-1-carbonyl)-4-(3-fluor-phenyl)-1,4-dihydro-tetrazol-5-on;
1-(3-Fluor-phenyl)-4-(4-propionyl-piperazin-1-carbonyl)-1,4-dihydro-tetrazol-5-on;
4-[4-(3-Fluor-phenyl)-5-oxo-4,5-dihydro-tetrazol-1-carbonyl]-piperazin-1-carbonsäurediethylamid;
1-(3-Fluor-phenyl)-4-(3-phenyl-pyrrolidin-1-carbonyl)-1,4-dihydro-tetrazol-5-on;
N-{(S)-1-[4-(3-Fluor-phenyl)-5-oxo-4,5-dihydro-tetrazol-1-carbonyl]-pyrrolidin-3-yl}-acetamid;
N-{(R)-1-[4-(3-Fluor-phenyl)-5-oxo-4,5-dihydro-tetrazol-1-carbonyl]-pyrrolidin-3-yl}-acetamid;
N-Ethyl-N-{1-[4-(3-fluor-phenyl)-5-oxo-4,5-dihydro-tetrazol-1-carbonyl]-pyrrolidin-3-yl}-acetamid;
1-(3,3-Difluor-pyrrolidin-1-carbonyl)-4-(3-fluor-phenyl)-1,4-dihydro-tetrazol-5-on;
1-(2,2-Dimethyl-pyrrolidin-1-carbonyl)-4-(3-fluor-phenyl)-1,4-dihydro-tetrazol-5-on;
1-(3,3-Dimethyl-pyrrolidin-1-carbonyl)-4-(3-fluor-phenyl)-1,4-dihydro-tetrazol-5-on;
1-(3-Fluor-phenyl)-4-(2-methyl-pyrrolidin-1-carbonyl)-1,4-dihydro-tetrazol-5-on;
N-{1-[4-(3-Fluor-phenyl)-5-oxo-4,5-dihydro-tetrazol-1-carbonyl]-pyrrolidin-3-yl)-N-methyl-acetamid;
4-[4-(3-Fluor-phenyl)-5-oxo-4,5-dihydro-tetrazol-1-carbonyl]-piperazin-1-carbonsäureethylester;
(S)-1-[4-(3-Fluor-phenyl)-5-oxo-4,5-dihydro-tetrazol-1-carbonyl]-pyrrolidin-2-carbonitril;
(R)-1-[4-(3-Fluor-phenyl)-5-oxo-4,5-dihydro-tetrazol-1-carbonyl]-pyrrolidin-2-carbonitril;
4-(6-Chlor-pyridin-3-yl)-5-oxo-4,5-dihydro-tetrazol-1-carbonsäure-benzyl-ethyl-amid;
4-(3-Fluor-phenyl)-5-oxo-4,5-dihydro-tetrazol-1-carbonsäure-(2-methoxy-ethyl)-methyl-amid;
4-(3-Fluor-phenyl)-5-oxo-4,5-dihydro-tetrazol-1-carbonsäure-isopropyl-(2-methoxyethyl)-amid;
4-(3-Fluor-phenyl)-5-oxo-4,5-dihydro-tetrazol-1-carbonsäure-(2-methoxy-ethyl)-propyl-amid;
4-(3-Fluor-phenyl)-5-oxo-4,5-dihydro-tetrazol-1-carbonsäure-bis-(2-methoxy-ethyl)-amid;
1-(2,6-Dimethyl-morpholin-4-carbonyl)-4-(3-fluor-phenyl)-1,4-dihydro-tetrazol-5-on;
1-((2S,6R)-2,6-Dimethyl-morpholin-4-carbonyl)-4-(3-fluor-phenyl)-1,4-dihydro-tetrazol-5-on;
1-(3-Fluor-phenyl)-4-(4-methoxymethyl-piperidin-1-carbonyl)-1,4-dihydro-tetrazol-5-on;
1-[4-(3-Fluor-phenyl)-5-oxo-4,5-dihydro-tetrazol-1-carbonyl]-piperidin-4-carbonitril;
4-tert-Butoxycarbonylamino-1-[4-(3-fluor-phenyl)-5-oxo-4,5-dihydro-tetrazol-1-carbonyl]-piperidin-4-carbonsäuremethylester;
1-(3,3-Dimethyl-piperidin-1-carbonyl)-4-(3-fluor-phenyl)-1,4-dihydro-tetrazol-5-on;
1-(3-Fluor-phenyl)-4-(2-oxa-8-aza-spiro[4.5]decan-8-carbonyl)-1,4-dihydro-tetrazol-5-on;
N-{(R)-1-[4-(3-Chlor-phenyl)-5-oxo-4,5-dihydro-tetrazol-1-carbonyl]-pyrrolidin-3-yl}-acetamid;
N-{(R)-1-[5-Oxo-4-(3-trifluormethoxy-phenyl)-4,5-dihydro-tetrazol-1-carbonyl]-pyrrolidin-3-yl -acetamid;
N-{(R)-1-[4-(4-Chlor-phenyl)-5-oxo-4,5-dihydro-tetrazol-1-carbonyl]-pyrrolidin-3-yl}-acetamid;
N-{(S)-1-[4-(3-Chlor-phenyl)-5-oxo-4,5-dihydro-tetrazol-1-carbonyl]-pyrrolidin-3-yl}-acetamid;
N-{(S)-1-[5-Oxo-4-(3-trifluormethoxy-phenyl)-4,5-dihydro-tetrazol-1-carbonyl]-pyrrolidin-3-yl}-acetamid;
N-{(S)-1-[4-(4-Chlor-phenyl)-5-oxo-4,5-dihydro-tetrazol-1-carbonyl]-pyrrolidin-3-yl}-acetamid;
N-{1-[4-(3-Chlor-phenyl)-5-oxo-4,5-dihydro-tetrazol-1-carbonyl]-pyrrolidin-3-yl}-N-ethyl-acetamid;
N-Ethyl-N-{1-[5-oxo-4-(3-trifluormethoxy-phenyl)-4,5-dihydro-tetrazol-1-carbonyl]-pyrrolidin-3-yl}-acetamid;
N-{1-[4-(4-Chlor-phenyl)-5-oxo-4,5-dihydro-tetrazol-1-carbonyl]-pyrrolidin-3-yl}-N-ethyl-acetamid;
1-(3-Chlor-phenyl)-4-[2-(3-methyl-isoxazol-5-yl)-pyrrolidin-1-carbonyl]-1,4-dihydro-tetrazol-5-on;
1-[2-(3-Methyl-isoxazol-5-yl)-pyrrolidin-1-carbonyl]-4-(3-trifluormethoxy-phenyl)-1,4-dihydro-tetrazol-5-on;
1-(4-Chlor-phenyl)-4-[2-(3-methyl-isoxazol-5-yl)-pyrrolidin-1-carbonyl]-1,4-dihydro-tetrazol-5-on;
1-(3-Chlor-phenyl)-4-(4,4-dimethyl-piperidin-1-carbonyl)-1,4-dihydro-tetrazol-5-on;
1-(4,4-Dimethyl-piperidin-1-carbonyl)-4-(3-trifluormethoxy-phenyl)-1,4-dihydro-tetrazol-5-on;
1-(4-Chlor-phenyl)-4-(4,4-dimethyl-piperidin-1-carbonyl)-1,4-dihydro-tetrazol-5-on;
4-(3-Chlor-phenyl)-5-oxo-4,5-dihydro-tetrazol-1-carbonsäure-ethyl-(2-methoxy-ethyl)-amid;
5-Oxo-4-(3-trifluormethoxy-phenyl)-4,5-dihydro-tetrazol-1-carbonsäure-ethyl-(2-methoxy-ethyl)-amid;
4-(4-Chlor-phenyl)-5-oxo-4,5-dihydro-tetrazol-1-carbonsäure-ethyl-(2-methoxy-ethyl)-amid;
4-(3-Chlor-phenyl)-5-oxo-4,5-dihydro-tetrazol-1-carbonsäure-isopropyl-(2-methoxyethyl)-amid;
5-Oxo-4-(3-trifluormethoxy-phenyl)-4,5-dihydro-tetrazol-1-carbonsäure-isopropyl-(2-methoxy-ethyl)-amid;
4-(4-Chlor-phenyl)-5-oxo-4,5-dihydro-tetrazol-1-carbonsäure-isopropyl-(2-methoxyethyl)-amid;
4-(3-Chlor-phenyl)-5-oxo-4,5-dihydro-tetrazol-1-carbonsäure-(3-methoxy-propyl)-methyl-amid;
5-Oxo-4-(3-trifluormethoxy-phenyl)-4,5-dihydro-tetrazol-1-carbonsäure-(3-methoxypropyl)-methyl-amid;
4-(4-Chlor-phenyl)-5-oxo-4,5-dihydro-tetrazol-1-carbonsäure-(3-methoxy-propyl)-methyl-amid;
1-(3-Chlor-phenyl)-4-[4-(2-morpholin-4-yl-2-oxo-ethyl)-piperazin-1-carbonyl]-1,4-dihydro-tetrazol-5-on;
1-[4-(2-Morpholin-4-yl-2-oxo-ethyl)-piperazin-1-carbonyl]-4-(3-trifluormethoxyphenyl)-1,4-dihydro-tetrazol-5-on;
1-(4-Chlor-phenyl)-4-[4-(2-morpholin-4-yl-2-oxo-ethyl)-piperazin-1-carbonyl]-1,4-dihydro-tetrazol-5-on;
2-{4-[4-(3-Chlor-phenyl)-5-oxo-4,5-dihydro-tetrazol-1-carbonyl]-piperazin-1-yl}-N,N-dimethylacetamid;
N,N-Dimethyl-2-{4-[5-oxo-4-(3-trifluormethoxy-phenyl)-4,5-dihydro-tetrazol-1-carbonyl]-piperazin-1-yl}-acetamid;
2-{4-[4-(4-Chlor-phenyl)-5-oxo-4,5-dihydro-tetrazol-1-carbonyl]-piperazin-1-yl}-N,N-dimethylacetamid;
1-(3-Chlor-phenyl)-4-[4-(2-oxo-2-pyrrolidin-1-yl-ethyl)-piperazin-1-carbonl]-1,4-dihydro-tetrazol-5-on;
1-[4-(2-Oxo-2-pyrrolidin-1-yl-ethyl)-piperazin-1-carbonyl]-4-(3-trifluormethoxyphenyl)-1,4-dihydro-tetrazol-5-on;
1-(4-Chlor-phenyl)-4-[4-(2-oxo-2-pyrrolidin-1-yl-ethyl)-piperazin-1-carbonyl]-1,4-dihydro-tetrazol-5-on;
4-(3-Chlor-phenyl)-5-oxo-4,5-dihydro-tetrazol-1-carbonsäure-(2-ethoxy-ethyl)-ethyl-amid;
5-Oxo-4-(3-trifluormethoxy-phenyl)-4,5-dihydro-tetrazol-1-carbonsäure-(2-ethoxyethyl)-ethyl-amid;
4-(4-Chlor-phenyl)-5-oxo-4,5-dihydro-tetrazol-1-carbonsäure-(2-ethoxy-ethyl)-ethyl-amid;
1-(3-Chlor-phenyl)-4-(morpholin-4-carbonyl)-1,4-dihydro-tetrazol-5-on;
1-(Morpholin-4-carbonyl)-4-(3-trifluormethoxy-phenyl)-1,4-dihydro-tetrazol-5-on;
1-(4-Chlor-phenyl)-4-(morpholin-4-carbonyl)-1,4-dihydro-tetrazol-5-on;
2-{4-[4-(3-Chlor-phenyl)-5-oxo-4,5-dihydro-tetrazol-1-carbonyl]-piperazin-1-yl}-N-ethyl-acetamid;
N-Ethyl-2-{4-[5-oxo-4-(3-trifluormethoxy-phenyl)-4,5-dihydro-tetrazol-1-carbonyl]-piperazin-1-yl}-acetamid;
2-{4-[4-(4-Chlor-phenyl)-5-oxo-4,5-dihydro-tetrazol-1-carbonyl]-piperazin-1-yl}-N-ethyl-acetamid;
N-Butyl-2-{4-[4-(3-chlor-phenyl)-5-oxo-4,5-dihydro-tetrazol-1-carbonyl]-piperazin-1-yl}-acetamid;
N-Butyl-2-{4-[5-oxo-4-(3-trifluormethoxy-phenyl)-4,5-dihydro-tetrazol-1-carbonyl]-piperazin-1-yl}-acetamid;
N-Butyl-2-{4-[4-(4-chlor-phenyl)-5-oxo-4,5-dihydro-tetrazol-1-carbonyl]-piperazin-1-yl}-acetamid;
4-(3-Fluor-phenyl)-5-oxo-4,5-dihydro-tetrazol-1-carbonsäure-(2-methoxy-ethyl)-amid;
4-(3-Chlor-phenyl)-5-oxo-4,5-dihydro-tetrazol-1-carbonsäure-(2-ethoxy-ethyl)-methyl-amid;
5-Oxo-4-(3-trifluormethoxy-phenyl)-4,5-dihydro-tetrazol-1-carbonsäure-(2-ethoxy-ethyl)-methyl-amid;
4-(4-Chlor-phenyl)-5-oxo-4,5-dihydro-tetrazol-1-carbonsäure-(2-ethoxy-ethyl)-methyl-amid;
[4-(3-Chlor-phenyl)-5-oxo-4,5-dihydro-tetrazol-1-yl]-essigsäureethylester;
1-(3-Chlor-phenyl)-4-(2-morpholin-4-yl-2-oxo-ethyl)-1,4-dihydro-tetrazol-5-on;
1-(3-Chlor-phenyl)-4-[4-(1-methyl-2-oxo-2-pyrrolidin-1-yl-ethyl)-piperazin-1-carbonyl]-1,4-dihydro-tetrazol-5-on;
1-[4-(1-Methyl-2-oxo-2-pyrrolidin-1-yl-ethyl)-piperazin-1-carbonyl]-4-(3-trifluormethoxy-phenyl)-1,4-dihydro-tetrazol-5-on;
1-(4-Chlor-phenyl)-4-[4-(1-methyl-2-oxo-2-pyrrolidin-1-yl-ethyl)-piperazin-1-carbonyl]-1,4-dihydro-tetrazol-5-on;
1-(Morpholin-4-carbonyl)-4-[2-(trifluormethyl)phenyl]tetrazol-5-on;
1-(Morpholin-4-carbonyl)-4-[3-(trifluormethyl)phenyl]tetrazol-5-on;
1-(Morpholin-4-carbonyl)-4-[4-(trifluormethyl)phenyl]tetrazol-5-on;
1-(2,6-Difluorphenyl)-4-(morpholin-4-carbonyl)tetrazol-5-on;
1-(2,4-Difluorphenyl)-4-(morpholin-4-carbonyl)tetrazol-5-on;
1-(2,5-Difluorphenyl)-4-(morpholin-4-carbonyl)tetrazol-5-on;
1-(3,4-Difluorphenyl)-4-(morpholin-4-carbonyl)tetrazol-5-on;
1-(3,5-Difluorphenyl)-4-(morpholin-4-carbonyl)tetrazol-5-on;
1-(3-Methylphenyl)-4-(morpholin-4-carbonyl)tetrazol-5-on;
1-(4-Methylphenyl)-4-(morpholin-4-carbonyl)tetrazol-5-on;
1-(3-Methoxyphenyl)-4-(morpholin-4-carbonyl)tetrazol-5-on;
1-(4-Methoxyphenyl)-4-(morpholin-4-carbonyl)tetrazol-5-on;
1-(2,3-Dichlorphenyl)-4-(morpholin-4-carbonyl)tetrazol-5-on;
1-(1-Methylindol-4-yl)-4-(morpholin-4-carbonyl)tetrazol-5-on;
1-(1-Methylindol-5-yl)-4-(morpholin-4-carbonyl)tetrazol-5-on;
1-(1-Methylindol-6-yl)-4-(morpholin-4-carbonyl)tetrazol-5-on;
1-(3-Bromphenyl)-4-(morpholin-4-carbonyl)tetrazol-5-on;
1-(3-Chlorphenyl)-4-[(2R,6S)-2,6-dimethylmorpholin-4-carbonyl]tetrazol-5-on;
1-(3-Chlorphenyl)-4-(2,6-dimethylmorpholin-4-carbonyl)tetrazol-5-on; und
1-(3-Chlorphenyl)-4-(3,3-dimethylpyrrolidin-1-carbonyl)tetrazol-5-on.

12. Eine Verbindung gemäß einem der Ansprüche 1 bis 11, ausgewählt aus:
4-(3-Fluor-phenyl)-5-oxo-4,5-dihydro-tetrazol-1-carbonsäure-[2-(3,4-dimethoxy-phenyl)-ethyl]-methyl-amid;
4-(3-Fluor-phenyl)-5-oxo-4,5-dihydro-tetrazol-1-carbonsäure-methyl-phenethyl-amid;
4-(3-Fluor-phenyl)-5-oxo-4,5-dihydro-tetrazol-1-carbonsäure-ethyl-pyridin-4-ylmethyl-amid;
4-(3-Fluor-phenyl)-5-oxo-4,5-dihydro-tetrazol-1-carbonsäure-benzyl-ethyl-amid;
1-(4,4-Dimethyl-piperidin-1-carbonyl)-4-(3-fluor-phenyl)-1,4-dihydro-tetrazol-5-on;
N-{(R)-1-[4-(3-Fluor-phenyl)-5-oxo-4,5-dihydro-tetrazol-1-carbonyl]-pyrrolidin-3-yl}-acetamid;
1-(3-Chlor-phenyl)-4-(4,4-dimethyl-piperidin-1-carbonyl)-1,4-dihydro-tetrazol-5-on;
1-(4-Chlor-phenyl)-4-(4,4-dimethyl-piperidin-1-carbonyl)-1,4-dihydro-tetrazol-5-on;
4-(3-Chlor-phenyl)-5-oxo-4,5-dihydro-tetrazol-1-carbonsäure-(3-methoxy-propyl)-methyl-amid; und
1-(3-Chlor-phenyl)-4-(morpholin-4-carbonyl)-1,4-dihydro-tetrazol-5-on.

13. Ein Verfahren zur Herstellung einer Verbindung gemäß einem der Ansprüche 1 bis 12, umfassend das Umsetzen einer Verbindung der Formel (A) 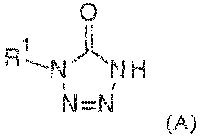
(a) in der Gegenwart von R⁵-X und einer Base; oder
(b) in der Gegenwart von Phosgen, Diphosgen oder Triphosgen, gefolgt von der Umsetzung in der Gegenwart von HNR³R⁴ und einer Base;
wobei X eine elektronenziehende Gruppe ist und wobei R¹, R³, R⁴ und R⁵ wie in einem der Ansprüche 1 bis 10 definiert sind.

14. Eine Verbindung gemäß einem der Ansprüche 1 bis 12, zur Verwendung als therapeutischer Wirkstoff.

15. Ein Arzneimittel, umfassend eine Verbindung gemäß einem der Ansprüche 1 bis 12 und einen therapeutisch inerten Träger.

16. Eine Verbindung gemäß einem der Ansprüche 1 bis 12 zur Verwendung bei der Behandlung oder Prophylaxe von Schmerz, Arteriosklerose, altersbedingter Makuladegeneration, diabetischer Retinopathie, Glaukom, Netzhautvenenverschluss, Frühgeborenenretinopathie, okularem Ischämie-Syndrom, geographischer Atrophie, Diabetes mellitus, Entzündung, entzündlicher Darmerkrankung, Ischämie-Reperfusionsverletzung, akutem Leberversagen, Leberfibrose, Lungenfibrose, Nierenfibrose, systemischer Fibrose, akuter Abstoßungsreaktion, chronischer Allograft-Nephropathie, diabetischer Nephropathie, Glomerulonephropathie, Kardiomyopathie, Herzinsuffizienz, Myokardischämie, Myokardinfarkt, systemischer Sklerose, thermischer Schädigung, Verbrennung, hypertrophen Narben, Keloiden, Gingivitis Pyrexie, Leberzirrhose oder Tumoren, Regulierung der Knochenmasse, amyotropher Lateralsklerose, multipler Sklerose, Alzheimer-Krankheit, Parkinson-Krankheit, Schlaganfall, transitorischem ischämischem Schock oder Uveitis.

## Revendications

1. Composé de formule (I) 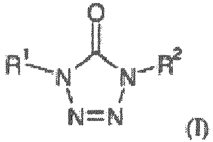 dans laquelle :
R¹ est un groupe n-propyle, isobutyle, cyclopropyle, 3-fluorophényle, 6-chloropyridine-3-yle, 3-chlorophényle, 4-chlorophényle, 3-trifluorométhoxyphényle, 2-(trifluoro-méthyl)phényle, 3-(trifluorométhyl)phényle, 4-(trifluoro-méthyl)phényle, 2,6-difluorophényle, 2,5-difluorophényle, 3,4-difluorophényle, 3,5-difluorophényle, 3-méthylphényle, 4-méthylphényle, 3-méthoxyphényle, 4-méthoxyphényle, 2,3-dichlorophényle, 1-méthylindolyle ou 3-bromophényle ;
R² est un groupe -C(O)NR³R⁴ ou R⁵ ;
un de R³ et R⁴ est un atome d'hydrogène, un groupe alkyle, cycloalkyle ou alkoxyalkyle et l'autre est un groupe -(CH₂)ₙ-R⁶ ;
ou R³ et R⁴ conjointement avec l'atome d'azote auquel ils sont attachés, forment un groupe hétérocyclyle ou hétérocyclyle substitué, où le groupe hétérocyclyle est un groupe pipéridinyle, morpholinyle, pyrrolidinyle, 3,4-dihydro-1H-isoquinolinyle, azétidinyle, pipérazinyle, 1,1-dioxothio-morpholinyle, ou 2-oxa-8-aza-spiro[4.5]décyle, et où le groupe hétérocyclyle substitué est un groupe hétérocyclyle substitué avec un substituant choisi parmi un atome d'halogène, un groupe alkyle, cyano, alkoxyalkyle, amino-carbonyle, dialkylaminocarbonyle, dialkylaminocarbonyl-alkyle, alkylaminocarbonylalkyle, phényle, halogénophényle, phénylalkyle, halogénophénylalkyle, méthylpyrazolyle, méthylisoxazolyle, alkoxyalkyle, alkylcarbonylamino, alkyl-carbonyle, alkoxycarbonyle, alkyl-[1,2,4]oxadiazolyle, pyrrolidinylcarbonylalkyle, pyrazinyle, (alkyl) (alkylcarbonyl)-amino, alkylisoxazolyle et morpholinylcarbonylalkyle, ou avec deux substituants choisis indépendamment parmi des groupes alkyle, halogéno, alkoxycarbonyle et alkoxy-carbonylamino ;
R⁵ est un groupe halogénophénylalkyle, alkoxycarbonyl-pipéridinyle, alkoxycarbonylalkyle, (alkylsulfonyl) (alkyl)-[1,2,4]triazolylalkyle ou morpholinylcarbonylalkyle ;
R⁶ est un groupe alkoxy, dialkoxyphényle, cyano, phényle, pyridinyle ou alkoxycarbonylalkyle ; et
n vaut 0, 1, 2 ou 3 ;
ou un de ses sels pharmaceutiquement acceptables ;
pourvu que :
la 1-cyclopropyl-4-(pyrrolidine-1-carbonyl)-1,4-dihydro-tétrazole-5-one ;
la 1-cyclopropyl-4-(2-méthyl-pyrrolidine-1-carbonyl)-1,4-dihydro-tétrazole-5-one ;
la 1-cyclopropyl-4-(2,5-diméthyl-pyrrolidine-1-carbonyl)-1,4-dihydro-tétrazole-5-one ;
la 1-cyclopropyl-4-(2,6-diméthyl-pipéridine-1-carbonyl)-1,4-dihydro-tétrazole-5-one ;
le benzyl-isopropyl-amide d'acide 4-cyclopropyl-5-oxo-4,5-dihydro-tétrazole-1-carboxylique ;
l'ester éthylique d'acide [4-(4-chlorophényl)-5-oxo-4,5-dihydro-tétrazole-1-yl]-acétique ;
l'ester éthylique d'acide 2-[4-(4-chlorophényl)-5-oxo-4,5-dihydro-tétrazole-1-yl]-propionique ;
l'ester méthylique d'acide 3-[4-(3-chlorophényl)-5-oxo-4,5-dihydro-tétrazole-1-yl]-propionique ;
la 1-(2,3-dichlorophényl)-4--(2-méthyl-pipéridine-1-carbonyl)-1,4-dihydro-tétrazole-5-one ;
la 1-(2,6-difluorophényl)-4-(3,4-dihydro-1H-iso-quinoléine-2-carbonyl)tétrazole-5-one ;
le 4-(2,6-difluorophényl)-5-oxo-N-phényl-N-propyl-tétrazole-1-carboxamide ;
le 4-(2,6-difluorophényl)-N-éthyl-5-oxo-N-phényl-tétrazole-1-carboxamide ;
le 4-(2,6-difluorophényl)-N-méthyl-5-oxo-N-phényl-tétrazole-1-carboxamide ;
le 4-(2,6-difluorophényl)-N-isopropyl-5-oxo-N-phényl-tétrazole-1-carboxamide ;
le N-cyclohexyl-4-(2,6-difluorophényl)-5-oxo-N-phényl-tétrazole-1-carboxamide ;
le 4-(2,6-difluorophényl)-N-méthyl-5-oxo-N-(2-pyridyl)-tétrazole-1-carboxamide ;
le N,4-dicyclopropyl-5-oxo-N-phényl-tétrazole-1-carboxamide ;
le N-butyl-4-cyclopropyl-5-oxo-N-phényl-tétrazole-1-carboxamide ;
le 4-cyclopropyl-N-isobutyl-5-oxo-N-phényl-tétrazole-1-carboxamide ;
le 4-cyclopropyl-N-isopropyl-5-oxo-N-phényl-tétrazole-1-carboxamide ;
le 4-cyclopropyl-5-oxo-N-phényl-N-propyl-tétrazole-1-carboxamide ;
le 4-cyclopropyl-N-méthyl-5-oxo-N-phényl-tétrazole-1-carboxamide ;
le 4-cyclopropyl-N-éthyl-5-oxo-N-phényl-tétrazole-1-carboxamide ;
le 4-cyclopropyl-5-oxo-N-phényl-5-N-sec.-butyl-tétrazole-1-carboxamide ;
le N-(1-benzyl-2-méthyl-propyl)-4-cyclopropyl-5-oxo-N-phényl-tétrazole-1-carboxamide ;
la 1-(2-méthylpipéridine-1-carbonyl)-4-(p-tolyl)-tétrazole-5-one ;
la 1-(2-méthylpipéridine-1-carbonyl)-4-(m-tolyl)-tétrazole-5-one ;
le N-isopropyl-5-oxo-N-phényl-4-propyl-tétrazole-1-carboxamide ;
la 1-(4-chlorophényl)-4-[(2-fluorophényl)méthyl]-tétrazole-5-one ;
la 1-(4-chlorophényl)-4-[(3-fluorophényl)méthyl]-tétrazole-5-one ;
la 1-(4-chlorophényl)-4-[(3-chlorophényl)méthyl]-tétrazole-5-one ;
la 1-[(4-bromophényl)méthyl]-4-(4-chlorophényl)tétrazole-5-one ; et
le 3-[5-oxo-4-(p-tolyl)tétrazole-1-yl]propanoate de méthyle
soient exclus ;
où
« alkyle », seul ou en combinaison, signifie un groupe alkyle à chaîne linéaire ou à chaîne ramifiée avec 1 à 8 atomes de carbone ;
« cycloalkyle », seul ou en combinaison, signifie un noyau cycloalkyle avec 3 à 8 atomes de carbone ; et
« alkoxy », seul ou en combinaison, signifie un groupe de formule alkyle-O- dans lequel le terme « alkyle » a la signification donnée précédemment.

2. Composé selon la revendication 1, dans lequel R¹ est un groupe 3-fluorophényle, 3-chlorophényle ou 4-chlorophényle.

3. Composé selon la revendication 1 ou 2, dans lequel un de R³ et R⁴ est un groupe alkyle et l'autre est un groupe - -(CH2)ₙ-R⁶.

4. Composé selon l'une quelconque des revendications 1. à 3, dans lequel un de R³ et de R⁴ est un groupe méthyle ou éthyle et l'autre est un groupe - (CH₂)ₙ-R⁶.

5. Composé selon l'une quelconque des revendications 1 et 2, dans lequel R³ et R⁴ conjointement avec l'atome d'azote auquel ils sont attachés, forment un groupe hétérocyclyle ou hétérocyclyle substitué, où le groupe hétérocyclyle est un groupe pipéridinyle, pyrrolidinyle ou morpholinyle, et où le groupe hétérocyclyle substitué est un groupe hétérocyclyle substitué avec un substituant choisi parmi les groupes alkyle et alkylcarbonylamino, ou avec deux substituants choisis indépendamment parmi un alkyle.

6. Composé selon l'une quelconque des revendications 1, 2 et 5, dans lequel R³ et R⁴ conjointement avec l'atome d'azote auquel ils sont attachés, forment un groupe morpholinyle, diméthylpipéridinyle ou méthylcarbonylamino-pyrrolidinyle.

7. Composé selon l'une quelconque des revendications 1 et 2, dans lequel R⁵ est un groupe tertio-butyloxy-carbonylpipéridinyle, fluorophénylméthyle, éthoxycarbonyl-méthyle, éthoxycarbonyl(diméthyl)méthyle, (méthylsulfonyl)-(méthyl)[1,2,4]triazolyle ou morpholinylcarbonylméthyle.

8. Composé selon l'une quelconque des revendications 1 à 4, dans lequel R⁶ est un groupe alkoxy, dialkoxyphényle, phényle ou pyridinyle.

9. Composé selon l'une quelconque des revendications 1 à 4 et 8, dans lequel R⁶ est un groupe méthoxy, diméthoxyphényle, phényle ou pyridinyle.

10. Composé selon l'une quelconque des revendications 1 à 4, 8 et 9, dans lequel n vaut 1, 2 ou 3.

11. Composé selon l'une quelconque des revendications 1 à 10 choisi parmi :
le [2-(3,4-diméthoxy-phényl)-éthyl]-méthyl-amide d'acide 4-(3-fluorophényl)-5-oxo-4,5-dihydro-tétrazole-1-carboxylique ;
le (2-cyano-éthyl)-méthyl-amide d'acide 4-(3-fluoro-phényl)-5-oxo-4,5-dihydro-tétrazole-1-carboxylique ;
l'amide d'acide 1-[4-(3-fluorophényl)-5-oxo-4,5-dihydro-tétrazole-1-carbonyl]-pipéridine-3-carboxylique ;
la 1-(3-fluorophényl)-4-(morpholine-4-carbonyl)-1,4-dihydro-tétrazole-5-one ;
le méthyl-phénéthyl-amide d'acide 4-(3-fluorophényl)-5-oxo-4,5-dihydro-tétrazole-1-carboxylique ;
l'éthyl-pyridine-4-ylméthyl-amide d'acide 4-(3-fluorophényl)-5-oxo-4,5-dihydro-tétrazole-1-carboxylique ;
l'éthyl-(2-méthoxy-éthyl)-amide d'acide 4-(3-fluorophényl)-5-oxo-4,5-dihydro-tétrazole-1-carboxylique ;
le méthyl-(3-phényl-propyl)-amide d'acide 4-(3-fluorophényl)-5-oxo-4,5-dihydro-tétrazole-1-carboxylique ;
la 1-(2-benzyl-pyrrolidine-1-carbonyl)-4-(3-fluorophényl)-1,4-dihydro-tétrazole-5-one ;
la 1-(3-fluorophényl)-4-[2-(4-fluorophényl)-pyrrolidine-1-carbonyl]-1,4-dihydro-tétrazole-5-one ;
la 1-(6-luoro-3,4-dihydro-1H-isoquinoléine-2-carbonyl)-4-(3-fluorophényl)-1,4-dihydro-tétrazole-5-one ;
le benzyl-éthyl-amide d'acide 4-(3-fluorophényl)-5-oxo-4,5-dihydro-tétrazole-1-carboxylique ;
l'ester méthylique d'acide (2S,3S)-2-{[4-(3-fluorophényl)-5-oxo-4,5-dihydro-tétrazole-1-carbonyl]-méthyl-amino}-3-méthyl-pentanoïque ;
la 1-(4-fluoro-benzyl)-4-(3-fluorophényl)-1,4-dihydro-tétrazole-5-one ;
l'ester tertio-butylique d'acide 4-[4-(3-fluorophényl)-5-oxo-4,5-dihydro-tétrazole-1-yl]-pipéridine-1-carboxylique ;
l'ester éthylique d'acide 2-[4-(3-fluorophényl)-5-oxo-4,5-dihydro-tétrazole-1-yl]-2-méthyl-propionique ;
la 1-(3-fluorophényl)-4-(5-méthanesulfonyl-4-méthyl-4H-[1,2,4]triazole-3-ylméthyl)-1,4-dihydro-tétrazole-5-one ;
la 1-(3-fluorophényl)-4-[2-(2-méthyl-2H-pyrazole-3-yl)-pyrrolidine-1-carbonyl]-1,4-dihydro-tétrazole-5-one ;
la 1-(3-fluorophényl)-4-[2-(1-méthyl-1H-pyrazole-3-y1)-pyrrolidine-1-carbonyl]-1,4-dihydro-tétrazole-5-one ;
la 1-(3-fluorophényl)-4-[2-(3-méthyl-isoxazole-5-yl)-pyrrolidine-1-carbonyl]-1,4-dihydro-tétrazole-5-one ;
la 1-(3-fluorophényl)-4-((S)-2-méthoxyméthyl-pyrrolidine-1-carbonyl)-1,4-dihydro-tétrazole-5-one ;
l'amide d'acide (S)-1-[4-(3-fluorophényl)-5-oxo-4,5-dihydro-tétrazole-1-carbonyl]-pyrrolidine-2-carboxylique ;
le N-{1-[4-(3-fluorophényl)-5-oxo-4,5-dihydro-tétrazole-1-carbonyl]-pyrrolidine-3-yl}-acétamide ;
l'ester méthylique d'acide 1-[4-(3-fluorophényl)-5-oxo-4,5-dihydro-tétrazole-1-carbonyl]-azétidine-3-carboxylique ;
la 1-(3-fluorophényl)-4-[2-(3-méthyl-[1,2,4]oxadiazole-5-yl)-pyrrolidine-1-carbonyl]-1,4-dihydro-tétrazole-5-one ;
le N-{1-[4-(3-fluorophényl)-5-oxo-4,5-dihydro-tétrazole-1-carbonyl]-pipérlidine-4-yl}-acétamide ;
la 1-(3-fluorophényl)-4-[4-(2-oxo-pyrrolidine-1-yl-éthyl)-pipérazine-1-carbonyl]-1,4-dihydro-tétrazole-5-one ;
la 1-(4,4-diméthyl-pipéridine-1-carbonyl)-4-(3-fluorophényl)-1,4-dihydro-tétrazole-5-one ;
la 1-(1,1-dioxo-1λ6-thiomorpholine-4-carbonyl)-4-(3-fluorophényl)-1,4-dihydro-tétrazole-5-one ;
la 1-(3-fluorophényl)-4-(2,3,5,6-tétrahydro-[1,2']-bipyrazinyl-4-carbonyl)-1,4-dihydro-tétrazole-5-one ;
le (2-cyano-éthyl)-cyclopropyl-amide d'acide 4-(3-fluorophényl)-5-oxo-4,5-dihydro-tétrazole-1-carboxylique ;
le méthyl-pyridine-2-yl-amide d'acide 4-(3-fluorphényl)-5-oxo-4,5-dihydro-tétrazole-1-carboxylique ;
le méthyl-pyridine-3-yl-amide d'acide 4-(3-fluorophényl)-5-oxo-4,5-dihydro-tétrazole-1-carboxylique ;
la 1-(3-fluorophényl)-4-(2-méthoxyméthyl-pipéridine-1-carbonyl)-1,4-dihydro-tétrazole-5-one ;
le méthyl-pyridine-3-yl-amide d'acide 4-cyclopropyl-5-oxo-4,5-dihydro-tétrazole-1-carboxylique ;
le méthyl-pyridine-3-yl-amide d'acide 5-oxo-4-propyl-4,5-dihydro-tétrazole-1-carboxylique ;
le méthyl-pyridine-3-yl-amide d'acide 4-isobutyl-5-oxo-4,5-dihydro-tétrazole-1-carboxylique ;
la 1-(4-acétyl-pipérazine-1-carbonyl)-4-(3-fluorophényl)-1,4-dihydro-tétrazole-5-one ;
la 1-(3-fluorophényl)-4-(4-propionyl-pipérazine-1-carbonyl)-1,4-dihydro-tétrazole-5-one ;
le diéthylamide d'acide 4-[4-(3-fluorophényl)-5-oxo-4,5-dihydro-tétrazole-1-carbonyl]-pipérazine-1-carboxylique ;
la 1-(3-fluorophényl)-4-(3-phényl-pyrrolidine-1-carbonyl)-1,4-dihydro-tétrazole-5-one ;
le N-{(S)-1-[4-(3-fluorophényl)-5-oxo-4,5-dihydro-tétrazole-1-carbonyl]-pyrrolidine-3-yl}-acétamide ;
le N-{(R)-1-[4-(3-fluorophényl)-5-oxo-4,5-dihydro-tétrazole-1-carbonyl]-pyrrolidine-3-yl}-acétamide ;
le N-éthyl-N-{1-[4-(3-fluorophényl)-5-oxo-4,5-dihydro-tétrazole-1-carbonyl]-pyrrolidine-3-yl}-acétamide ;
la 1-(3,3-difluoro-pyrrolidine-1-carbonyl)-4-(3-fluorophényl)-1,4-dihydro-tétrazole-5-one ;
la 1-(2,2-diméthyl-pyrrolidine-1-carbonyl)-4-(3-fluorophényl)-1,4-dihydro-tétrazole-5-one ;
la 1-(3,3-diméthyl-pyrrolidine-1-carbonyl)-4-(3-fluorophényl)-1,4-dihydro-tétrazole-5-one ;
la 2-(3-fluorophényl)-4-(2-méthyl-pyrrolidine-1-carbonyl)-1,4-dihydro-tétrazole-5-one ;
le N-{1-[4-(3-fluorophényl)-5-oxo-4,5-dihydro-tétrazole-1-carbonyl]-pyrrolidine-3-yl}-N-méthyl-acétamide ;
l'ester éthylique d'acide 4-[4-(3-fluorophényl)-5-oxo-4,5-dihydro-tétrazole-1-carbonyl]-pipérazine-1-carboxylique ;
le (S)-1-[4-(3-fluorophényl)-5-oxo-4,5-dihydro-tétrazole-1-carbonyl]-pyrrolidine-2-carbonitrile ;
le (R)-1-[4-(3-fluorophényl)-5-oxo-4,5-dihydro-tétrazole-1-carbonyl]-pyrrolidine-2-carbonitrile ;
le benzyl-éthyl-amide d'acide 4-(6-chloro-pyridine-3-yl)-5-oxo-4,5-dihydro-tétrazole-1-carboxylique ;
le (2-méthoxy-éthyl)-méthyl-amide d'acide 4-(3-fluorophényl)-5-oxo-4,5-dihydro-tétrazole-1-carboxylique ;
l'isopropyl-(2-méthoxy-éthyl)-amide d'acide 4-(3-fluorophényl)-5-oxo-4,5-dihydro-tétrazole-1-carboxylique ;
le (2-méthoxy-éthyl)-propyl-amide d'acide 4-(3-fluorophényl)-5-oxo-4,5-dihydro-tétrazole-1-carboxylique ;
le bis-(2-méthoxy-éthyl)-amide d'acide 4-(3-fluorophényl)-5-oxo-4,5-dihydro-tétrazole-1-carboxylique ;
la 1-(2,6-diméthyl-morpholine-4-carbonyl)-4-(3-fluorophényl)-1,4-dihydro-tétrazole-5-one ;
la 1-((2S,6R)-2,6-diméthyl-morpholine-4-carbonyl)-4-(3-fluorophényl)-1,4-dihydro-tétrazole-5-one ;
la 1-(3-fluorophényl)-4-(4-méthoxyméthyl-pipéridine-1-carbonyl)-1,4-dihydro-tétrazole-5-one ;
le 1-[4-(3-fluorophényl)-5-oxo-4,5-dihydro-tétrazole-1-carbonyl]-pipéridine-4-carbonitrile ;
l'ester méthylique d'acide 4-tertio-butoxycarbonyl-amino-1-[4-(3-fluoraphényl)-5-oxo-4,5-dihydro-tétrazole-1-carbonyl]-pipéridine-4-carboxylique ;
la 1-(3,3-diméthyl-pipéridine-1-carbonyl)-4-(3-fluorophényl)-1,4-dihydro-tétrazole-5-one ;
la 1-(3-fluorophényl)-4-(2-oxa-8-aza-spiro[4.5]décane-8-carbonyl)-1,4-dihydro-tétrazole-5-one ;
le N-{(R)-1-[4-(3-chlorophényl)-5-oxo-4,5-dihydro-tétrazole-1-carbonyl]-pyrrolidine-3-yl}-acétamide ;
le N-{(R)-1-[5-oxo-4-(3-trifluorométhoxy-phényl)-4,5-dihydro-tétrazole-1-carbonyl]-pyrrolidine-3-yl}-acétamide ;
le N-{(R)-1-[4-(4-chlorophényl)-5-oxo-4,5-dihydro-tétrazole-1-carbonyl]-pyrrolidine-3-yl}-acétamide ;
le N-{(S)-1-[4-(3-chlorophényl)-5-oxo-4,5-dihydro-tétrazole-1-carbonyl]-pyrrolidine-3-yl}-acétamide ;
le N-{(S)-1-[5-oxo-4-(3-trifluorométhoxy-phényl)-4,5-dihydro-tétrazole-1-carbonyl]-pyrrolidine-3-yl}-acétamide ;
le N-{(S)-1-[4-(4-chlorophényl)-5-oxo-4,5-dihydro-tétrazole-1-carbonyl]-pyrrolidine-3-yl}-acétamide ;
le N-{1-[4-(3-chlorophényl)-5-oxo-4,5-dihydro-tétrazole-1-carbonyl]-pyrrolidine-3-yl}-N-éthyl-acétamide ;
le N-éthyl-N-{1-[5-oxo-4-(3-trifluorométhoxy-phényl)-4,5-dihydro-tétrazole-1-carbonyl]-pyrrolidine-3-yl}-acétamide ;
le N-{1-[4-(4-chlorophényl)-5-oxo-4,5-dihydro-tétrazole-1-carbonyl]-pyrrolidine-3-yl}-N-éthyl-acétamide ;
la 1-(3-chlorophényl)-4-[2-(3-méthyl-isoxazole-5-yl)-pyrrolidine-1-carbonyl]-1,4-dihydro-tétrazole-5-one ;
la 1-[2-(3-méthyl-isoxazole-5-yl)-pyrrolidine-1-carbonyl]-4-(3-trifluorométhoxy-phényl)-1,4-dihydro-tétrazole-5-one ;
la 1-(4-chlorophényl)-4-[2-(3-méthyl-isoxazole-5-yl)-pyrrolidine-1-carbonyl]-1,4-dihydro-tétrazole-5-one ;
la 1-(3-chlorophényl)-4-(4,4-diméthyl-pipéridine-1-carbonyl)-1,4-dihydro-tétrazole-5-one ;
la 1-(4,4-diméthyl-pipéridine-1-carbonyl)-4-(3-trifluorométhoxy-phényl)-1,4-dihydro-tétrazole-5-one ;
la 1-(4-chlorophényl)-4-(4,4-diméthyl-pipéridine-1-carbonyl)-1,4-dihydro-tétrazole-5-one ;
l'éthyl-(2-méthoxy-éthyl)-amide d'acide 4-(3-chlorophényl)-5-oxo-4,5-dihydro-tétrazole-1-carboxylique ;
l'éthyl-(2-méthoxy-éthyl)-amide d'acide 5-oxo-4-(3-trifluorométhoxy-phényl)-4,5-dihydro-tétrazole-1-carboxylique ;
l'éthyl-(2-méthoxy-éthyl)-amide d'acide 4-(4-chlorophényl)-5-oxo-4,5-dihydro-tétrazole-1-carboxylique ;
l'isopropyl-(2-méthoxy-éthyl)-amide d'acide 4-(3-chlorophényl)-5-oxo-4,5-dihydro-tétrazole-1-carboxylique ;
l'isopropyl-(2-méthoxy-éthyl)-amide d'acide 5-oxo-4-(3-trifluorométhoxy-phényl)-4,5-dihydro-tétrazole-1-carboxylique ;
l'isopropyl-(2-méthoxy-éthyl)-amide d'acide 4-(4-chlorophényl)-5-oxo-4,5-dihydro-tétrazole-1-carboxylique ;
le (3-méthoxy-propyl)-méthyl-amide d'acide 4-(3-chlorophényl)-5-oxo-4,5-dihydro-tétrazole-1-carboxylique ;
le (3-méthoxy-propyl)-méthyl-amide d'acide 5-oxo-4-(3-trifluorométhoxy-phényl)-4,5-dihydro-tétrazole-1-carboxylique ;
le (3-méthoxy-propyl)-méthyl-amide d'acide 4-(4-chlorophényl)-5-oxo-4,5-dihydro-tétrazole-1-carboxylique ;
la 1-(3-chlorophényl)-4-[4-(2-morpholine-4-yl-2-oxo-éthyl)-pipérazine-1-carbonyl]-1,4-dihydro-tétrazole-5-one ;
la 1-[4-(2-morpholine-4-yl-2-oxo-éthyl)-pipérazine-1-carbonyl]-4-(3-trifluorométhoxy-phényl)-1,4-dihydro-tétrazole-5-one ;
la 1-(4-chlorophényl)-4-[4-(2-morpholine-4-yl-2-oxo-éthyl)-pipérazine-1-carbonyl]-1,4-dihydro-tétrazole-5-one ;
le 2-{4-[4-(3-chlorophényl)-5-oxo-4,5-dihydro-tétrazole-1-carbonyl]-pipérazine-1-yl}-N,N-diméthyl-acétamide ;
le N,N-diméthyl-2-{4-[5-oxo-4-(3-trifluorométhoxy)-phényl)-4,5-dihydro-tétrazole-1-carbonyl]-pipérazine-1-yl}-acétamide ;
le 2-{4-[4-(4-chlorophényl)-5-oxo-4,5-dihydro-tétrazole-1-carbonyl]pipérazine-1-yl}-N,N-diméthyl-acétamide ;
la 1-(3-chlorophényl)-4-[4-(2-oxo-2-pyrrolidine-1-yl-éthyl)-pipérazine-1-carbonyl]-1,4-dihydro-tétrazole-5-one ;
la 1-[4-(2-oxo-2-pyrrolidine-1-yl-éthyl)-pipérazine-1-carbonyl]-4-(3-trifluorométhoxy-phényl)-1,4-dihydro-tétrazole--5-one ;
la 1-(4-chlorophényl)-4-[4-(2-oxo-pyrrolidine-1-yl-éthyl)-pipérazine-1-carbonyl]-1,4-dihydro-tétrazole-5-one ;
le (2-éthoxy-éthyl)-éthyl-amide d'acide 4-(3-chlorophényl)-5-oxo-4,5-dihydro-tétrazole-1-carboxylique ;
le (2-éthoxy-éthyl)-éthyl-amide d'acide 5-oxo-4-(3-trifluorométhoxy-phényl)-4,5-dihydro-tétrazole-1-carboxylique ;
le (2-éthoxy-éthyl)-éthyl-amide d'acide 4-(4-chlorophényl)-5-oxo-4,5-dihydro-tétrazole-1-carboxylique ;
à 1-(3-chlorophényl)-4-(morpholine-4-carbonyl)-1,4-dihydro-tétrazole-5-one ;
la 1-(morpholine-4-carbonyl)-4-(3-trifluorométhoxy-phényl)-1,4-dihydro-tétrazole-5-one ;
la 1-(4-chlorophényl)-4-(morpholine-4-carbonyl)-1,4-dihydro-tétrazole-5-one ;
le 2-{4-[4-(3-chlorophényl)-5-oxo-4,5-dihydro-tétrazole-1-carbonyl]-pipérazine-1-yl}-N-éthyl-acétamide ;
le N-éthyl-2-{4-[5-oxo-4-(3-trifluorométhoxy-phényl)-4, 5-dihydro-tétrazole-1-carbonyl] -pipérazine-1-yl}-acétamide ;
le 2-{4-[4-(4-chlorophényl)-5-oxo-4,5-dihydro-tétrazole-1-carbonyl]-pipérazine-1-yl}-N-éthyl-acétamide ;
le N-butyl-2-{4-[4-(3-chlorophényl)-5-oxo-4,5-dihydro-tétrazole-1-carbonyl]-pipérazine-1-yl}-acétamide ;
le N-butyl-2-{4-[5-oxo-4-(3-trifluorométhoxy-phényl)-4,5-dihydro-tétrazole-1-carbonyl]-pipérazïne-1-yl}-acétamide ;
le N-butyl-2-{4-[4-(4-chlorophényl)-5-oxo-4,5-dihydro-tétrazole-1-carbonyl]-pipérazine-1-yl}-acétamide ;
le (2-méthoxy-éthyl)-amide d'acide 4-(3-fluorophényl)-5-oxo-4,5-dihydro-tétrazole-1-carboxylique ;
le (2-éthoxy-éthyl)-méthyl-amide d'acide 4-(3-chlorophényl)-5-oxo-4,5-dihydro-tétrazole-1-carboxylique ;
le (2-éthoxy-éthyl)-méthyl-amide d'acide 5-oxo-4-(3-trifluorométhoxy-phényl)-4,5-dihydro-tétrazole-1-carboxylique ;
le (2-éthoxy-éthyl)-méthyl-amide d'acide 4-(4-chlorophényl)-5-oxo-4,5-dihydro-tétrazole-1-carboxylique ;
l'ester éthylique d'acide [4-(3-chlorophényl)-5-oxo-4,5-dihydro-tétrazole-1-yl]-acétique ;
la 1-(3-chlorophényl)-4-(2-morpholine-4-yl-2-oxo-éthyl)-1,4-dihydro-tétrazole-5-one ;
la 1-(3-chlorophényl)-4-[4-(1-méthyl-2-oxo-2-pyrrolidine-1-yl-éthyl)-pipérazine-1-carbonyl]-1,4-dihydro-tétrazole-5-one ;
la 1-[4-(1-méthyl-2-oxo-pyrrolidine-1-yl-éthyl)-pipérazine-1-carbonyl]-4-(3-trifluorométhoxy-phényl)-1,4-dihydro-tétrazole-5-one ;
la 1-(4-chlorophényl)-4-[4-(1-méthyl-2-oxo-pyrrolidine-1-yl-éthyl)-pipérazine-1-carbonyl]-1,4-dihydro-tétrazole-5-one ;
la 1-(morpholine-4-carbonyl)-4-[2-(trifluorométhyl)-phényl]tétrazole-5-one ;
la 1-(morpholine-4-carbonyl)-4-[3-(trifluorométhyl)-phényl]tétrazole-5-one ;
la 1-(morpholine-4-carbonyl)-4-[4-(trifluorométhyl)-phényl]tétrazole-5-one ;
la 1-(2,6-difluorophényl)-4-(morpholine-4-carbonyl)-tétrazole-5-one ;
la 1-(2,4-difluorophényl)-4-(morpholine-4-carbonyl)-tétrazole-5-one ;
la 1-(2,5-difluorophényl)-4-(morpholine-4-carbonyl)-tétrazole-5-one ;
la 1-(3,4-difluorophényl)-4-(morpholine-4-carbonyl)-tétrazole-5-one ;
la 1-(3,5-difluorophényl)-4-(morpholine-4-carbonyl)-tétrazole-5-one ;
la 1-(3-méthylphényl)-4-(morpholine-4-carbonyl)-tétrazole-5-one ;
la 1-(4-méthylphényl)-4-(morpholine-4-carbonyl)-tétrazole-5-one ;
la 1-(3-méthoxyphényl)-4-(morpholine-4-carbonyl)-tétrazole-5-one ;
la 1-(4-méthoxyphényl)-4-(morpholine-4-carbonyl)-tétrazole-5-one ;
la 1-(2,3-dichlorophényl)-4-(morpholine-4-carbonyl)-tétrazole-5-one ;
la 1-(1-méthylindole-4-yl)-4-(morpholine-4-carbonyl)-tétrazole-5-one ;
la 1-(1-méthylindole-5-yl)-4-(morpholine-4-carbonyl)-tétrazole-5-one ;
la 1-(1-méthylindole-6-yl)-4-(morpholine-4-carbonyl)-tétrazole-5-one ;
la 1-(3-bromophényl)-4-(morpholine-4-carbonyl)-tétrazole-5-one ;
la 1-(3-chlorophényl)-4-[(2R,6S)-2,6-diméthyl-morpholine-4-carbonyl]tétrazole-5-one ;
la 1-(3-chlorophényl)-4-(2,6,-diméthylmorpholine-4-carbonyl)tétrazole-5-one ; et
la 1-(3-chlorophényl)-4-(3,3-diméthylpyrrolidine-1-carbonyl)tétrazole-5-one.

12. Composé selon l'une quelconque des revendications 1 à 11, choisi parmi :
le [2-(3,4-diméthoxy-phényl)-éthyl]-méthyl-amide d'acide 4-(3-fluorophényl)-5-oxo-4,5-dihydro-tétrazole-1-carboxylique ;
le méthyl-phénéthyl-amide d'acide 4-(3-fluorophényl)-5-oxo-4,5-dihydro-tétrazole-1-carboxylique ;
l'éthyl-pyridine-4-ylméthyl-amide d'acide 4-(3-fluorophényl)-5-oxo-4,5-dihydro-tétrazole-1-carboxylique ;
le benzyl-éthyl-amide d'acide 4-(3-fluorophényl)-5-oxo-4,5-dihydro-tétrazole-1-carboxylique ;
la 1-(4,4-diméthyl-pipéridine-1-carbonyl)-4-(3-fluorophényl)-1,4-dihydro-tétrazole-5-one ;
le N-{(R)-1-[4-(3-fluorophényl)-5-oxo-4,5-dihydro-tétrazole-1-carbonyl]-pyrrolidine-3-yl}-acétamide ;
la 1-(3-chlorophényl)-4-(4,4-diméthyl-pipéridine-1-carbonyl)-1,4-dihydro-tétrazole-5-one ;
la 1-(4-chlorophényl)-4-(4,4-diméthyl-pipéridine-1-carbonyl)-1,4-dihydro-tétrazole-5-one ;
le (3-méthoxy-propyl)-méthyl-amide d'acide 4-(3-chlorophényl)-5-oxo--4,5-dihydro-tétrazole-1-carboxylique ; et
la 1-(3-chlorophényl)-4-(morpholine-4-carbonyl)-1,4-dihydro-tétrazole-5-one.

13. Procédé de préparation d'un composé selon l'une quelconque des revendications 1 à 12, comprenant la réaction d'un composé de formule (A) 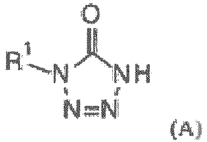
(a) en présence d'un groupe R⁵-X et d'une base ; ou
(b) en présence de phosgène, de diphosgène ou de triphosgène suivi par une réaction en présence d'un groupe HNR³R⁴ et d'une base ;
dans laquelle X est un groupe attracteur d'électrons et dans laquelle R¹, R³, R⁴ et R⁵ sont tels que définis dans l'une quelconque des revendications 1 à 10.

14. Composé selon l'une quelconque des revendications 1 à 12, pour une utilisation comme substance thérapeutiquement active.

15. Composition pharmaceutique comprenant un composé selon l'une quelconque des revendications 1 à 12 et un véhicule thérapeutiquement inerte.

16. Composé selon l'une quelconque des revendications 1 à 12, pour une utilisation dans le traitement ou la prophylaxie de la douleur, de l'athérosclérose, de la dégénérescence maculaire liée à l'âge, de la rétinopathie diabétique, du glaucome, de l'occlusion de veine rétinienne, de la rétinopathie des prématurés, du syndrome ischémique oculaire, de l'atrophie géographique, du diabète sucré, de l'inflammation, de la maladie de l'intestin inflammatoire, de la lésion d'ischémie-reperfusion, de l'insuffisance hépatique aiguë, de la fibrose hépatique, de la fibrose pulmonaire, de la fibrose rénale, de la fibrose systémique, du rejet d'allogreffe aigu, de la néphropathie d'allogreffe chronique, de la néphropathie diabétique, de la glomérulonéphropathie, de la cardiomyopathie, de l'insuffisance cardiaque, de l'ischémie myocardiaque, de l'infarctus du myocarde, de la sclérose systémique, d'une lésion thermique, de brûlures, de cicatrices hyper-trophiées, de chéloïdes, de la gingivite, de la pyrexie, de la cirrhose ou de tumeurs hépatiques, de la régulation de la masse osseuse, de la sclérose latérale amyotrophique, de la sclérose en plaques, de la maladie d'Alzheimer, de la maladie de Parkinson, d'un ictus, de l'attaque ischémique transitoire ou de l'uvéite.
